# EUROPÄISCHE PATENTSCHRIFT

(11) **EP 1 501 565 B1**
(45) Veröffentlichungstag und Bekanntmachung des Hinweises auf die Patenterteilung: **02.11.2006**
(21) Anmeldenummer: 03729829.6
(22) Anmeldetag: 15.04.2003
(51) Int. Cl.: A61L 31/16, A61L 33/08, C08L 5/10, C08B 37/10

(54) **VERBINDUNGEN UND VERFAHREN ZUR HEMOKOMPATIBLEN BESCHICHTUNG VON OBERFLÄCHEN**
COMPOUNDS AND METHOD FOR COATING SURFACES IN A HAEMOCOMPATIBLE MANNER
COMPOSES ET PROCEDES POUR RECOUVRIR DE FA ON HEMOCOMPATIBLE DES SURFACES

(30) Priorität: 09.05.2002 US 378676 P; 10.05.2002 DE 10221055
(43) Veröffentlichungstag der Anmeldung: 02.02.2005
(73) Patentinhaber: Hemoteq GmbH, 52146 Würselen (DE)
(72) Erfinder: HORRES, Roland, 52223 Stolberg (DE); LINSSEN, Marita, Katharina, 52066 Aachen (DE); HOFFMANN, Michael, 52249 Eschweiler (DE); FAUST, Volker, 52080 Aachen (DE); HOFFMANN, Erika, 52249 Eschweiler (DE); DI BIASE, Donato, 52080 Aachen (DE)
(74) Vertreter: Arth, Hans-Lothar
(86) Internationale Anmeldenummer: PCT/DE2003/001253
(87) Internationale Veröffentlichungsnummer: WO 2003/094990

(56) Entgegenhaltungen:
- WO-A-99/26983
- WO-A-99/27976

## Beschreibung

Die Erfindung betrifft die Verwendung von Oligo- und/oder Polysacchariden, enthaltend den Zuckerbaustein N-Acylglucosamin und/oder N-Acylgalactosamin für die Herstellung hemokompatibler Oberflächen, Verfahren zur hemokompatiblen Beschichtung von Oberflächen mit diesen Oligo- und/oder Polysacchariden sowie die Verwendung der hemokompatibel beschichteten Oberflächen.

Im menschlichen Körper kommt das Blut nur im Falle einer Verletzung mit anderen Oberflächen in Kontakt als der Innenseite von natürlichen Blutgefäßen. Daher wird das Blutgerinnungssystem immer dann aktiviert, wenn Blut mit fremden Oberflächen in Kontakt kommt, um die Blutung zu stillen und einen lebensbedrohlichen Blutverlust zu verhindern. Da ein Implantat ebenfalls eine fremde Oberfläche darstellt, werden alle Patienten, die ein Implantat erhalten, das dauerhaft mit Blut in Kontakt steht, für die Dauer des Blutkontaktes mit Medikamenten behandelt, mit sogenannten Antikoagulantien, welche die Blutgerinnung unterdrücken. Dies gilt ebenso für Patienten, bei denen eine extrakorporale Zirkulation angewendet wird, wie zum Beispiel Hämodialysepatienten. Diese gerinnungsunterdrückende Medikation ist jedoch mit zum Teil erheblichen Nebenwirkungen behaftet, die von Haarausfall, Nausea und Erbrechen über Thrombozytopenie, hämorrhagischen Hautnekrosen und erhöhter Blutungsneigung bis zu tödlich verlaufenden Nebenwirkungen wie beispielsweise Hirnblutungen reichen.

Somit besteht ein Bedarf an nicht-thrombogenen, hemokompatiblen Werkstoffen, beispielsweise Prothesen, Organersatzteilen, Membranen, Kanülen, Schläuchen, Blutbehältern, Stents, usw., welche bei Blutkontakt nicht das Gerinnungssystem auslösen und zur Koagulation des Blutes führen.

EP-B-O 333 730 beschreibt ein Verfahren zur Herstellung hemokompatibler Substrate durch Einarbeitung, Adhäsion und/oder Modifizierung und Verankerung von nicht-thrombogenem Endothelzelloberflächen-Polysaccharid (HS I). Die Immobilisierung dieses spezifischen Endothelzelloberflächenproteoheparansulfats HS I auf biologischen oder künstlichen Oberflächen bewirkt, daß derartig beschichtete Oberflächen blutverträglich werden und für den dauerhaften Blutkontakt geeignet sind. Nachteilig ist hingegen, daß dieses Verfahren für die Gewinnung von HS I die Kultivierung von Endothelzellen voraussetzt, so daß die wirtschaftliche Verwertbarkeit dieses Verfahrens stark eingeschränkt ist, da die Kultivierung von Endothelzellen zeitaufwendig ist und größere Mengen an kultivierten Endothelzellen nur mit immensem Kostenaufwand erhältlich sind.

Einen hemokompatiblen Werkstoff sowie ein Verfahren zu dessen Herstellung offenbart die internationale Patentanmeldung PCT/EP98/07668 (Veröffentlichungsnummer WO 99/27976 A), worin N-desulfatierte Heparine und nicht thrombogene Polymere enthaltend ein Basispolymer beschrieben werden, an welches diese Heparine kovalent gebunden sind. Als Basispolymer werden bevorzugt Polyvinylchlorid oder Silicon eingesetzt.

Einen anderen Werkstoff zur Verhinderung von arterieller Thrombose offenbart WO 99/26983 A. Darin werden heparinähnliche Verbindungen umfassend Heparine oder heparinähnliche Glykosaminoglykane als solche oder als Proteoglykane oder gebunden an ein globuläres Kernmolekül beschrieben. Die Antithrombose-Eigenschaften ergeben sich aus der hohen Kopplungsdichte von negativ geladenem Heparin oder den heparinähnlichen Glykosaminoglykanen, welche direkt oder über einen Spacer oder Linker an die globulären Kernmoleküle gebunden werden.

Aufgabe der vorliegenden Erfindung ist es, Substanzen für die hemokompatible Beschichtung von Oberflächen sowie Verfahren zur hemokompatiblen Beschichtung von Oberflächen zur Verfügung zu stellen und ihre Anwendung auf Oberflächen zur Verhinderung oder Minimierung unerwünschter Reaktionen. Insbesondere ist es die Aufgabe der vorliegenden Erfindung Medizinprodukte bereitzustellen, welche ein kontinuierliches kontrolliertes Einwachsen des Medizinproduktes erlauben - einerseits durch Unterdrückung der zellulären Reaktionen in den ersten Tagen und Wochen nach der Implantation mit Hilfe der ausgewählten Wirkstoffe und Wirkstoffkombinationen und andererseits durch die Bereitstellung einer athrombogenen bzw. inerten bzw. biokompatiblen Oberfläche, die gewährleistet, dass mit Abklingen des Wirkstoffeinflusses keine Reaktionen auf die bestehende Fremdoberfläche mehr einsetzen, die langzeitlich ebenfalls zu Komplikationen führen können.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung, den Figuren sowie den Beispielen.

Die vorliegende Erfindung offenbart Polysaccharide der allgemeinen Formel Ia 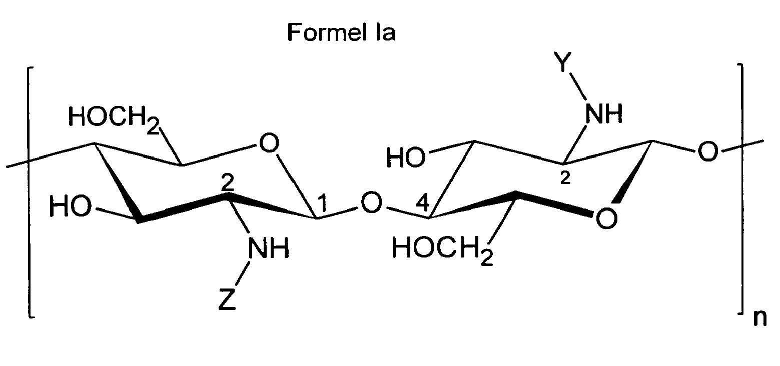 sowie strukturell sehr ähnliche Polysaccharide der allgemeinen Formel Ib

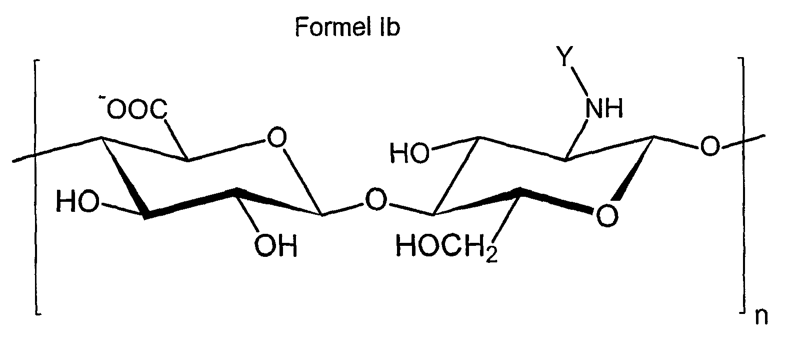

Die Polysaccharide gemäß Formel Ia weisen Molekulargewichte von 2kD bis 400kD auf, bevorzugt von 5kD bis 150kD, mehr bevorzugt von 10kD bis 100kD und insbesondere bevorzugt von 30kD bis 80kD. Die Polysaccharide gemäß Formel Ib weisen Molekulargewichte von 2kD bis 15kD auf, bevorzugt von 4kD bis 13kD, mehr bevorzugt von 6kD bis 12kD und insbesondere bevorzugt von 8kD bis 11kD. Die Variable n ist eine ganze Zahl im Bereich von 4 bis 1050. Bevorzugt ist n eine ganze Zahl von 9 bis 400, mehr bevorzugt von 14 bis 260 und insbesondere bevorzugt eine ganze Zahl zwischen 19 und 210.

Die allgemeine Formel Ia und Ib gibt ein Disaccharid wieder, welches als Grundbaustein des erfindungsgemäßen Polysaccharides anzusehen ist und durch n-fache Aneinanderreihung des Grundbausteins das Polysaccharid ergibt. Dieser aus zwei Zuckermolekülen aufgebaute Grundbaustein soll nicht dahingehend ausgelegt werden, daß unter die allgemeinen Formeln Ia und Ib nur Polysaccharide mit einer geraden Anzahl an Zuckermolekülen fallen. Natürlich umfaßt die allgemeine Formel Ia sowie die Formel Ib auch Polysaccharide mit einer ungeraden Anzahl an Zuckerbausteinen. Als Endgruppen der Oligo- bzw. Polysaccharide liegen Hydroxygruppen vor.

Die Reste Y und Z repräsentieren unabhängig voneinander die folgenden chemischen Acyl- oder Carboxyalkylgruppen:
-CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻.

Bevorzugt sind die Acylreste -COCH₃, -COC₂H₅, -COC₃H₇ sowie die Carboxyalkylreste -CH₂COO⁻, -C₂H₄COO⁻, -C₃H₆COO⁻. Mehr bevorzugt sind Acetyl- und Propanoylgruppe sowie der Carboxymethyl- und Carboxyethylrest. Insbesondere bevorzugt sind die Acetylgruppe und der Carboxymethylrest. Zudem ist bevorzugt, wenn der Rest Y eine Acylgruppe und der Rest Z eine Carboxyalkylgruppe repräsentiert. Mehr bevorzugt ist, wenn Y ein Rest -COCH₃, -COC₂H₅ oder -COC₃H₇ und insbesondere -COCH₃ ist. Zudem ist ferner bevorzugt, wenn Z ein Carboxyethyl- oder Carboxymethylrest ist, wobei der Carboxymethylrest insbesondere bevorzugt ist.

Der gemäß Formel Ia gezeigte Disaccharidgrundbaustein enthält jeweils einen Substituenten Y und einen weiteren Rest Z. Dies soll verdeutlichen, daß das erfindungsgemäße Polysaccharid zwei verschiedene Reste, nämlich Y und Z enthält. Dabei soll die allgemeine Formel Ia gerade nicht nur Polysaccharide umfassen, welche die Reste Y und Z in streng alternierender Abfolge enthalten, wie sich aus der Aneinanderreihung der Disaccharidgrundbausteine ergeben würde, sondern auch Polysaccharide, welche die Reste Y und Z in vollständig statistischer Abfolge an den Aminogruppen tragen. Ferner soll die allgemeine Formel Ia auch solche Polysaccharide umfassen, die die Reste Y und Z in unterschiedlicher Anzahl enthalten. Verhältnisse zwischen der Anzahl an Resten Y zu der Anzahl an Resten X können zwischen 70% : 30%, bevorzugt zwischen 60% : 40% und besonders bevorzugt zwischen 45% : 55% liegen. Insbesondere bevorzugt sind derartige Polysaccharide der allgemeinen Formel Ia, welche im wesentlichen an der Hälfte der Aminogruppen den Rest Y und an der anderen Hälfte der Aminogruppen den Rest Z in einer rein statistischen Verteilung tragen. Der Begriff "im wesentlichen die Hälfte" bedeutet im Idealfall exakt 50%, soll jedoch den Bereich von 45% bis 55% und insbesondere 48% bis 52% mit umfassen.

Bevorzugt sind die Verbindungen der allgemeinen Formel Ia, worin die Reste Y und Z folgende Bedeutung haben:

| | | |
|---|---|---|
| Y = -CHO | und | Z = -C₂H₄COO⁻ |
| Y = -CHO | und | Z = -CH₂COO⁻ |
| Y = -COCH₃ | und | Z = -C₂H₄COO⁻ |
| Y = -COCH₃ | und | Z = -CH₂COO⁻ |
| Y = -COC₂H₅ | und | Z = -C₂H₄COO⁻ |
| Y = -COC₂H₅ | und | Z = -CH₂COO⁻ |

Insbesondere bevorzugt sind die Verbindungen der allgemeinen Formel Ia, worin die Reste Y und Z folgende Bedeutung haben:

| | | |
|---|---|---|
| Y = -CHO | und | Z = -C₂H₄COO⁻ |
| Y = -COCH₃ | und | Z = -CH₂COO⁻ |

Bevorzugt sind die Verbindungen der allgemeinen Formel Ib, worin Y eine der folgenden Gruppen ist: -CHO, -COCH₃, -COC₂H₅ oder -COC₃H₇. Weiter bevorzugt sind die Gruppen -CHO, -COCH₃, -COC₂H₅ und insbesondere bevorzugt ist die Gruppe -COCH₃.
Die Verbindungen der allgemeinen Formel Ib enthalten nur noch einen geringen Anteil an freien Aminogruppen. Da mit dem Ninhydrintest keine freien Aminogruppen mehr nachgewiesen werden konnten, kann aufgrund der Empfindlichkeit dieses Tests geschlossen werden, dass weniger als 2%, bevorzugt weniger als 1 % und insbesondere bevorzugt weniger als 0,5% aller -NH-Y-Gruppen als freie Aminogruppen vorliegen, d.h. bei diesem geringen Prozentsatz der Gruppen -NH-Y bedeutet Y Wasserstoff.

Da die Polysaccharide der allgemeinen Formeln Ia und Ib Carboxylatgruppen und Aminogruppen enthalten, umfassen die allgemeinen Formlen Ia und Ib auch Alkali- sowie Erdalkalimetallsalze der entsprechenden Polysaccharide. So können Alkalimetallsalze wie das Natriumsalz, das Kaliumsalz, das Lithiumsalz oder Erdalkalimetallsalze wie beispielsweise das Magnesiumsalz oder das Calciumsalz genannt werden. Ferner können mit Ammoniak, primären, sekundären, tertiären und quaternären Aminen, Pyridin und Pyridinderivaten Ammoniumsalze, bevorzugt Alkylammoniumsalze und Pyridiniumsalze gebildet werden. Zu den Basen, welche mit den Polysacchariden Salze bilden, zählen anorganische und organische Basen wie beispielsweise NaOH, KOH, LiOH, CaCO₃, Fe(OH)₃, NH₄OH, Tetraalkylammoniumhydroxide und ähnliche Verbindungen.

Heparansulfate kommen ubiquitär auf Zelloberflächen von Säugetieren vor. Von Zelltyp zu Zelltyp unterscheiden sie sich stark in Molekulargewicht, Acetylierungsgrad und Sulfatierungsgrad. Leberheparansulfat weist beispielweise einen Acetylierungsgrad von ca. 50% auf, wohingegen das Heparansulfat aus der Glykokalix von Endothelzellen einen Acetylierungsgrad von bis zu 90% und größer aufweisen kann. Heparin weist nur einen sehr geringen Acetylierungsgrad von bis zu 5% auf. Der Sulfatierungsgrad liegt beim Leberheparansulfat und Heparin bei - 2 pro Disaccharideinheit, bei Endothelzellheparansulfat nahe bei 0 und bei Heparansulfaten aus anderen Zelltypen zwischen 0 und 2 pro Disaccharideinheit.

Die folgende Abbildung zeigt eine Tetrasaccharideinheit eines Heparins oder Heparansulfates mit statistischer Verteilung der Sulfatgruppen und einem Sulfatierungsgrad von 2 pro Disaccharideinheit wie er für Heparin typisch ist:

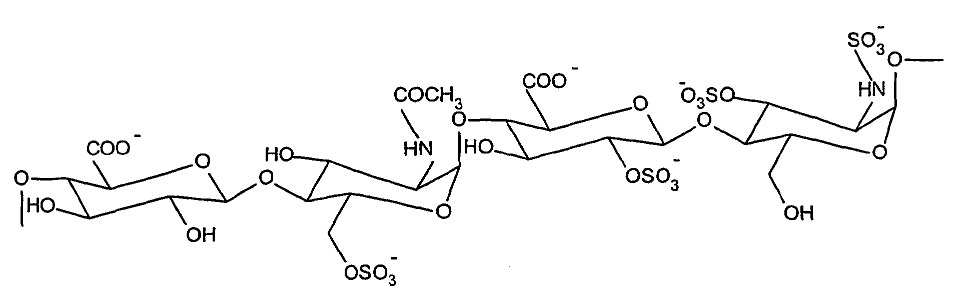

Allen Heparansulfaten ist mit Heparin der Ablauf der Biosynthese gemeinsam. Dabei wird als erstes das Coreprotein mit der Xylose-haltigen Bindungsregion aufgebaut. Sie besteht aus der Xylose und zwei damit verbundenen Galactoseresten. An den letzten der beiden Galactosereste wird dann abwechselnd je eine Glucuronsäure und ein Galactosamin gebunden, bis die entsprechende Kettenlänge erreicht ist. Abschließend erfolgt eine mehrstufige enzymatische Modifizierung dieses gemeinsamen Vorläufer-Polysaccharides aller Heparansulfate und des Heparins durch Sulfotransferasen und Epimerasen, die durch ihre unterschiedlich vollständigen Umsetzungen das breite Spektrum an verschiedenen Heparansulfaten bis hin zum Heparin generieren.

Heparin ist alternierend aus D-Glucosamin und D-Glucuronsäure bzw. L-Iduronsäure aufgebaut, wobei D-Glucosamin und D-Glucuronsäure β-1,4-glykosidisch (bzw. L-Iduronsäure in α-1,4-glykosidischer Verknüpfung) zum Disaccharid verknüpft sind, welches die Heparinuntereinheiten bildet. Diese Untereinheiten sind wiederum miteinander β-1,4-glykosidisch verknüpft und führen zum Heparin. Die Stellung der Sulfonylgruppen kann wechseln. Eine Tetrasaccharideinheit enthält durchschnittlich 4 bis 5 Schwefelsäurereste. Heparansulfat, auch als Heparitinsulfat bezeichnet, enthält mit Ausnahme des Leberheparansulfates weniger N- und O-gebundene Sulfonylgruppen als Heparin, dafür aber mehr N-Acetylgruppen.

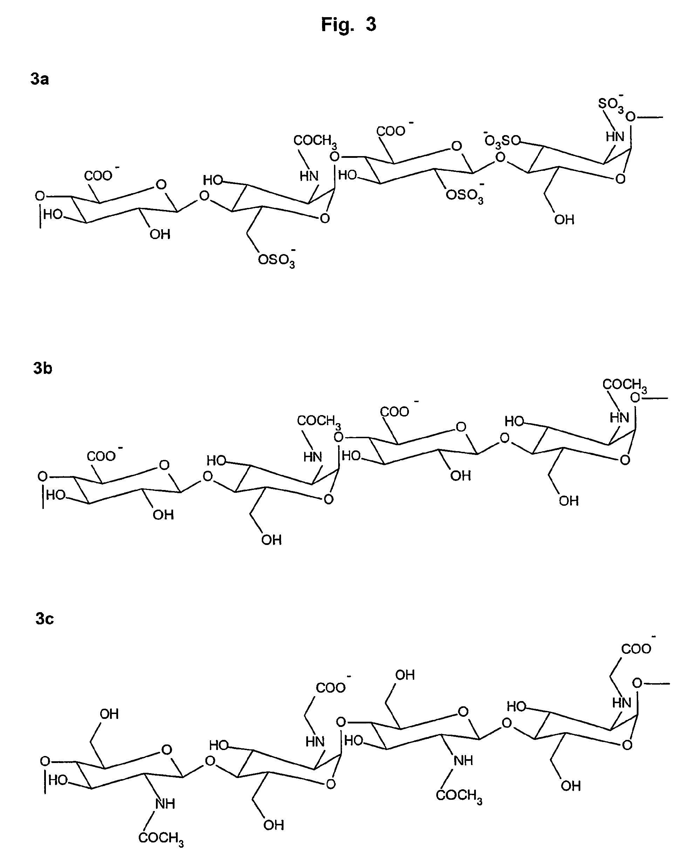

Wie aus Figur 3 deutlich wird, sind die Verbindungen der allgemeinen Formel Ia (s. Fig. 3c als Beispiel) und die Verbindungen der allgemeinen Formel Ib (s. Fig. 3b als Beispiel) dem natürlichen Heparansulfat der Endothelzellen strukturell ähnlich, vermeiden aber die eingangs geschilderten Nachteile bei der Verwendung von Endothelzellheparansulfaten.

Für die antithrombotische Wirksamkeit wird eine bestimmte Pentasaccharideinheit verantwortlich gemacht, die sich in handelsüblichen Heparinpräparaten in ca. jedem dritten Molekül wiederfindet. Durch spezielle Trennverfahren lassen sich Heparinpräparate unterschiedlicher antithrombotischer Aktivität herstellen. In hochaktiven, beispielsweise durch Antithrombin-III-Affinitätschromatographie erhaltenen Präparationen ("High-Affinity"-Heparin) findet sich diese aktive Sequenz in jedem Heparinmolekül, wogegen in "No-Affinity"-Präparationen keine charakteristischen Pentasaccharidsequenzen und damit keine aktive Gerinnungshemmung nachgewiesen werden können. Durch die Wechselwirkung mit diesem Pentasaccharid wird die Aktivität von Antithrombin III, einem Inhibitor des Gerinnungs-Schlüsselfaktors Thrombin, wesentlich potenziert (Bindungsaffinitätssteigerung bis um den Faktor 2X10³) [Stiekema J.C.J.; *Clin Nephrology* 26, Suppl. Nr 1, S3-S8, (1986)].

Die Aminogruppen des Heparins sind meistenteils N-sulfatiert oder N-acetyliert. Die wichtigsten O-Sulfatierungspositionen sind das C2 in der Iduronsäure, sowie das C6 und das C3 in Glucosamin. Für die Wirkung des Pentasaccharids auf die plasmatische Gerinnung wird hauptsächlich die Sulfatgruppe am C6 verantwortlich gemacht, im geringeren Maße auch die anderen funktionellen Gruppen.

Mit Heparin oder Heparansulfaten beschichtete Oberflächen medizinischer Implantate sind und bleiben durch die Beschichtung nur bedingt hemokompatibel. Das auf die künstliche Oberfläche aufgebrachte Heparin oder Heparansulfat verliert teilweise in drastischem Maße seine antithrombotische Wirkung, was mit einer aufgrund sterischer Hinderung eingeschränkten Wechselwirkung der besagten Pentasaccharideinheiten mit Antithrombin III in Zusammenhang gebracht wird.

In allen Fällen wird aufgrund der Immobilisierung dieser polyanionischen Substanzen eine starke Plasmaproteinadsorption auf der heparinisierten Oberfläche beobachtet, was einerseits die gerinnungsunterdrückende Wirkung des Heparins bzw. der Heparansulfate aufhebt und andererseits spezifische Gerinnungsvorgänge durch adhärierte und somit tertiärstrukturveränderte Plasmaproteine (z.B. Albumin, Fibrinogen, Thrombin) und darauf adhärierende Thrombozyten auslöst.

Somit besteht einerseits ein Zusammenhang zwischen der durch die Immobilisierung eingeschränkten Wechselwirkung der Pentasaccharideinheiten mit Antithrombin III, andererseits kommt es zu Ablagerungen von Plasmaproteinen auf der Heparin- bzw. Heparansulfatschicht auf dem medizinischen Implantat, was zu Verlusten der antithrombotischen Eigenschaften der Beschichtung führt und sich sogar ins Gegenteil umkehren kann, da die innerhalb weniger Sekunden eintretende Plasmaproteinadsorption zum Verlust der antikoagulierenden Oberfläche führt und die adhärierenden Plasmaproteine ihre Tertiärstruktur ändern, wodurch eine thrombogene Oberfläche entsteht. Es konnte überraschenderweise festgestellt werden, dass die Verbindungen der allgemeinen Formel Ia und Ib trotz der strukturellen Unterschiede zum Heparin bzw. Heparansulfat die hemokompatiblen Eigenschaften von Heparin weiterhin aufweisen und zudem bei der Immobilisierung der Verbindungen der allgemeinen Formel Ia oder Ib keine nennenswerten Ablagerungen von Plasmaproteinen, die einen initialen Schritt in der Aktivierung der Gerinnungskaskade darstellen, beobachtet werden konnten. Die hemokompatiblen Eigenschaften der erfindungsgemäßen Verbindungen bleiben auch nach ihrer Immobilisierung auf künstlichen Oberflächen weiterhin erhalten.

Ferner wird vermutet, dass die Sulfatgruppen des Heparins bzw. der Heparansulfate für die Wechselwirkung mit Antithrombin III notwendig sind und dadurch dem Heparin bzw. Heparansulfat die antikoagulatorische Wirkung verleihen. Da durch eine weitgehend vollständige Desulfatierung die Sulfatgruppen bei den Verbindungen der allgemeinen Formel Ib bis auf einen geringen Anteil von unter 0,2 Sulfatgruppen pro Disaccharideinheit entfernt werden, wirken die erfindungsgemäßen Verbindungen nach Formel Ib als auch die Verbindungen nach Formel Ia nicht selbst aktiv gerinnungsunterdrückend, d.h. antikoagulativ.

Die erfindungsgemäßen Verbindungen der allgemeinen Formel Ib können aus Heparin oder Heparansulfaten hergestellt werden, indem zuerst das Polysaccharid im wesentlichen vollständig desulfatiert und danach im wesentlichen vollständig N-acyliert wird. Der Begriff "im wesentlichen vollständig desulfatiert" steht für einen Desulfatierungsgrad von größer 90%, bevorzugt größer 95% und besonders bevorzugt größer 98%. Der Desulfatierungsgrad kann gemäß dem sogenannten Ninhydrintest bestimmt werden, der freie Aminogruppen nachweist. Die Desulfatierung erfolgt in dem Maße, daß mit DMMB (Dimethylmethylenblau) keine Farbreaktion mehr erhalten wird. Dieser Farbtest ist zum Nachweis sulfatierter Polysaccharide geeignet, seine Nachweisgrenze ist in der Fachliteratur jedoch nicht bekannt. Die Desulfatierung kann beispielsweise durch Erhitzen des Pyridiniumsalzes in einem Lösungsmittelgemisch durchgeführt werden. Insbesondere hat sich eine Mischung von DMSO, 1,4-Dioxan und Methanol bewährt.

Heparansulfate sowie Heparin wurden durch Totalhydrolyse desulfatiert und anschließend reacyliert. Danach wurde die Zahl der Sulfatgruppen pro Disaccharideinheit (S/D) mittels C¹³-NMR bestimmt. Die folgende Tabelle 1 gibt diese Resultate am Beispiel von Heparin und desulfatiertem, reacetyliertem Heparin (Ac-Heparin) wieder.

**Tab. 1 : Verteilung der funktionellen Gruppen pro Disaccharideinheit am Beispiel von Heparin und Ac-Heparin bestimmt mittels ¹³C-NMR-Messungen**

| | **2-S** | **6-S** | **3-S** | **NS** | **N-Ac** | **NH**_{**2**} | **S/D** |
|---|---|---|---|---|---|---|---|
| Heparin | 0,63 | 0,88 | 0,05 | 0,90 | 0,08 | 0,02 | 2,47 |
| Ac-Heparin | 0,03 | 0 | 0 | 0 | 1,00 | - | 0,03 |

| | | | | | | | |
|---|---|---|---|---|---|---|---|
| 2-S, 3-S, 6-S: Sulfatgruppen am Position 2, 3 bzw. 6 NS: Sulfatgruppen an den Aminogruppen N-Ac: Acetylgruppen an den Aminogruppen NH₂: freie Aminogruppen S/D: Sulfatgruppen pro Disaccharideinheit | | | | | | | |

Reproduzierbar ergab sich ein Sulfatgehalt von ca. 0,03 Sulfatgruppen/Disaccharideinheit (S/D) beim Ac-Heparin im Vergleich mit ca. 2,5 Sulfatgruppen/Disaccharideinheit beim Heparin

Wie oben beschrieben hat der Unterschied in den Sulfatgehalten von Heparin bzw. Heparansulfaten und denen der Verbindungen der allgemeinen Formeln Ia und Ib einen wesentlichen Einfluss auf die Aktivität gegenüber Antithrombin III und der antikoagulativen Wirkung dieser Verbindungen.

Die Verbindungen der allgemeinen Formeln Ia und Ib weisen einen Gehalt an Sulfatgruppen pro Disaccharideinheit von weniger als 0,2, bevorzugt weniger als 0,07, weiter bevorzugt weniger als 0,05 und insbesondere bevorzugt weniger als 0,03 Sulfatgruppen pro Disaccharideinheit auf.

Durch die Entfernung der Sulfatgruppen des Heparins, denen die aktiven gerinnungsunterdrückenden Wirkungsmechanismen zugeschrieben werden, erhält man ein zur Oberflächenveredelung geeignetes hemokompatibles, gerinnungsinertes Oligo- bzw. Polysaccharid, das einerseits keine aktive Rolle im Gerinnungsprozess mehr einnimmt, und das andererseits vom Gerinnungssystem nicht als Fremdoberfläche erkannt wird. Diese Beschichtung imitiert erfolgreich den von der Natur vorgegebenen Höchststandard der Hemokompatibilität und Passivität gegenüber den gerinnungsaktiven Komponenten des Blutes.

Die Beispiele 5 und 6 verdeutlichen, dass Oberflächen, welche mit den erfindungsgemäßen Verbindungen gemäß den allgemeinen Formeln Ia und/oder Ib beschichtet werden, insbesondere kovalent beschichtet werden, eine passivierende, athrombogene, hemokompatible, antiproliferative und/oder antiinflammatorische Beschichtung ergeben. Dies wird eindeutig am Beispiel des Ac-Heparins belegt.

Im wesentlichen vollständig N-acyliert bezieht sich auf einen N-Acylierungsgrad von größer 94%, bevorzugt größer 97% und besonders bevorzugt größer 98%. Die Acylierung verläuft derart vollständig, daß mit dem Ninhydrinnachweis auf freie Aminogruppen keine Farbreaktion mehr erhalten wird. Als Acylierungsmittel werden bevorzugt Carbonsäurechloride, -bromide oder -anhydride eingesetzt. Essigsäureanhydrid, Propionsäureanhydrid, Buttersäureanhydrid, Essigsäurechlorid, Propionsäurechlorid oder Buttersäurechlorid eigenen sich beispielsweise zur Herstellung der erfindungsgemäßen Verbindungen. Insbesondere eignen sich Carbonsäureanhydride als Acylierungsmitttel.

Als Lösungsmittel insbesondere für die Carbonsäureanhydride wird deionisiertes Wasser verwendet, bevorzugt zusammen mit einem Cosolvens, welches in einer Menge von 10 bis 30 Volumenprozent beigesetzt wird. Als Cosolventien eignen sich Methanol, Ethanol, DMSO, DMF, Aceton, Dioxan, THF, Essigsäureethylester und andere polare Lösungsmittel. Bei der Verwendung von Carbonsäurehalogeniden werden bevorzugt polare wasserfreie Lösungsmittel wie DMSO oder DMF eingesetzt.

Als Lösungsmittel wird deionisiertes Wasser verwendet, bevorzugt zusammen mit einem Cosolvens, welches in einer Menge von 10 bis 30 Volumenprozent beigesetzt wird. Als Cosolventien eignen sich Methanol, Ethanol, DMSO, DMF, Aceton, Dioxan, THF, Essigsäureethylester und andere polare Lösungsmittel.

Die erfindungsgemäßen Verbindungen der allgemeinen Formel Ia weisen an der Hälfte der Zuckermoleküle eine Carboxylatgruppe und an der anderen Hälfte eine N-Acylgruppe auf.

Derartige Verbindungen lassen sich auch aus den Polysacchariden Hyaluronsäure, Dermatansulfat, Chondroitinsulfat herstellen.
Unterschiede zu Heparin und Heparansulfat ergeben sich in der Verknüpfung der Monosaccharide, die hier nicht 1,4-glykosidisch sondern 1,3-glycosidisch vorliegt. Die Disaccharide sind wieder 1,4-gylcosidisch miteinander verknüpft. Bei den ebenfalls in der Blutgerinnung antithrombotisch aktiven Dermatansulfaten [Biochem. J 289, 313-330 (1993)] und bei den Chondroitinsulfaten ist N-Acetylglucosamin durch N-Acetylgalactosamin ersetzt, die sich in der räumlichen Stellung der Hydroxylgruppe am C-Atom 4 voneinander unterscheiden.

Aufgrund ihrer verwandten Struktur werden die Polysaccharide anhand der vorhandenen Unterschiede folgendermaßen zugeordnet:
Typ 1) Uronsäure-Galactosamintyp (HexA-GaIN)n :
   Hierzu gehören Chondroitinsulfat und Dermatansulfat. Typisch für diese Gruppe ist die β-1,3-glycosidische Bindung der Uronsäure an das Galactosamin. Galactosamin ist seinerseits β-1,4-glycosidisch an die nächste Uronsäure gebunden. Dermatansulfat unterscheidet sich von Chondroitinsulfat durch einen hohen Anteil einer weiteren auch bei Heparin und Heparansulfat vorkommenden Uronsäure, der L-Iduronsäure.
   Der Sulfatierungsgrad von Chondroitinsulfat liegt bei 0,1 bis 1,3 Sulfatgruppen pro Disaccharid. Dermatansulfat hat mit 1,0 bis 3,0 Sulfatgruppen pro Disaccharid einen durchschnittlich höheren Sulfatierungsgrad als Chondroitinsulfat und erreicht damit Heparin-ähnliche Werte. Die Aminogruppen sind N-acetyliert.
   Die Desulfatierung und N-Reacylierung führt hier wie im Falle von Heparin und Heparansulfat zu Verbindungen, die ebenfalls für die Verwendung als athrombogenen Beschichtung geeignet sind.
Typ 2) Uronsäure-Glucosamintyp (HexA-GlcN)n:
   Hierzu gehören Heparin, Heparansulfat und Hyaluronan. Heparin und Heparansulfat sind ausschließlich β-1,4-verknüpft, während im Hyaluronan, das ebenfalls diesem Typ zugeordnet ist, die Monosaccharide D-Glucuronsäure und D-Glucosamin β-1,3-verknüpfte Monossaccharide sind. Dieses Polysaccharid besitzt als einziges keine Sulfatgruppen und ist N-acetyliert. Das Molekulargewicht erreicht im Vergleich zu Heparin und Heparansulfat Maximalwerte bis zu 8000 kD. Die Verringerung der Kettenlänge und der Erhalt der Acetylgruppen bzw. die N-Reacylierung führt zu einer sich von der Formel Ib allein in der β-1,3-glycosidischen Verknüpfung der Monosaccharide unterscheidbaren Struktur.

Weitere Verbindungen lassen sich auch aus Chitin oder Chitosan herstellen.

Chitin ist ein stickstoffhaltiges Polysaccharid, dessen monomere Einheiten aus N-Acetyl-D-Glucosamin bestehen, welche β-1,4-glykosidisch verknüpft sind. Dadurch ergeben sich lineare Polymere, die aus ca. 2000 Zuckerbausteinen bestehen und ein Molekulargewicht von ca. 400.000 g/mol aufweisen. Chitin weist eine sehr schlechte Löslichkeit auf und ist in Wasser, organischen Lösungsmitteln sowie verdünnten Säuren oder verdünnten Laugen fast unlöslich. Ein Versetzen mit starken Säuren führt zu einer Hydrolyse, bei der D-Glucosamin und Essigsäure entstehen. Die Behandlung mit starken Laugen führt hingegen zu Chitosan und Acetat.

Chitosan kann leicht durch Verseifung von Chitin gewonnen werden. Chitosan besteht aus β-1,4-glykosidisch verknüpftem Glucosamin (2-Amino-2-deoxy-D-glucose). Chitosan ist aufgrund seiner Filmbildungseigenschaften bekannt und wird zudem als Basismaterial für Ionentauscher und als Mittel zur Senkung des Cholesterinspiegels im Blutserum und zur Gewichtsreduktion eingesetzt.

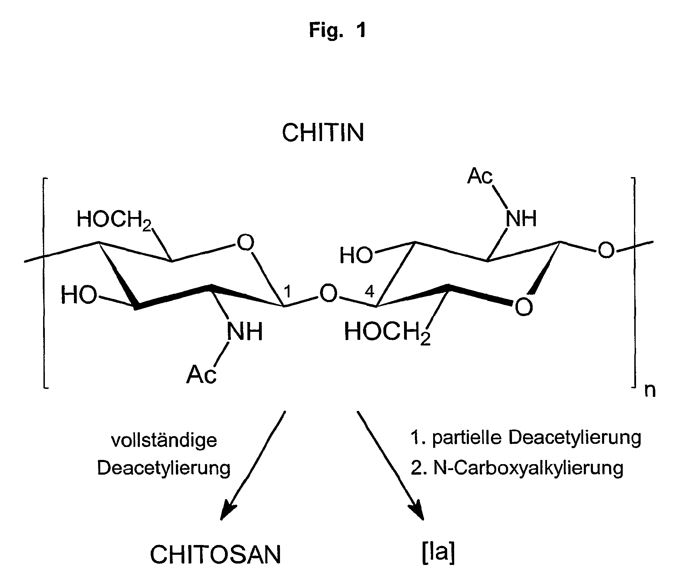

Die erfindungsgemäßen Substanzen der allgemeinen Formel Ia können aus Chitin hergestellt werden, indem Chitin mittels starker Basen teilweise deacetyliert wird und danach die freien Animogruppen monocarboxyalkyliert werden (s. Figur 1). Der Deacetylierungsgrad, d.h. die Menge an demaskierten primären Aminogruppen kann volumetrisch bestimmt werden. Der quantitative Nachweis der freien Aminogruppen erfolgt mittels Ninhydrinreaktion. Je nach Versuchsdurchführung können Deacetylierungsgrade von 20 bis 80% erhalten werden. Bevorzugt sind Deacetylierungsgrade von 40 bis 60%, insbesondere bevorzugt sind 45 bis 55%.

Auf diesem Syntheseweg sind Polysaccharide erhältlich, deren Zuckerbausteine entweder eine N-Acetylgruppe oder eine N-Carboxyalkylgruppe in rein statistischer Verteilung enthalten.

Chitosan, welches durch basische Hydrolyse der N-Acetylgruppen des Chitins leicht zugänglich ist (siehe Fig. 1), dient gleichermaßen als Ausgangsmaterial zur Synthese der Polysaccharide gemäß Formel Ia.

Chitosan besitzt nur sehr wenige N-Acetylgruppen. Somit können die erfindungsgemäßen Verbindungen zum einen dadurch erhalten werden, daß im wesentlichen die Hälfte der freien Aminogruppen in einem ersten Schritt carboxyalkyliert werden und danach die verbleibenden freien Aminogruppen acyliert werden, oder man zuerst die Acylierung durchführt und danach die verbleibenden freien Aminogruppen mit einem geeigneten Carboxyalkylierungsmittel umsetzt. Bevorzugt ist, wenn im wesentlichen die Hälfte der Aminogruppen acyliert und die verbleibende Hälfte carboxyalkyliert wird.

Unter partiell N-acyliertem Chitosan wird ein N-Acylierungsgrad von 30 - 70%, bevorzugt von 40 - 60% und besonders bevorzugt von 45 - 55% verstanden. Insbesondere bevorzugt sind Chitosanderivate, welche im wesentlichen an der Hälfte der Aminogruppen den Rest Y und an der anderen Hälfte der Aminogruppen den Rest Z in einer rein statistischen Verteilung tragen. Der Begriff "im wesentlichen die Hälfte" bedeutet im Idealfall exakt 50%, soll jedoch den Bereich von 45% bis 55% mit umfassen. Der Carboxyalkylierungs- und Acylierungsgrad lassen sich beispielsweise mittels ¹³C-NMR bestimmen (Fehlertoleranz ±3%).

Aufgrund der Tatsache, daß in einem ersten Reaktionsschritt eine bestimmte Anzahl der freien Aminogruppen acyliert oder carboxyalkyliert wird, ergibt sich dadurch zwangsläufig eine vollständig statistische Verteilung der Acylreste bzw. Carboxyalkylreste in dem Polysaccharid der allgemeinen Formel Ia. Die Formel Ia soll daher nur einen Disaccharidbaustein des erfindungsgemäßen Polysaccharides wiedergeben, nicht aber eine alternierende Abfolge der Acylgruppen und Carboxyalkylgruppen festlegen.

Die folgende Abbildung zeigt eine typische Tetrasaccharideinheit eines N-carboxymethylierten, N-acetylierten Chitosans:

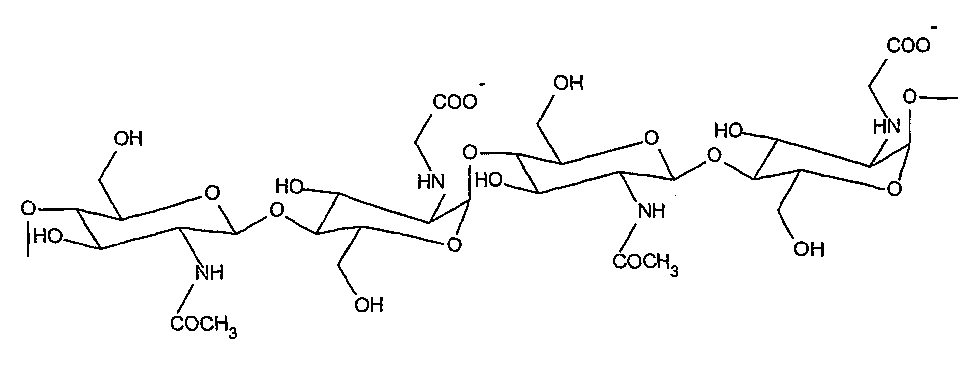

Die vorliegende Erfindung beschreibt die Verwendung der Verbindungen der allgemeinen Formel Ia und/oder Ib sowie Salze dieser Verbindungen für die Beschichtung, insbesondere eine hemokompatible Beschichtung von natürlichen und/oder künstlichen Oberflächen. Unter "hemokompatibel" wird die Eigenschaft der erfindungsgemäßen Verbindungen verstanden, nicht mit den Stoffen des Blutgerinnungssystems oder den Blutplättchen wechselzuwirken und daher nicht die Blutgerinnungskaskade auszulösen.

Zudem offenbart die Erfindung Oligo- und/oder Polysacchariden für die hemokompatible Beschichtung von Oberflächen. Bevorzugt sind Polysaccharide innerhalb der oben genannten Molekulargewichtsgrenzen. Die verwendeten Oligo- und/oder Polysaccharide zeichnen sich dadurch aus, daß sie den Zuckerbaustein N-Acylglucosamin oder N-Acylgalactosamin in großer Menge enthalten. Dies bedeutet, dass 40 bis 60%, bevorzugt 45 - 55% und insbesondere bevorzugt 48 - 52% der Zuckerbausteine N-Acylglucosamin oder N-Acylgalactosamin sind und im wesentlichen die restlichen Zuckerbausteine jeweils einen Carboxylrest aufweisen. Die Oligo- und/oder Polysaccharide bestehen somit gewöhnlich zu über 95%, bevorzugt zu über 98%, aus nur zwei Zuckerbausteinen, wobei ein Zuckerbaustein einen Carboxylrest und der andere einen N-Acylrest trägt.

Ein Zuckerbaustein der Oligo- und/oder Polysaccharide ist N-Acylglucosamin bzw. N-Acylgalactosamin., bevorzugt N-Acetylglucosamin bzw. N-Acetylgalactosamin, und bei dem anderen handelt es sich um Uronsäuren, bevorzugt um Glucuronsäure und Iduronsäure.

Bevorzugt sind Oligo- und/oder Polysaccharide, welche im wesentlichen aus dem Zucker Glucosamin bzw. Galactosamin bestehen, wobei im wesentlichen die Hälfte der Zuckerbausteine eine N-Acylgruppe trägt, bevorzugt eine N-Acetylgruppe, und die andere Hälfte der Glucosaminbausteine eine über die Aminogruppe direkt oder über eine oder mehrere Methylenylgruppen gebundene Carboxylgruppe trägt. Bei diesen an die Aminogruppe gebundenen Carbonsäurereste handelt es sich bevorzugt um Carboxymethyl- oder Carboxyethylgruppen. Ferner sind Oligo- und/oder Polysaccharide bevorzugt, welche im wesentlichen zur Hälfte, d.h. zu 48 - 52%, bevorzugt zu 49 - 51 % und insbesondere bevorzugt zu 49,5 - 50,5%, aus N-Acylglucosamin bzw. N-Acylgalactosamin, bevorzugt aus N-Acetylglucosamin oder N-Acetylgalactosamin und im wesentlichen zur anderen Hälfte aus einer Uronsäure, bevorzugt Glucuronsäure und Iduronsäure, bestehen. Besonders bevorzugt sind die Oligo- und/oder Polysaccharide, welche eine im wesentlichen alternierende Abfolge (d.h. abgesehen von der statistischen Fehlerrate bei der alternierenden Verknüpfung) der beiden Zuckerbausteine aufweisen. Die Rate der Fehlverknüpfungen sollte unter 1%, bevorzugt unter 0,1% liegen.

Überraschenderweise hat sich gezeigt, daß sich für die erfindungsgemäßen Verwendungen insbesondere im wesentlichen desulfatiertes und im wesentlichen N-acyliertes Heparin sowie partiell N-carboxyalkyliertes und N-acyliertes Chitosan als auch desulfatiertes und im wesentlichen N-acyliertes Dermatansulfat, Chondroitinsulfat und auch in der Kettenlänge reduzierte Hyaluronsäure besonders gut eignen. Insbesondere sind für die hemokompatible Beschichtung N-acetyliertes Heparin sowie partiell N-carboxymethyliertes und N-acetyliertes Chitosan geeignet.

Die durch "im wesentlichen" definierten Desulfatierungs- und Acylierungsgrade wurden bereits weiter oben definiert. Der Begriff "im wesentlichen" soll verdeutlichen, daß statistische Abweichungen zu berücksichtigen sind. Eine im wesentlichen alternierende Abfolge der Zuckerbausteine besagt, daß in der Regel keine zwei gleichen Zuckerbausteine aneinander gebunden sind, schließt aber eine derartige Fehlverknüpfung nicht vollkommen aus. Entsprechend bedeutet "im wesentlichen zur Hälfte" annähernd 50%, läßt aber geringe Schwankungen zu, da gerade bei biosynthetisch hergestellten Makromolekülen nie der Idealfall erreicht wird und immer gewisse Abweichungen berücksichtigt werden müssen, da Enzyme nicht perfekt arbeiten und bei der Katalyse mit einer gewissen Fehlerrate zu rechnen ist. Im Falle des natürlichen Heparins liegt hingegen eine streng alternierende Abfolge von N-Acetylglucosamin- und Uronsäureeinheiten (statt Glucuron-) vor.

Des weiteren wird ein Verfahren zur hemokompatiblen Beschichtung von Oberflächen offenbart, welche für den direkten Blutkontakt bestimmt sind. Bei diesem Verfahren wird eine natürliche und/oder künstliche Oberfläche bereitgestellt und die oben beschriebenen Oligo- und/oder Polysaccharide werden auf dieser Oberfläche immobilisiert.

Die Immobilisierung der Oligo- und/oder Polysaccharide auf diesen Oberflächen kann mittels hydrophober Wechselwirkungen, van der Waals Kräften, elektrostatischer Wechselwirkungen, Wasserstoffbrücken, ionischer Wechselwirkungen, Quervernetzung der Oligo- und/oder Polysaccharide und/oder durch kovalente Bindung an die Oberfläche bewirkt werden. Bevorzugt ist die kovalente Verknüpfung der Oligo- und/oder Polysaccharide (side-on Bindung), mehr bevorzugt die kovalente Einzelpunktverknüpfung (side-on Bindung) und insbesondere bevorzugt die kovalente Endpunktverknüpfung (end-on Bindung).

Dabei können beliebige natürliche und/oder künstliche Oberflächen von Medizinprodukten eingesetzt werden, beispielsweise Oberflächen von Prothesen, Organe, Gefäße, Aorten, Herzklappen, Schläuchen, Organersatzteilen, Implantaten, Fasern, Hohlfasern, Stents, Kanülen, Spritzen, Membranen, Konserven, Blutbehältern, Titerplatten, Herzschrittmachern, Adsorbermedien, Chromatographiemedien, Chromatographiesäulen, Dialysatoren, Verbindungsstücke, Sensoren, Ventile, Zentrifugenkammern, Wärmeaustauschem, Endoskope, Filter, Pumpenkammern sowie andere Oberflächen, welche hemokompatible Eigenschaften aufweisen sollen. Der Begriff Medizinprodukte ist weit zu fassen und bezeichnet insbesondere die Oberflächen solcher Produkte, die kurzzeitig (z.B. Endoskope) oder dauerhaft (z.B. Stents) mit Blut in Kontakt kommen.

Im folgenden werden die erfindungsgemäßen Beschichtungsverfahren beschrieben. Biologische und/oder künstliche Oberflächen von Medizinprodukten können nach folgendem Verfahren mit einer hemokompatiblen Beschichtung versehen werden:
a) Bereitstellen einer Oberfläche eines Medizinprodukts und
b) Aufbringen mindestens eines erfindungsgemäßen Oligo- und/oder Polysaccharids nach Formel Ia oder Ib, und/oder
   mindestens eines Oligo- und/oder Polysaccharids, welches zwischen 40% und 60% den Zuckerbaustein N-Acylglucosamin oder N-Acylgalactosamin enthält und im wesentlichen die restlichen Zuckerbausteine einen Carboxylrest pro Zuckerbaustein aufweisen.

Unter "Aufbringen" soll die zumindest teilweise Beschichtung einer Oberfläche mit den entsprechenden Verbindungen verstanden werden, wobei die Verbindungen auf und/oder in die darunterliegende Oberfläche auf- und/oder eingebracht und/oder immobilisiert oder sonstwie verankert werden.

Unter "im wesentlichen die restlichen Zuckerbausteine" ist zu verstehen, dass 93% der restlichen Zuckerbausteine, bevorzugt 96% und insbesondere bevorzugt 98% der restlichen 60% - 40% der Zuckerbausteine einen Carboxylrest tragen.

Bevorzugt wird eine unbeschichtete und/oder nicht hemokompatible Oberfläche bereitgestellt. Unter "nicht hemokompatible" Oberflächen sollen solche Oberflächen verstanden werden, welche das Blutgerinnungssystem aktivieren können, also mehr oder minder thrombogen sind.

Eine alternative Ausführungsform umfaßt die Schritte:
a) Bereitstellen einer Oberfläche eines Medizinprodukts und
b') Aufbringen einer biostabilen Schicht auf die Oberfläche des Medizinprodukts.
oder
a) Bereitstellen einer Oberfläche eines Medizinprodukts und
b) Aufbringen mindestens eines erfindungsgemäßen Oligo- und/oder Polysaccharids nach Formel Ia oder Ib,
   und/oder
   mindestens eines Oligo- und/oder Polysaccharids, welches zwischen 40% und 60% den Zuckerbaustein N-Acylglucosamin oder N-Acylgalactosamin enthält und im wesentlichen die restlichen Zuckerbausteine einen Carboxylrest pro Zuckerbaustein aufweisen.
b') Aufbringen einer biostabilen Schicht auf die Oberfläche des Medizinprodukts und
d') Aufbringen einer weiteren hemokompatiblen Schicht aus mindestens einem erfindungsgemäßen Oligo- und/oder Polysaccharid nach Formel Ia oder Ib, und/oder
   mindestens einem Oligo- und/oder Polysaccharids, welches zwischen 40% und 60% den Zuckerbaustein N-Acylglucosamin oder N-Acylgalactosamin enthält und im wesentlichen die restlichen Zuckerbausteine einen Carboxylrest pro Zuckerbaustein aufweisen.

Die letztgenannte Ausführungsform stellt selbst bei mechanischen Beschädigung der Polymerschicht und damit auch der äußeren hemokompatiblen Schicht, wie sie beispielsweise bei unsachgemäßem Transport oder durch erschwerte Umstände während der Implantation, sicher, dass die Oberflächenbeschichtung ihre Eigenschaft, blutverträglich zu sein, nicht verliert.

Unter "biologische und künstliche" Oberfläche ist die Kombination eines künstlichen Medizinprodukts mit einem künstlichen Teil zu verstehen, z.B. ein Schweineherz mit einer künstlichen Herzklappe
Die einzelnen Schichten werden bevorzugt durch Tauch- oder Sprühverfahren aufgetragen, wobei man zugleich mit der Auftragung einer Schicht auch einen oder mehrere Wirkstoffe auf die Medizinproduktoberfläche aufbringen kann, welche(r) dann in der jeweiligen Schicht kovalent und/oder adhäsiv gebunden enthalten sind(ist). So kann zugleich mit dem Aufbringen einer hemokompatiblen Schicht auf dem Medizinprodukt einer oder mehrere Wirkstoffe aufgebracht werden.

Die Wirkstoffe, sowie die Substanzen, welche für eine biostabile oder bioabbaubare Schicht eingesetzt werden können, sind weiter unter aufgeführt.

Auf diese erste biostabile oder hemokompatible Schicht kann dann in einem weiteren nicht zwingenden Schritt c) eine Wirkstoffschicht aus einem oder mehreren Wirkstoffen aufgetragen werden. In einer bevorzugten Ausführungsform wird der oder die Wirkstoffe kovalent an die darunterliegende Schicht gebunden. Auch der Wirkstoff wird bevorzugt im Tauch- oder Sprühverfahren aufgetragen.

Nach dem Schritt b) oder dem Schritt c) kann ein weiterer Schritt d) folgen, welcher das Aufbringen mindestens einer bioabbaubaren Schicht und/oder mindestens einer biostabilen Schicht auf die hemokompatible Schicht bzw. die Wirkstoffschicht umfaßt.

Entsprechend der alternativen Ausführungsform kann nach Schritt b') oder Schritt c) ein Schritt d') folgen, welcher das Aufbringen oder Immobilisieren mindestens eines erfindungsgemäßen Oligo- und/oder Polysaccharids gemäß Formel Ia oder Ib und/oder mindestens eines Oligo- und/oder Polysaccharids, welches zwischen 40% und 60% den Zuckerbaustein N-Acylglucosamin oder N-Acylgalactosamin enthält und im wesentlichen die restlichen Zuckerbausteine einen Carboxylrest pro Zuckerbaustein aufweisen, als hemokompatible Schicht, umfaßt. Bevorzugt folgt nach Schritt b') der Schritt d').

Nach Schritt d) bzw. d') kann das Aufbringen einer eventuell weiteren Wirkstoffschicht aus einem oder mehreren Wirkstoffen in oder auf die darunterliegende bioabbaubare und/oder biostabile Schicht oder hemokompatible Schicht erfolgen.

Zusätzlich zu den aufgebrachten Wirkstoffschichten können die biostabilen, bioabbaubaren und/oder hemokompatiblen Schichten weitere Wirkstoffe enthalten, welche zusammen mit den biostabilen oder/und bioabbaubaren Substanzen oder der hemokompatiblen Oligo- und/oder Polysacchariden auf das Medizinprodukt aufgebracht worden und in den jeweiligen Schichten enthalten sind.

Gemäß einer bevorzugten Ausführungsform wird die biostabile Schicht kovalent oder /und adhäsiv an die Oberfläche des Medizinprodukts gebunden und vollständig oder unvollständig mit einer hemokompatiblen Schicht überzogen, Welche (bevorzugt kovalent) an die biostabile Schicht gebunden wird.

Bevorzugt besteht die hemokompatible Schicht aus Heparin nativen Ursprungs regioselektiv hergestellter Derivate unterschiedlicher Sulfatierungsgrade und Acylierungsgrade im Molekulargewichtsbereich des für die antithrombotische Wirkung verantwortlichen Pentasaccharides bis zum Standardmolekulargewicht des käuflichen Heparins von ca. 13kD, Heparansulfate und seine Derivate, Oligo- und Polysaccharide der Erythrocytenglycocalix, desulfatiertes und N-reacetyliertes Heparin, N-carboxymethyliertes und/oder partiell N-acetyliertes Chitosan sowie aus Mischungen dieser Substanzen.

Gegenstand der Erfindung sind auch die Medizinprodukte, welche nach einem der hierin genannten Verfahren hemokompatibel beschichtet worden sind.

Des weiteren sind Medizinprodukte Gegenstand dieser Erfindung, wobei die Oberfläche der Medizinprodukte direkt oder über mindestens eine dazwischenliegende biostabile und/oder bioabbaubare Schicht und/oder Wirkstoffschicht mit einer hemokompatiblen Schicht überzogen ist, welche aus mindestens einem Oligo- und/oder Polysaccharid besteht, welches zwischen 40% und 60% den Zuckerbaustein N-Acylglucosamin oder N-Acylgalactosamin enthält und im wesentlichen die restlichen Zuckerbausteine einen Carboxylrest pro Zuckerbaustein aufweisen.

Gemäß einer bevorzugten Ausführungsform befindet sich unter der hemokompatiblen Schicht aus den vorgenannten Oligo- und/oder Polysacchariden mindestens eine biostabile Schicht, welche zudem bevorzugt kovalent an die Oberfläche des Medizinprodukts gebunden ist.

Ferner ist bevorzugt, wenn sich auf der hemokompatiblen Schicht mindestens eine biostabile und/oder mindestens eine bioabbaubare Schicht befindet, welche die hemokompatible Schicht vollständig oder unvollständig überzieht. Insbesondere bevorzugt ist eine die hemokompatible Schicht überziehende bioabbaubare Schicht.

Eine weitere bevorzugte Ausführungsform weist zwischen der biostabilen unteren Schicht und der darüberliegenden hemokompatiblen Schicht eine Wirkstoffschicht aus mindestens einem antiproliferativen, antiinflammatorischen und/oder antithrombotischen Wirkstoff auf, der kovalent und/oder adhäsiv an die biostabile und/oder hemokompatible Schicht gebunden ist. Alternativ zur Wirkstoffschicht oder zusätzlich zur Wirkstoffschicht kann die biostabile untere und/oder die hemokompatible obere Schicht weitere Wirkstoffe enthalten, welche bevorzugt zusammen mit dem Aufbringen der jeweiligen Schicht aufgetragen werden.

Grundsätzlich kann jede Schicht, d.h. eine biostabile Schicht, eine bioabbaubare Schicht und eine hemokompatible Schicht einen oder mehrere antiproliferative, antiinflammatorische und/oder antithrombotische Wirkstoffe enthalten und zudem können sich zwischen den vorgenannten Schichten noch Wirkstoffschichten aus einem oder mehreren Wirkstoffen befinden. Bevorzugt sind Beschichtungssysteme aus zwei Schichten, einer biostabilen und einer hemokompatiblen Schicht, wobei die hemokompatible Schicht die äußere Schicht ist und beide Schichten einen oder mehrere Wirkstoffe enthalten können. Ferner ist bevorzugt, wenn auf oder unter der hemokompatiblen Schicht sich eine Wirkstoffschicht aus einem oder mehreren Wirkstoffen befindet. Auch bevorzugt sind Dreischichtsysteme, welche aus einer biostabilen, bioabbaubaren und hemokompatiblen Schicht bestehen. Dabei ist vorzugsweise die unterste Schicht eine biostabile Schicht. Zusätzlich sind ein oder zwei Wirkstoffschichten möglich. Es ist auch möglich, zwei Wirkstoffschichten direkt übereinander aufzutragen. In einer weiteren bevorzugten Ausführungsform, wird zumindest ein Wirkstoff kovalent an oder in einer Schicht gebunden.

Bevorzugt werden bei den Verfahren zur Beschichtung und auf den Medizinprodukten als Wirkstoffe Tacrolimus, Pimecrolimus, Pl 88, Thymosin a-1, PETN (Pentaerythrittetranitrat), Baccatin und seine Derivate, Docetaxel, Colchicin, Paclitaxel und seine Derivate, Trapidil, α- und β-Estradiol, Dermicidin, Tialin (2-Methylthiazolidin-2,4-dicarbonsäure), Tialin-Na (Natriumsalz von Tialin), Simvastatin, Macrocyclisches Kohlensuboxid (MCS) und dessen Derivate, Sirolimus, Tyrphostine, D24851, Colchicin, Fumarsäure und ihre Ester, aktiviertes Protein C, Interleukin 1β-Inhibitoren und Melanocyte-stimulating hormon (α-MSH) sowie Mischungen dieser Wirkstoffe verwendet.

Die natürlichen und/oder künstlichen Oberflächen der Medizinprodukte, welche nach den hierin beschriebenen Verfahren mit einer hemokompatiblen Schicht der erfindungsgemäßen Oligo- und/oder Polysaccharide bzw. den Oligo- und/oder Polysacchariden, welche zwischen 40% und 60% den Zuckerbaustein N-Acylglucosamin oder N-Acylgalactosamin enthalten und im wesentlichen die restlichen Zuckerbausteine einen Carboxylrest pro Zuckerbaustein aufweisen, überzogen sind, eignen sich insbesondere als Implantate bzw. Organersatzteile, welche in direktem Kontakt mit dem Blutkreislauf und Blut stehen. Die erfindungsgemäß beschichteten Medizinprodukte eignen sich insbesondere, jedoch nicht nur, für den direkten und dauerhaften Blutkontakt, sondern zeichnen sich überraschenderweise auch durch die Eigenschaft aus, die Anhaftung von Proteinen auf derartig beschichteten Oberflächen zu verringern oder gar zu verhindern.
Die Ablagerung von Plasmaproteinen auf Fremdoberflächen in Kontakt mit Blut ist ein essentieller und initialer Schritt für das weitere Geschehen in Bezug auf die Erkennung und die einsetzenden Maßnahmen von Seiten des Blutsystems.

Dies ist beispielsweise wichtig bei der in vitro Diagnostik aus Körperflüssigkeiten. Somit verhindert oder zumindest verringert das Aufbringen der erfindungsgemäßen Beschichtung beispielsweise auf Mikrotiterplatten oder anderen Trägermedien, welche für diagnostische Nachweisverfahren eingesetzt werden, die unspezifische Ablagerung von Proteinen, welche die in der Regel empfindlichen Nachweisreaktionen stören und zu einer Verfälschung des Analyseresultates führen können.

Durch die Verwendung der erfindungsgemäßen Beschichtung auf Adsorbermedien oder Chromatographiemedien wird ebenfalls die unspezifische Ablagerung von Proteinen verhindert oder verringert, wodurch bessere Trennungen erreicht werden und Produkte von größerer Reinheit gewonnen werden können.

Insbesondere werden Stents nach den erfindungsgemäßen Verfahren beschichtet. In den letzten Jahren hat das Einsetzen von Stents bei der Ballondilatation von verschlossenen Blutgefäßen immer weitere Verbreitung gefunden. Obwohl Stents das Risiko eines erneuten Gefäßverschlusses verkleinern, sind sie bisher nicht in der Lage, solche Restenosen vollständig zu verhindern.
Eine genaue begriffliche Beschreibung der Restenose ist in der Fachliteratur nicht aufzufinden. Die am häufigsten verwendete morphologische Definition der Restenose ist diejenige, die nach erfolgreicher PTA (perkutane transluminale Angioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.

Für die durch den Stent verursachte Restenose gibt es zwei verschiedene Ursachen:
a.) In der ersten Zeit nach der Implantation ist die Oberfläche des Stents dem Blut direkt ausgesetzt und es kann aufgrund der nun vorhandenen Fremdoberfläche zu einer akuten Thrombose kommen, die das Blutgefäß wieder verschließt.
b.) Mit Implantation des Stent werden Gefäßverletzungen verursacht, die Entzündungsreaktionen hervorrufen, die für den Heilungsprozeß in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einem erneuten Verschluß des Gefäßes aufgrund unkontrollierten Wachstums führen.
c.) Nach einigen Wochen beginnt der Stent in das Gewebe des Blutgefäßes einzuwachsen. Das heißt, daß der Stent vollständig von glatten Muskelzellen umhüllt wird und keinen Kontakt mehr zum Blut hat. Diese Vernarbung kann zu stark ausgeprägt sein (Neointimahyperplasie) und dazu führen, daß nicht nur die Stentoberfläche bedeckt, sondern der ganze Innenraum des Stents verschlossen wird.

Man hat vergeblich versucht, das Problem der Restenose durch die Beschichtung der Stents mit Heparin zu lösen (J. Whörle et al, European Heart Journal (2001) 22 1808-1816). Heparin adressiert als Antikoagulanz jedoch nur die erstgenannte Ursache und kann darüber hinaus nur in Lösung seine volle Wirkung entfalten. Dieses erste Problem läßt sich mittlerweile medikamentös durch die Gabe von Antikoagulantien fast vollständig vermeiden. Das zweite und dritte Problem versucht man zur Zeit zu lösen, indem man das Wachstum der glatten Muskelzellen lokal am Stent hemmt. Das wird z.B. mit radioaktiven Stents oder mit Stents versucht, welche pharmazeutische Wirkstoffe enthalten.

So offenbart US-A-5 891 108 beispielsweise einen hohl ausgeformten Stent, welcher in seinem Inneren pharmazeutische Wirkstoffe enthalten kann, welche durch eine Vielzahl von Öffnungen im Stent freigesetzt werden. EP-A-1 127 582 beschreibt hingegen einen Stent, der auf seiner Oberfläche Einbuchtungen von 0,1 - 1 mm Tiefe und 7-15 mm Länge aufweist, welche zur Aufnahme eines Wirkstoffes geeignet sind. Diese Wirkstoffreservoire setzen, vergleichbar der Öffnungen in dem hohlen Stent, den enthaltenen pharmazeutischen Wirkstoff punktuell in hoher Konzentration und über einen relativ langen Zeitraum frei, was aber dazu führt, daß die glatten Muskelzellen nicht mehr oder nur sehr verzögert in der Lage sind, den Stent zu umhüllen. Daher ist der Stent viel länger dem Blut ausgesetzt, was wieder vermehrt zu Gefäßverschlüssen durch Thrombosen führt (Liistro F., Colombo A., Late acute thrombosis after Paclitaxel eluting stent implantation. Heart (2001) 86 262-4).

Ein Lösungsversuch für dieses Problem stellt die Phosphorylcholinbeschichtung von Biocompatibles (WO 0101957) dar, indem hier Phosphorylcholin, eine Zellmembrankomponente der Erythrocyten, als Bestandteil der aufgebrachten nicht bioabbaubaren Polymerschicht auf dem Stent eine nichtthrombogene Oberfläche erzeugen soll. Dabei wird der Wirkstoff abhängig vom Molekulargewicht von dieser polymerhaltigen Phosphorylcholinschicht absorbiert oder auf der Oberfläche adsorbiert.

Die erfindungsgemäßen Stents sind mit einer hemokompatiblen Schicht überzogen und besitzen eine oder mehrere weitere Schichten, die mindestens einen antiproliferativen oder/und entzündungshemmenden und bei Bedarf auch antithrombotischen Wirkstoff enthalten.
Die hemokompatible Beschichtung eines Stents sorgt für die notwendige Blutverträglichkeit und der Wirkstoff (oder Wirkstoffkombination), der vorzugsweise über die Gesamtoberfläche des Stents gleichmäßig verteilt ist, bewirkt, daß der Bewuchs der Stentoberfläche mit Zellen, insbesondere glatte Muskel- und Endothelzellen, in einer kontrollierten Weise abläuft. Es findet somit keine rasche Besiedelung und Überwucherung der Stentoberfläche mit Zellen statt, was zu einer Restenose führen könnte, wohingegen der Bewuchs der Stentoberfläche mit Zellen aber auch nicht durch eine hohe Medikamentenkonzentration vollkommen unterbunden wird, was die Gefahr einer Thrombose mit sich bringt.

Somit gewährleistet die Einbettung von Wirkstoffen, dass der Wirkstoff oder die Wirkstoffkombination, kovalent oder/und adhäsiv an die darunterliegende Schicht gebunden oder/und kovalent oder/und adhäsiv in die Schicht eingelagert, kontinuierlich und in geringen Dosen freigesetzt wird, so dass nicht die Besiedelung der Stentoberfläche mit Zellen unterbunden, jedoch eine Überbesiedelung verhindert wird. Diese Kombination beider Effekte verleiht dem erfindungsgemäßen Stent die Fähigkeit, schnell in die Gefäßwand einzuwachsen und vermindert sowohl das Risiko einer Restenose als auch das Risiko einer Thrombose. Die Freisetzung des oder der Wirkstoffe erstreckt sich über einen Zeitraum von 1 bis 12 Monaten, bevorzugt über 1 - 3 Monate nach Implantation.

Als Wirkstoffe werden antiproliferative Substanzen, antiphlogistische als auch antithrombotische Stoffe eingesetzt. Als antiproliferative Wirkstoffe werden bevorzugt Cytostatika, Makrolidantibiotika, und/oder Statine eingesetzt. Anwendbare antiproliferative Wirkstoffe sind Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastätin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Betulinsäure, Camptothecin, Lapachol, β-Lapachon, Podophyllotoxin, Betulin, Tropfosfamid, Podophyllsäure-2-ethylhydrazid, Ifosfamid, Chlorambucil, Bendamustin, Dacarbazin, Busulfan, Procarbazin, Treosulfan, Tremozolomid, Melanocyte-stimulating hormon (α-MSH), Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat Oxaceprol, Celecoxib, β-Sitosterin, Ademetionin, Myrtecain, Polidocanol, Nonivamid, Levomenthol, Benzocain, Aescin, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, trastuzumab, Exemestan, Letrozol, Goserelin, Chephalomannin, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense Selectin (Cytokinantagonist) CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, Folsäure und Derivate, Vitamine der B-Reihe, Vitamin D-Derivate wie z.B. Calcipotriol und Tacalcitol, D24851, Thymosin α-1, Fumarsäure und ihre Derivate wie z.B. Dimethylfumarat, IL-1β-Inhibitor, Colchicin, NO-Donoren wie Pentaerythrityltetranitrat und Syndnoeimine, S-Nitrosoderivate, Tamoxifen, Staurosporin, β-Estradiol, a-Estradiol, Estron, Estriol, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate (6-α-Hydroxy-Paclitaxel, Baccatine u.a.), synthetisch hergestellte als auch aus nativen Quellen gewonnene macrocyclische Oligomere des Kohlensuboxids (MCS) und seine Derivate, Molgramostim (rhuGM-CSF), Peginterferon α-2b, Lanograstim (r-HuG-CSF), Filgrastim, Macrogol, Dacarbazin, Letrozol, Goserelin, Chephalomannin, Trastuzumab, Exemestan, Basiliximab, Daclizumab, Ellipticin, D-24851 (Calbiochem), Colcemid, Cytochalasin A-E, Indanocine, Nocadazole, S 100 Protein, Pl-88, Melanocyte Stimulating Hormon (α-MSH), Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Interleukin 1β-Inhibitoren, Antisense Oligonucleotide, VEGF-Inhibitoren, IGF-1 genannt.

Aus der Gruppe der Antibiotika finden des weiteren Cefadroxil, Cefazolin, Cefaclor, Cefotixin, Tobramycin, Gentamycin Anwendung. Positiven Einfluß auf die postoperative Phase haben auch Penicilline wie Dicloxacillin, Oxacillin, Sulfonamide, Metronidazol, Antithrombotika wie Argatroban, Asprin, Abciximab, synthetisches Antithrombin, Bivalirudin, Coumadin, Dermicidin, Enoxoparin, Hemoparin, Gewebe-Plasminogen-Aktivator, GpIIb/IIIa-Plättchenmembranrezeptor, Faktor Xₐ-Inhibitor, aktiviertes Protein C, Dermicidin, Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Caspaseinhibitoren, Apoptoseinhibitoren, Apoptoseregulatoren wie p65, NF-kB und Bcl-xL-Antisense-Oligonukleotiden und Prostacyclin, Vapiprost, α,β- und γ-Interferon, Histaminantagonisten, Serotoninblocker, Halofuginon, Nifedipin, Tocopherol, Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron. Weitere Wirkstoffe sind Steroide (Hydrocortison, Betamethason, Dexamethason), nichtsteroidale Substanzen (NSAIDS) wie Fenoporfen, Ibuprofen, Indomethacin, Naproxen, Phenylbutazon und andere. Antivirale Agentien wie Acyclovir, Ganciclovir und Zidovudin sind ebenfalls einsetzbar. Verschiedene Antimykotika finden Anwendung in diesem Bereich. Beispiele sind Clotrimazol, Flucytosin, Griseofulvin, Ketoconazol, Miconazol, Nystatin, Terbinafin. Antiprozoale Agentien wie Chloroquin, Mefloquin, Quinin sind gleichermassen wirksame Agentien, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3, Tubeimosid, Bruceanole A,B,C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, 1,11-Dimethoxycanthin-6-on, 1-Hydroxy-11-Methoxycanthin-6-on, Scopolectin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-4,16-dien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B, weitere natürliche Terpenoide wie Hippocaesculin, 14-Dehydroagrostistachin, Agroskerin, Agrostistachin, 17-Hydroxyagrostistachin, Ovatodiolide, 4,7-Oxycycloanisomelsäure, Yadanzioside N und P, lsodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin.

Die Wirkstoffe werden einzeln oder kombiniert in gleicher oder unterschiedlicher Konzentration eingesetzt. Besonders bevorzugt sind Wirkstoffe, welche neben ihrer antiproliferativen Wirkung auch immunsuppressive Eigenschaften aufweisen. Zu derartigen Wirkstoffen zählen Erythromycin, Midecamycin, Tacrolimus, Sirolimus, Paclitaxel und Josamycin. Zudem bevorzugt ist eine Kombination von mehreren antiproliferativ wirkenden Substanzen oder von antiproliferativen Wirkstoffen mit immunsuppressiven Wirkstoffen.

Bevorzugt für die vorliegende Erfindung sind Tacrolimus, Pimecrolimus, PI 88, Thymosin a-1, PETN, Baccatin und seine Derivate, Docetaxel, Colchicin, Paclitaxel und seine Derivate, Trapidil, α- und β-Estradiol, Dermicidin, Simvastatin Macrocyclisches Kohlensuboxid (MCS) und dessen Derivate, Sirolimus, Tyrphostine, D24851, Colchicin, Fumarsäure und ihre Ester, aktiviertes Protein C, Interleukin 1β-Inhibitoren und Melanocyte-stimulating hormon (α-MSH) und Tialin (2-Methylthiazolidin-2,4-dicarbonsäure) als auch Tialin-Na (Natriumsalz von Tialin).

Der Wirkstoff ist bevorzugt in einer pharmazeutisch aktiven Konzentration von 0,001-10 mg pro cm² Stentoberfläche und pro Wirkstoffschicht oder Wirkstoffenthaltender Schicht enthalten. Weitere Wirkstoffe können in ähnlicher Konzentration in derselben oder in weiteren Schichten enthalten sein.

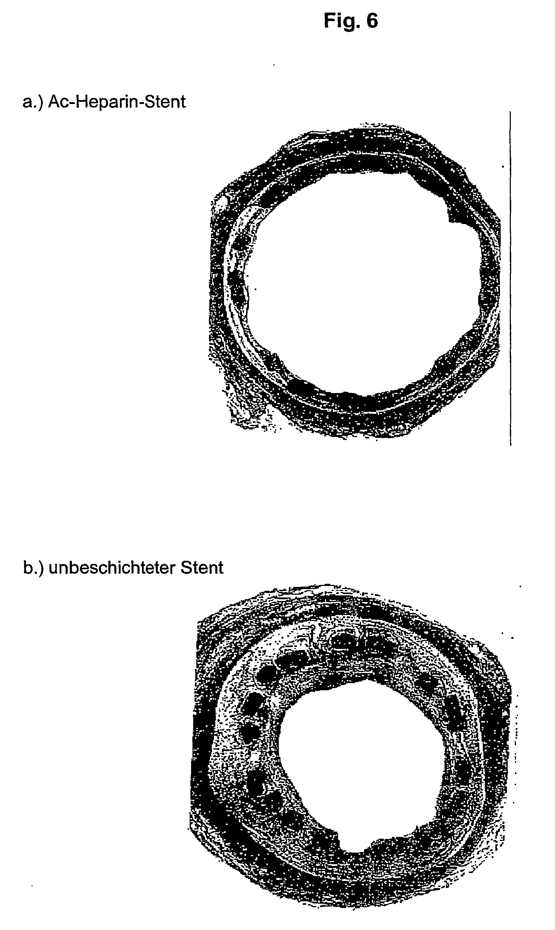

Die erfindungsgemäß beschichteten Medizinprodukte, insbesondere die erfindungsgemäß beschichteten Stents, können den oder die Wirkstoffe kontinuierlich und kontrolliert freisetzen und eignen sich zur Verhinderung oder Reduzierung von Restenose (s. Fig. 6).

Die den Stent unmittelbar bedeckende hemokompatible Schicht besteht bevorzugt aus synthetisch hergestellten Derivaten von Heparin nativen Ursprungs unterschiedlicher Sulfatierungsgrade und Acteylierungsgrade im Molekulargewichtsbereich von des für die antithrombotische Wirkung verantwortlichen Pentasaccharides bis zum Standardmolekulargewicht des käuflichen Heparins als auch Heparansulfaten und dessen Derivate, Oligo- und Polysaccharide der Erythrocytenglycolcalix, die in perfekter Weise die athrombogene Oberfläche der Erythrocyten wiedergeben, da hier im Gegensatz zum Phosphorylcholin der tatsächliche Kontakt von Blut und Erythrocytenoberfläche stattfindet, vollständig desulfatiertem und N-reacetyliertem Heparin, desulfatiertem und N-reacetyliertem Heparin, N-carboxymethyliertem und/oder partiell N-acetyliertem Chitosan, Chitosan und/oder Mischungen dieser Substanzen. Diese Stents mit einer hemokompatiblen, Beschichtung werden hergestellt, indem man herkömmliche in der Regel unbeschichtete Stents bereitstellt und bevorzugt kovalent eine hemokompatible Schicht aufbringt, welche nach Wirkstofffreigabe und damit nach Abklingen des Wirkstoffeinflusses und Abbau der Matrix die Oberfläche des Implantates permanent maskiert.

Die herkömmlichen Stents, welche gemäß der erfindungsgemäßen Verfahren beschichtet werden können, bestehen aus Edelstahl, Nitinol oder anderen Metallen und Legierungen oder aus synthetischen Polymeren.

Eine weitere bevorzugte Ausführungsform der erfindungsgemäßen Stents weist eine Beschichtung auf, welche aus mindestens zwei Schichten besteht. Auch Multischichtsysteme finden Anwendung. Bei derartigen Mehrschichtsystemen wird als erste Schicht diejenige bezeichnet, welche direkt auf den Stent aufgebracht ist. Als zweite Schicht wird diejenige Schicht bezeichnet, welche auf die erste Schicht aufgebracht wird, usw.
Gemäß der Zweischichtausführung besteht die erste Schicht aus einer hemokompatiblen Schicht, welche im wesentlichen vollständig durch eine bioabbaubare Schicht überzogen ist, die mindestens einen antiproliferativen, antiphlogistischen und/oder antithrombotischen Wirkstoff, kovalent oder/und adhäsiv gebunden, enthält. Ebenso werden auch Wirkstoff-Kombinationen eingesetzt, die sich in ihrer Wirkung gegenseitig unterstützen und ergänzen.

Als bioabbaubare Substanzen, welche für die äußere Schicht eingesetzt werden können, zählen Polyvalerolactone, Poly-ε-Decalactone, Polylactonsäure, Polyglycolsäure Polylactide, Polyglycolide, Copolymere der Polylactide und Polyglycolide, Poly-ε-caprotacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-p-dioxanone, Polyanhydride wie Polymaleinsäure-anhydride, Polyhydroxy-methacrylate, Fibrin, Polycyanoacrylate, Polycaprolactondimethylacrylate, Poly-b-Maleinsäure Polycaprolactonbutyl-acrylate, Multiblockpolymere wie z. B. aus Oligocaprolactondiole und Oligodioxanondiole, Polyetherestermultiblockpolymere wie z. B. PEG und Polybutylenterephtalat. Polypivotolactone, Polyglycolsäuretrimethyl-carbonate, Polycaprolactonglycolide, Poly-g-ethylglutamat, Poly(DTH-Iminocarbonat), Poly(DTE-co-DT-carbonat), Poly(Bisphenol A-iminocarbonat), Polyorthoester, Polyglycolsäuretrimethyl-carbonate, Polytrimethylcarbonate Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyurethane mit Aminosäureresten im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen, Collagen-N-Hydroxysuccinimid, Lipide, Phospholipide, Modifikationen und Copolymere und/oder Mischungen der vorgenannten Substanzen eingesetzt.

Die wirkstoffenthaltende Schicht bzw. Schichten wird langsam durch Blutbestandteile abgebaut, so daß der Wirkstoff entsprechend der Geschwindigkeit des Abbaus der äußeren Schicht freigesetzt wird als auch sich entsprechend seines Elutionsverhalten aus der Matrix löst. Die erste hemokompatible Schicht gewährleistet die notwendige Blutverträglichkeit des Stents, sobald die bioabbaubare Schicht degradiert worden ist. Durch diesen biologischen Abbau der äußeren Schicht und die dementsprechende Wirkstofffreisetzung wird ein Anwachsen von Zellen nur für eine gewisse Zeit stark reduziert und ein gezieltes kontrolliertes Anwachsen dort ermöglicht, wo die äußere Schicht bereits weitgehend abgebaut worden ist. Der biologische Abbau der äußeren Schicht erstreckt sich vorteilhafterweise über 1 bis 36 Monate, bevorzugt aber 1 - 6 Monate, besonders bevorzugt 1 - 2 Monate. Es hat sich gezeigt, daß bei derartigen Stents Restenosen verhindert oder zumindest sehr stark reduziert werden. In dieser Zeit spielen sich die wichtigen Heilungsprozesse ab. Schlussendlich bleibt die hemokompatible Schicht als athrombogene Oberfläche erhalten und maskiert die Fremdoberfläche derart, dass weiterhin keine lebensbedrohliche Reaktion eintreten kann.

Die auf die Oberflächen der Medizinprodukte, bevorzugt der Stents, aufgetragene Polymermengen liegen zwischen 0,01 mg bis 3 mg / Schicht, bevorzugt zwischen 0,20 mg bis 1 mg / Schicht und insbesondere bevorzugt zwischen 0,2 mg bis 0,5 mg / Schicht.

Derartige Stents lassen sich durch ein Verfahren zur hemokompatiblen Beschichtung von Stents herstellen, dem folgendes Prinzip zugrundeliegt :
a. Bereitstellen eines unbeschichteten Stents,
b. Aufbringen einer bevorzugt kovalent gebundenen hemokompatiblen Schicht,
c. Diffusion von Wirkstoff in die hemokompatible Schicht, oder
c'. im wesentlichen vollständiges Überziehen der hemokompatiblen Schicht im Tauch- oder Sprühverfahren mit mindestens einen Wirkstoff, oder
c". im wesentlichen vollständiges Überziehen oder/und unvollständiges Überziehen der hemokompatiblen Schicht im Tauch- oder Sprühverfahren mit mindestens einer bioabbaubaren oder/und biostabilen Schicht, welche mindestens einen Wirkstoff enthält oder/und den Wirkstoff selbst darstellt.

Das Beschichtungsprinzip bietet eine große Variationsbreite bezüglich der gestellten Anforderungen an den Wirkstoff und lässt sich in verschiedene Beschichtungstypen aufteilen, die ebenfalls untereinander kombinierbar sind.

### Beschichtungsprinzip I :

a.) Bereitstellen eines unbeschichteten Stents;
b.) Aufbringen einer hemokompatiblen Schicht;
c.) Aufbringen eines Wirkstoffes oder einer Wirkstoffkombination auf die hemokompatible Schicht ohne Matrix;
d.) Aufbringen eines Wirkstoffes oder einer Wirkstoffkombination auf die hemokompatible Schicht ohne Matrix und im wesentlichen vollständiges oder/und unvollständiges Überziehen der Schichten mit einem bioabbaubaren oder/und biostabilen Material zur Diffusionskontrolle.

### Beschichtungsprinzip II :

a.) Bereitstellen eines unbeschichteten Stents;
b.) Aufbringen einer hemokompatiblen Schicht;
c.) im wesentlichen vollständiges Überziehen oder/und unvollständiges Überziehen der hemokompatiblen Schicht mit mindestens einer bioabbaubaren oder/und biostabilen Schicht, welche mindestens einen Wirkstoff kovalent an die hemokompatible Schicht gebunden oder/und adhäsiv enthält;
d.) im wesentlichen vollständiges Überziehen der hemokompatiblen Schicht mit mindestens einer bioabbaubaren oder/und biostabilen Schicht, welche mindestens einen Wirkstoff kovalent an die Matrix gebunden oder/und adhäsiv enthält und eine weitere, die darunterliegende Schicht vollständig oder/und teilweise überdeckende bioabbaubare oder/und biostabile Schicht ohne Wirkstoff als Diffusionsbarriere.

### Beschichtungsprinzip III :

a.) Bereitstellen eines unbeschichteten Stents;
b.) Aufbringen einer hemokompatiblen Schicht;
c.) im wesentlichen vollständiges Überziehen der hemokompatiblen Schicht mit mindestens einer bioabbaubaren oder/und biostabilen Schicht, welche mindestens einen Wirkstoff kovalent gebunden oder/und adhäsiv enthält;
d.) Aufbringen eines Wirkstoffes oder einer Wirkstoffkombination kovalent an die darunterliegende Schicht gebunden oder/und adhäsiv;
e.) im wesentlichen vollständiges Überziehen der hemokompatiblen Schicht mit mindestens einer bioabbaubaren oder/und biostabilen Schicht, welche mindestens einen Wirkstoff, kovalent gebunden oder/und adhäsiv, enthält, Aufbringen eines Wirkstoffes oder einer Wirkstoffkombination und eine weitere, die darunterliegende Schicht vollständig oder/und teilweise überdeckende bioabbaubare oder/und biostabile Schicht ohne Wirkstoff als Diffusionsbarriere.

### Beschichtungsprinzip IV :

a.) Bereitstellen eines unbeschichteten Stents;
b.) Aufbringen einer hemokompatiblen Schicht;
c.) im wesentlichen vollständiges oder/und unvollständiges Überziehen der hemokompatiblen Schicht mit mindestens zwei bioabbaubaren oder/und biostabilen Schichten, welche mindestens, kovalent oder/und adhäsiv, einen Wirkstoff in unterschiedlicher Konzentration je Schicht enthalten;
d.) im wesentlichen vollständiges oder/und unvollständiges Überziehen der hemokompatiblen Schicht mit mindestens zwei bioabbaubaren oder/und biostabilen Schichten, welche mindestens einen Wirkstoff kovalent oder/und adhäsiv in unterschiedlicher Konzentration je Schicht enthalten und mindestens eine weitere, die darunterliegende Schicht vollständig oder/und teilweise überdeckende, bioabbaubare oder/und biostabile Schicht ohne Wirkstoff als Diffusionsbarriere;
e.) im wesentlichen vollständiges oder/und unvollständiges Überziehen der hemokompatiblen Schicht mit mindestens einer bioabbaubaren oder/und biostabilen Schicht, welche mindestens einen Wirkstoff oder/und mindestens einen weiteren Wirkstoff derselben Gruppe oder aus einer anderen Gruppe mit komplementären Eigenschaften in gleicher oder unterschiedlicher Konzentrationen in kovalenter oder/und adhäsiver Form enthält;
f.) im wesentlichen vollständiges oder/und unvollständiges Überziehen der hemokompatiblen Schicht mit mindestens zwei bioabbaubaren oder/und biostabilen Schichten, welche mindestens einen Wirkstoff oder/und mindestens einen weiteren Wirkstoff derselben Gruppe oder aus einer anderen Gruppe mit komplementären Eigenschaften in gleicher oder unterschiedlicher Konzentrationen enthalten und mindestens eine weitere, die darunterliegende Schicht vollständig oder/und teilweise überdeckende, bioabbaubare oder/und biostabile Schicht ohne Wirkstoff als Diffusionsbarriere;
g.) im wesentlichen vollständiges Überziehen der hemokompatiblen Schicht mit mindestens zwei bioabbaubaren oder/und biostabilen Schichten, welche mindestens einen Wirkstoffen, kovalent oder/und adhäsiv, in gleicher oder/und unterschiedlichen Konzentrationen enthalten und eine weitere, die darunterliegende Schicht vollständig oder auch nur teilweise überdeckende, bioabbaubare oder/und biostabile Schicht ohne Wirkstoff Diffusionsbarriere und einer diese Schicht überziehenden Wirkstoffschicht bestehend aus mindestens einem Wirkstoff kovalent an die darunterliegende Matrix gebunden oder/und adhäsiv ohne Träger.

Eine weitere vorteilhafte Ausführungsform stellt ein Stent mit einer mindestens dreilagigen Beschichtung dar, wobei die erste Schicht die Oberfläche des Stents mit der hemokompatiblen Schicht bedeckt, die zweite Schicht den Wirkstoff enthält und nicht bioabbaubar ist und mit einer dritten hemokompatiblen Schicht überzogen worden ist. Hierbei verleiht die äußere Schicht dem Stent die notwendige Blutverträglichkeit und die zweite Schicht dient als Wirkstoffreservoir. Der Wirkstoff, je nach Bedarf über eine hydrolyselabile Bindung kovalent an die Matrix gebunden oder/und der, für das Beschichtungsverfahren notwendig, in Lösungsmittel gelösten Matrix beigefügt, wird daher kontinuierlich und in geringen Konzentrationen aus der zweiten Schicht freigesetzt und diffundiert ungehindert durch die äußere hemokompatible Schicht. Auch dieser Schichtaufbau liefert das Resultat, dass der Bewuchs der Stentoberfläche mit Zellen nicht unterbunden, jedoch auf ein ideales Maß reduziert wird. Die erste Schicht bietet hierbei eine Risikominimierung für eventuelle eintretende Beschädigungen der beschichteten Stentoberfläche bei der Implantation, z. B. durch Abschürfungen durch die vorhandende Plaque oder in den Vorbereitungen z.B. beim Crimpen. Eine zweite Sicherheits-Gewährleistung ergibt sich durch die Tatsache, dass auch ein biostabiles Polymer sich im Körper innerhalb eines mehr oder weniger langen Zeitraums abbauen wird und die Stentoberfläche zumindest teilweise freilegt. Kombinationen vor allem mit biodegradierbarem Material, wie in den Beschichtungsprinzipien beschrieben, sind hier ebenfalls möglich.

Derartige Stents lassen sich herstellen, indem man einen herkömmlichen Stent bereitstellt, auf dessen Oberfläche man eine hemokompatible erste Schicht aufbringt, eine nicht-bioabbaubare Schicht aufträgt, welche mindestens einen Wirkstoff als auch Kombinationen mit anderen Wirkstoffen aus anderen Gruppen enthält, kovalent oder/und adhäsiv gebunden, und diese Schicht im wesentlichen vollständig mit einer weiteren hemokompatiblen Schicht überzieht.

Substanzen, welche für die biostabile Schicht in Frage kommen, sind alle in der Medizin verwendeten beständigen Materialien. Dazu zählen Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Epoxyharze, ABS-Harze, EPDM-Gummis, Silicone wie Polysiloxane, Polyvinylhalogene und Copolymere, Celluloseether, Cellulosetriacetate, Chitosan und Copolymere und/oder Mischungen der vorgenannten Stoffe.

Bei Mehrschichtsystemen überdeckt die neu aufgebrachte Schicht die darunterliegende Schicht im wesentlichen vollständig. "Im wesentlichen" bedeutet in diesem Zusammenhang, dass zumindest die mit der Gefäßwand in Kontakt kommende Stentoberfläche vollständig überdeckt ist, bzw. mindestens 90%, bevorzugt 95% und insbesondere bevorzugt mindestens 98% der Stentoberfläche überdeckt sind.

Die erfindungsgemäßen Stents lösen sowohl das Problem der akuten Thrombose als auch das Problem der Neointimahyperplasie nach einer Stentimplantation. Zudem eignen sich die erfindungsgemäßen Stents aufgrund ihrer Beschichtung, ob als Einzelschicht oder als Mehrschichtsystem, besonders gut zur kontinuierlichen Freisetzung eines oder mehrerer antiproliferativer, immunsuppressiver und/oder Wirkstoffe. Aufgrund dieser Fähigkeit der gezielten kontinuierlichen Wirkstofffreisetzung in erforderlicher Menge verhindern die erfindungsgemäß beschichteten Stents die Gefahr der Restenose fast vollständig.

Zudem können gemäß dem beschriebenen Verfahren beliebige Kunststoffoberflächen mit einer hemokompatiblen Schicht der Oligo- und/oder Polysaccharide überzogen werden. Als Kunststoffe eignen sich synthetische Polymere sowie Biopolymere, beispielsweise zusammengesetzt aus den Monomeren Ethen, Vinylacetat, Methacrylsäure, Vinylcarbazol, Trifluorethylen, Propen, Buten, Methylpenten, isobuten, Styrol, Chlorstyrol, Aminostyrol, Acrylnitril, Butadien, Acrylester, Divinylbenzol, Isopren, Vinylchlorid, Vinylalkohol, Vinylpyridin, Vinylpyrrolidon, Tetrafluorethen, Trifluorchlorethen, Vinylfluorid, Hexafluorisobuten, Acrylsäure, Acrolein, Acrylamid, Methacrylamid, Maleinsäure, Hydroxymethylmethacrylsäure, Methylmethacrylsäure, Maleinsäureanhydrid, Methacrylsäureanhydrid, Methacrylnitril, Fluorstyrol, Fluoranilid, 3,4-Isothiocyanatostyrol, Allylalkohol, Sulfonsäure, Methallylsulfonsäure, Diallylphthalsäure, Cyanoacrylsäure, Dimethylaminoethylmethacrylsäure, Laurylmethacrylsäure, Acetaminophenylethoxymethacrylsäure, Glykoldimethacrylsäure, 2-Hydroxyethylmethacrylsäure, Formaldehyd, Fluoral, Chloral, Ethylenoxid, Tetrahydrofuran, Propylenoxid, Allylglycidylether, Epichlorhydrin, Glycerin, Trimethylpropan, Pentaerythrit, Sorbit, Phthalsäure, Bersteinsäure, Fumarsäure, Adipinsäure, thiophen, Ethylenimin, Hexamethylenadipamid, Hexamethylensebacamid, Hexamethylendodecandiamid, Aminobenzamid, Phenylendiamin, Amidhydrazide, Dimethylpiperazin, Benzimidazol, Tetraaminobenzol, Pyrone, ε-Caprolactam, Isophthalsäure, Glutaminsäure, Leucin, Phenylalanin, Valin, Lysin, Harnstoff, Diisocyanate, Thioharnstoff und anderen oder Gemische aus den vorgenannten Monomeren. Ferner kommen als Polymere in Betracht: Silicone, Cellulose und Cellulosederivate, Öle, Polycarbonate, Polyurethane, Agarose, Polysaccharide, Dextrane, Stärke, Chitin, Glykosaminoglykane, Gelatine, Kollagen I - XII und andere Proteine.

### Figurenbeschreibung

- Figur 1: zeigt ein Disaccharidstrukturfragment des Chitins, welches durch basische Hydrolyse zum Chitosan oder durch partielle Deacetylierung mit anschließender N-Carboxyalkylierung zu den Verbindungen der allgemeinen Formel Ia umgesetzt werden kann.

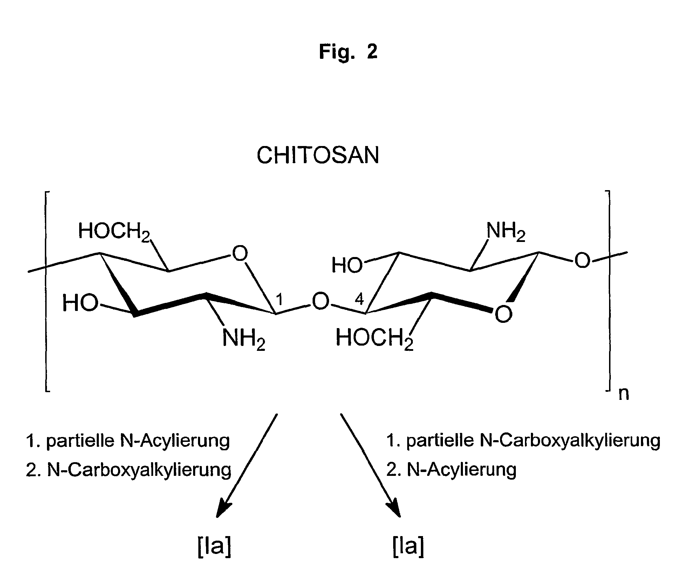

- Figur 2: zeigt ein Disaccharidstrukturfragment des Chitosans, welches durch partielle N-Acylierung mit anschließender N-Carboxyalkylierung oder durch partielle N-Carboxyalkylierung mit anschließender N-Acylierung zu den Verbindungen der allgemeinen Formel Ia umgesetzt werden kann.
- Figur 3: zeigt eine Tetrasaccharideinheit eines Heparins oder Heparansulfates mit statistischer Verteilung der Sulfatgruppen und einem Sulfatierungsgrad von 2 pro Disaccharideinheit wie er für Heparin typisch ist (Figur 3a). Zum Vergleich der strukturellen Ähnlichkeiten zeigt Figur 3b ein Beispiel einer Verbindung gemäß allgemeiner Formel Ib und Figur 3c zeigt einen Ausschnitt mit typischer Struktur für ein N-carboxymethyliertes, partiell N-acetyliertes Chitosan.

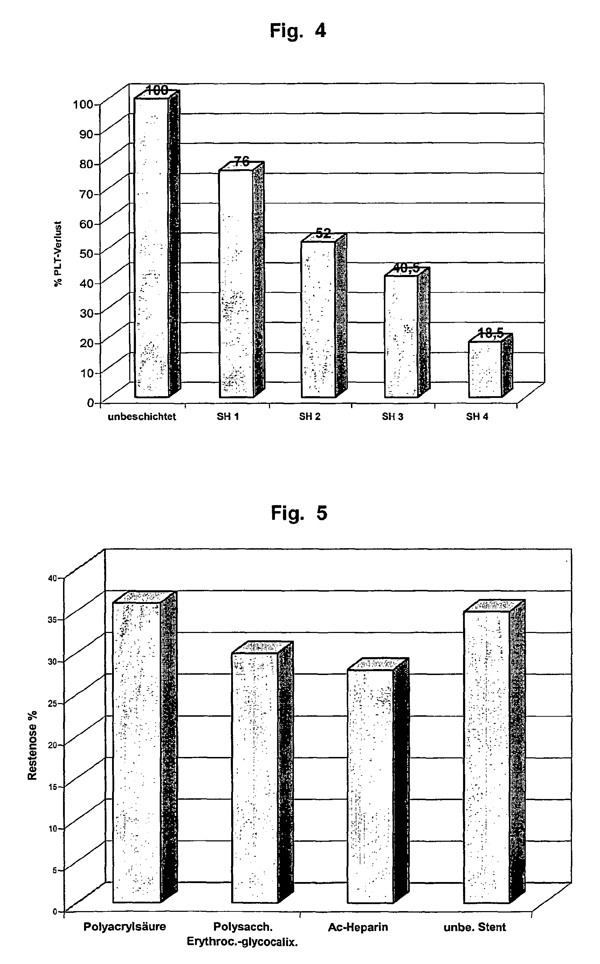

- Figur 4: zeigt den Einfluss von in einen PCV-Schlauch expandierten, oberflächenmodifizierten Edelstahl-Koronarstents auf den Thrombozytenverlust (PLT-Verlust). Als Referenz wurde ein unbeschichteter Edelstahl-Koronarstent vermessen (unbeschichtet). Als Nullwert wurde das Maß des Thrombozytenverlusts beim PCV-Schlauch ohne Edelstahl-Koronarstent angesetzt.
Dabei ist SH1 ist ein kovalent mit Heparin beschichteter Stent, SH2 ist ein mit Chondroitinsulfat beschichteter Stent; SH3 ist von der Erythrocytenglycocalix gewonnenen Polysacchariden beschichteter Stent und SH4 ist ein mit Ac-Heparin kovalent beschichteter Edelstahl-Koronarstent.
- Figur 5: zeigt eine bildliche Darstellung der Restenose-Rate von mit vollständig desulfatiertem und N-reacetyliertem Heparin (Ac-Heparin) kovalent beschichteten Stents und mit Oligo- und Polysacchariden der Erythrocytenglycocalix beschichteten Stents im Vergleich zum unbeschichteten Stent und mit Polyacrylsäure (PAS) beschichteten Stents nach 4 Wochen Implantationszeit im Schwein.
- Figur 6: Quantitative Koronarangiographie:
Aufnahmen der Querschnitte durch das den Stent enthaltende Gefäß-Segment eines mit Ac-Heparin beschichteten Stents (a.) und zum Vergleich die eines unbeschichteten (unbe.) Stents (b.). Nach vier Wochen im Tierversuch (Schwein) ist ein deutlicher Unterschied in den Dicken der gebildeten Neointimae zu erkennen.
- Figur 7: Elutionsdiagramm von Paclitaxel vom Stent (ohne Trägermaterial).
- Figur 8: Elutionsdiagramm von Paclitaxel eingebettet in PLGA-Matrix.
- Figur 9: Elutionsdiagramm von Paclitaxel eingebettet in PLGA-Matrix und einer die Basisbeschichtung vollständig überziehender Schicht aus reinem Paclitaxel.
- Figur 10: Elutionsdiagramm eines hydrophilen Wirkstoffes, eingelagert in der Matrix, und einem darüberliegenden, die Basisbeschichtung vollständig überziehendem wirkstofffreiem Polymer (Topcoat) zur Diffusionskontrolle.

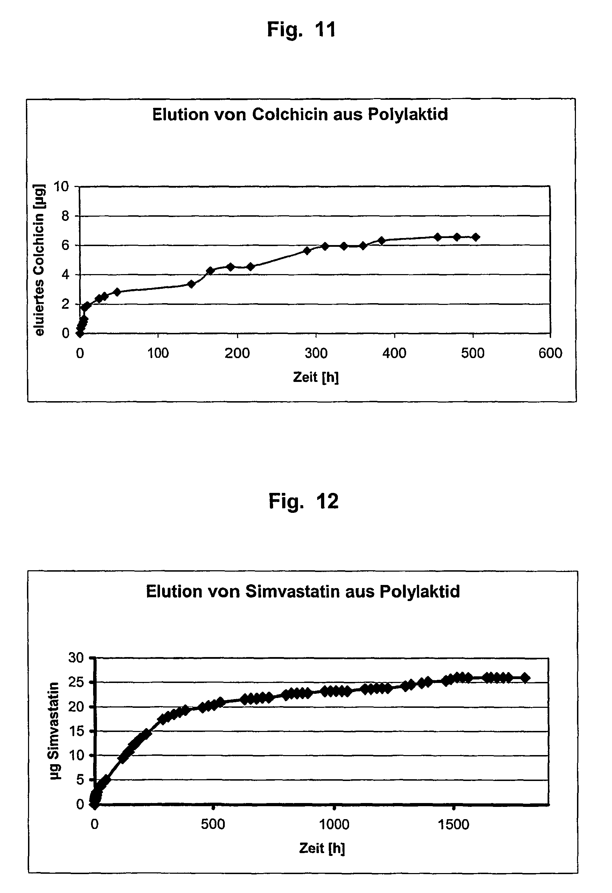

- Figur 11: Elutionsdiagramm von Colchicin aus PLGA-Matrix.
- Figur 12: Elutionsdiagramm von Simvastatin aus PLGA-Matrix.
- Figur 13: Elutionsdiagramm eines Statins aus der Matrix mit Polystyrol als die Basisbeschichtung vollständig überziehende diffusionskontrollierende Schicht.
- Figur 14: Vergleich der Thrombocytenzahl (Plättchenzahl) im Blut nach Chandler-Loop zwischen beschichtetem (besch.) und unbeschichtetem Stent (unbe.) in Bezug zum Leerschlauch (Kontrolle), zur Plättchenzahl frisch entnommenen Blutes (Donor) und nach Lagerung von 60 Min in der Spritze (Spritze 60).
- Figur 15: Vergleich des Plättchenfaktor 4-Gehaltes im frisch entnommenen Blut (Donor), im Leerschlauch (Kontrolle) nach 60 Minuten und unbeschichteten Stents (unbe.) mit beschichtetem Stent (besch.).
- Figur 16: Vergleichendes Diagramm zum aktivierter Komplementfaktor C5a im frisch entnommenen Blut (Donor), im Leerschlauch (Kontrolle) nach 60 Minuten und unbeschichteten Stents (unbe.) mit beschichtetem Stent (besch.).

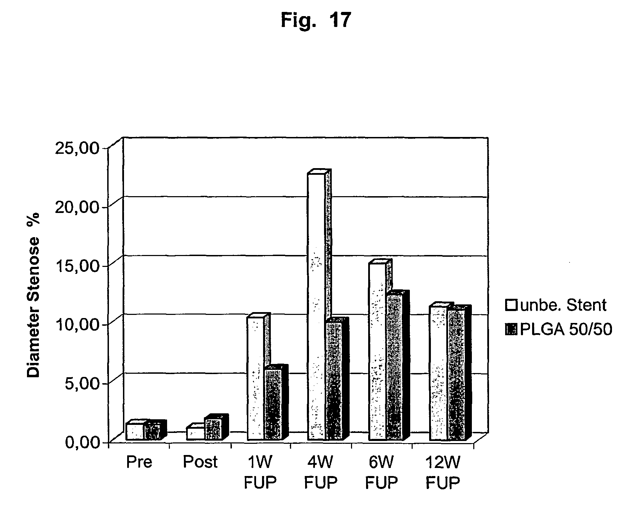

- Figur 17: Bildliche Darstellung der % Diameter-Stenose-Rate mit vollständig desulfatiertem und N-reacetyliertem Heparin (Ac-Heparin) kovalent beschichteten Stents und einer 2. Schicht aus Poly(D,L-lactid-co-glycolid) im Vergleich zum unbeschichteten Stent (nach 12 Wochen Implantationszeit im Schwein). Dargestellt wird der zeitliche Verlauf der Stenoseentwicklung bei PLGA, wobei "% Diameter-Stenose-Rate" der prozentual auf den Ausgangszustand direkt nach Einsatz des Stents (Post) bezogene Durchmesser des Gefäßes darstellt. Für die Untersuchungen wurden sechs bis neun Monate alte Hausschweine verwendet, der Gefäßdurchmesser vor (Pre) und nach dem Einsatz des Stents (Post) über intravasculärem Ultraschall (IVUS) gemessen. Nach einer Woche (1WoFUP), einem Monat (4WoFUP), sechs Wochen (6WoFUP) und nach drei Monaten (12WoFUP) wurden die gestenteten Bereiche jeweils koronarangiographisch und mit intravasculärem Ultraschall (IVUS) untersucht. Die erhaltenen Daten lassen einen unerwarteten erstaunlich positiven Effekt erkennen, der zweifelsohne auf die Beschichtung zurückzuführen ist. Obwohl sich die Werte der Stenose nach 3 Monaten für den unbeschichteten Stent und für den beschichteten Stent kaum noch unterscheiden, ist die Reaktion der Gefäßwand auf den PLGA-beschichteten Stent wesentlich milder. Nach einer Woche liegt der Stenosewert mit 6% signifikant unter dem Wert der unbeschichteten Implantate mit 10,4%. Die Maskierung der Metalloberfläche ergibt nach vier Wochen sogar mit 10% (einem Anstieg von 33%) eine um mehr als Faktor zwei geringere Stenoserate als der unbeschichtete Stent, der nach dieser Zeit seinen maximalen Wert von 22,6 % (einem Anstieg von 54%) erreicht. Der beschichtete Stent zeigt ein Maximum nach sechs Wochen mit lediglich 12,33 %. Nach 12 Wochen gleichen sich die Werte beider Systeme mit ca. 11 % aneinander an.

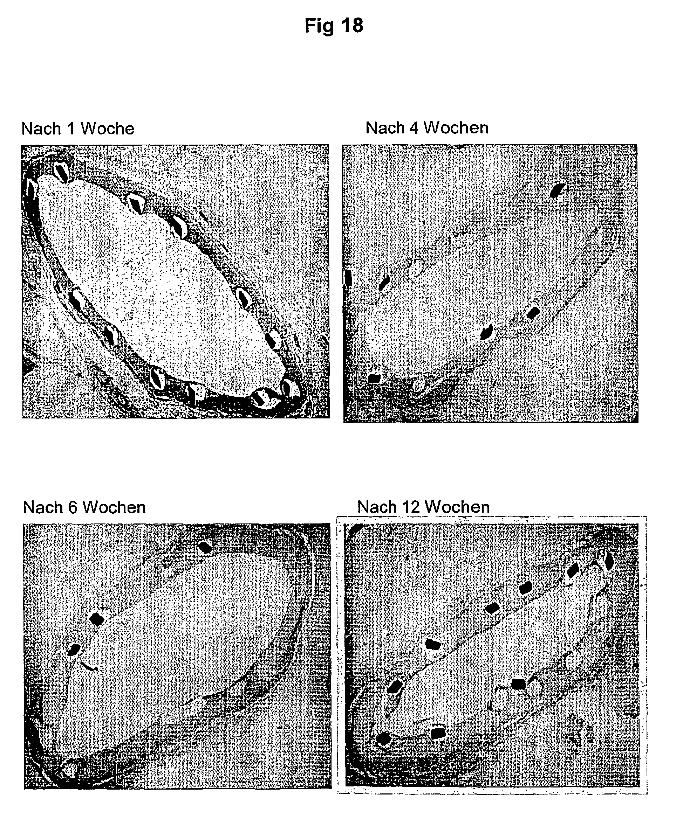

- Figur 18: Bilder der quantitativen Koronarangiographie der Tierversuche bzgl. Figur 17 nach 1 Woche, 4 Wochen, 6 Wochen und 3 Monaten von hemokompatibel ausgerüsteten PLGA-beschichteten Stents im Schwein.

### Beispiel 1

### Herstellung von desulfatiertem reacetyliertem Heparin:

100 ml Amberlite IR-122 Kationenaustauscherharz wurden in eine Säule mit 2 cm Durchmesser gefüllt, mit 400 ml 3M HCl in die H⁺-Form überführt und mit destilliertem Wasser gespült, bis das Eluat chloridfrei und pH neutral war. 1 g Natrium-Heparin wurde in 10 ml Wasser gelöst, auf die Kationenaustauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH⁻-Form und anschließend 150 µl Essigsäureanhydrid gegeben und 2 Stunden bei 4°C gerührt. Danach wird das Harz abfiltriert und die Lösung gegen Wasser dialysiert und gefriergetrocknet.

### Beispiel 2

### N-carboxymethyliertes, partiell N-acetyliertes Chitosan:

In 150 ml 0,1 N HCl wurde 2 g Chitosan gelöst und unter Stickstoff 24 Stunden unter Rückfluß gekocht. Nach dem Abkühlen auf Raumtemperatur wurde der pH der Lösung mit 2 N NaOH auf 5,8 eingestellt. Die Lösung wurde gegen demineralisiertes Wasser dialysiert und gefriergetrocknet.

1 g des so partiell hydrolysierten Chitosans wurden in 100 ml 1 %iger Essigsäure gelöst. Nach hinzufügen von 100 ml Methanol wurden 605 µl Essigsäureanhydrid gelöst in 30 ml Methanol zugegeben und 40 Minuten bei Raumtemperatur gerührt. Das Produkt wurde durch Eingießen in eine Mischung von 140 ml Methanol und 60 ml 25%iger NH₃-Lösung ausgefällt. Es wurde abfiltriert, mit Methanol und Diethylether gewaschen und unter Vakuum über Nacht getrocknet.

1 g des partiell hydrolysierten und partiell N-acetylierten Chitosans wurde in 50 ml Wasser suspendiert. Nach dem Hinzufügen von 0,57 g Glyoxylsäuremonohydrat löste sich das Chitosanderivat innerhalb der nächsten 45 Minuten auf. Der pH Wert der Lösung wurde mit 2 N NaOH auf 12 eingestellt. Eine Lösung von 0,4 g Natriumcyanoborhydrid in möglichst wenig Wasser wurde zugegeben und für 45 Minuten gerührt. Das Produkt wurde in 400 ml Ethanol ausgefällt, abfiltriert, mit Ethanol gewaschen und über Nacht im Vakuum getrocknet.

### Beispiel 3

### Herstellung von desulfatiertem N-propionyliertem Heparin:

100 ml Amberlite IR-122 Kationenaustauscherharz wurden in eine Säule mit 2 cm Durchmesser gefüllt, mit 400 ml 3M HCl in die H⁺-Form überführt und mit destilliertem Wasser gespült, bis das Eluat chloridfrei und pH neutral war. 1 g Natrium-Heparin wurde in 10 ml Wasser gelöst, auf die Kationenaustauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH⁻-Form und anschließend 192 µl Propionsäureanhydrid gegeben und 2 Stunden bei 4°C gerührt. Danach wird das Harz abfiltriert und die Lösung gegen Wasser dialysiert und gefriergetrocknet.

### Beispiel 4

### N-carboxymethyliertes, partiell N-propionyliertes Chitosan:

In 150 ml 0,1 N HCl wurde 2 g Chitosan gelöst und unter Stickstoff 24 Stunden unter Rückfluß gekocht. Nach dem Abkühlen auf Raumtemperatur wurde der pH der Lösung mit 2 N NaOH auf 5,8 eingestellt. Die Lösung wurde gegen demineralisiertes Wasser dialysiert und gefriergetrocknet.

1 g des so partiell hydrolysierten Chitosans wurden in 100 ml 1%iger Essigsäure gelöst. Nach hinzufügen von 100 ml Methanol wurden 772 µl Propionsäureanhydrid gelöst in 30 ml Methanol zugegeben und 40 Minuten bei Raumtemperatur gerührt. Das Produkt wurde durch Eingießen in eine Mischung von 140 ml Methanol und 60 ml 25%iger NH₃-Lösung ausgefällt. Es wurde abfiltriert, mit Methanol und Diethylether gewaschen und unter Vakuum über Nacht getrocknet.

1 g des partiell hydrolysierten und partiell N-acetylierten Chitosans wurde in 50 ml Wasser suspendiert. Nach dem Hinzufügen von 0,57 g Glyoxylsäuremonohydrat löste sich das Chitosanderivat innerhalb der nächsten 45 Minuten auf. Der pH Wert der Lösung wurde mit 2 N NaOH auf 12 eingestellt. Eine Lösung von 0,4 g Natriumcyanoborhydrid in möglichst wenig Wasser wurde zugegeben und für 45 Minuten gerührt. Das Produkt wurde in 400 ml Ethanol ausgefällt, abfiltriert, mit Ethanol gewaschen und über Nacht im Vakuum getrocknet.

### Beispiel 5

### Hemokompatibilitätbestimmungen von Verbindungen gemäß den allgemeinen Formeln Ia und Ib nach ISO 10933-4 (in vitro Untersuchungen)

Zur Untersuchung der Hemokompatibilität der Verbindungen gemäß Formel Ia und Ib wurden Cellulosemembranen, Silikonschläuche und Edelstahlstents mit einer Verbindung gemäß Formel Ia oder Ib kovalent beschichtet und gegen Heparin sowie gegen die entsprechenden, in den einzelnen Versuchen verwendeten unbeschichteten Material-Oberflächen getestet.

### 5.1. Cellulosemembranen (Cuprophan) beschichtet mit desulfatiertem, reacetyliertem Heparin (Ac-Heparin)

Zur Untersuchung der gerinnungsphysiologischen Wechselwirkungen zwischen citriertem Vollblut und den Ac-Heparin- bzw. Heparin-beschichteten Cuprophanmembranen wird das offene Perfusionssystem der von Sakariassen modifizierten Baumgartner-Kammer eingesetzt [Sakariassen K.S. et al.; *J. Lab. Clin. Med.* 102 : 522-535 (1983)]. Die sich aus vier Bauteilen zzgl. Dichtringen und Verschraubungen zusammensetzende Kammer ist aus Polymethylmethacrylat gefertigt und gestattet die parallele Untersuchung von zwei modifizierten Membranen, so dass in jeden Lauf bereits eine statistische Absicherung eingeht. Die Bauweise dieser Kammer erlaubt quasi-laminare Perfusionsbedingungen.

Nach 5-minütiger Perfusion bei 37°C werden die Membranen entnommen und nach Fixierung der adhärierten Blutplättchen die Thrombozytenbelegung bestimmt. Die jeweiligen Messwerte werden in Bezug zur hochthrombogenen Subendothelialen Matrix als Negativstandard mit einer 100%igen Plättchenbelegung gesetzt. Die Anlagerung der Thrombozyten erfolgt sekundär an die sich zuvor auf dem Fremdmaterial bildende Plasmaproteinschicht. Das Plasmaprotein Fibrinogen wirkt als Cofaktor der Plättchenaggregation. Die derart induzierte Aktivierung der Blutplättchen zieht die Bindung einer ganzen Reihe von gerinnungsassoziierten Plasmaproteinen, wie z.B. Vitronectin, Fibronectin und von Willebrand-Faktor auf der Thrombozytenoberfläche nach sich. Durch deren Einwirkung kommt es schließlich zur irreversiblen Aggregation der Thrombozyten. Die Thrombozytenbelegung stellt aufgrund der beschriebenen Wechselwirkungen ein anerkanntes Maß für die Thrombogenität von Oberflächen im Fall des Fremdoberflächenkontaktes von Blut dar. Aus dieser Tatsache ergibt sich die Schlussfolgerung: je geringer die Thrombozytenbelegung auf der perfundierten Oberfläche ist, als um so höher ist die Hemokompatibilität der untersuchten Oberfläche zu bewerten.

Die Ergebnisse der untersuchten Heparin-beschichteten und Ac-Heparin-beschichteten Membranen zeigen deutlich die Verbesserung der Hemokompatibilität der Fremdoberfläche durch die Beschichtung mit Ac-Heparin. Heparin-beschichtete Membranen zeigen eine 45 - 65%ige Thrombozytenbelegung, während Ac-Heparin-beschichtete Oberflächen Werte von 0 - 5% aufweisen (Bezug zur Subendothelialen Matrix mit 100%iger ThrombozytenBelegung).

Die Adhäsion der Thrombozyten auf der Ac-heparinisierten Oberfläche ist aufgrund der fehlenden, für die Aktivierung der Blutplättchen notwendigen, Plasmaproteine extrem erschwert. Im Gegensatz dazu bietet die Heparinbeschichtete Oberfläche mit der sofort einsetzenden Plasmaproteinadsorption optimale Voraussetzungen zur Aktivierung, Ablagerung und Aggregation der Thrombozyten; und letztendlich reagiert das Blut durch Aktivierung der Gerinnungskaskade mit den entsprechenden Abwehrmechanismen auf die eingesetzte Fremdoberfläche. Ac-Heparin erfüllt weitaus besser als Heparin die Ansprüche an die Hemokompatibilität der Fremdoberfläche.

Das Zusammenspiel von Plasmaproteinadsorption und Thrombozytenbelegung als direktes Maß für die Thrombogenität einer Oberfläche, in Abhängigkeit von der dem Blut dargebotenen Beschichtung, wird durch diesen in-vitro-Test besonders gut verdeutlicht. Demnach ist die Verwendung von kovalent gebundenem Heparin als antithrombotisch wirksame Oberfläche nur sehr eingeschränkt bis nicht möglich. Die Wechselwirkungen von immobilisiertem Heparin mit Blut kehren sich hier ins unerwünschte Gegenteil um - die heparinbeschichtete Oberfläche wird thrombogen.
Offensichtlich ist die herausragende Bedeutung von Heparin als Antithrombotikum nicht auf kovalent immobilisiertes Heparin übertragbar. In der systemischen Verabreichung kann es in gelöster Form seine Wirkung voll entfalten. Wird Heparin jedoch kovalent immobilisiert, ist seine antithrombotische Wirkung, wenn überhaupt, nur von kurzer Dauer. Anders das Ac-Heparin ("No-affinity"-Heparin), welches durch die Desulfatierung und N-Reacetylierung die aktiven antithrombotischen Eigenschaften des Ausgangsmoleküls zwar komplett verliert, dafür aber im Gegenzug ausgeprägte athrombogene Eigenschaften erwirbt, die nachweislich in der Passivität gegenüber Antithrombin III und der fehlenden Affinität gegenüber koagulationsinitüerenden Vorgängen begründet sind und nach kovalenter Bindung erhalten bleiben.

Dadurch eignet sich Ac-Heparin und damit die Verbindungen der allgemeinen Formeln Ia und Ib insgesamt bestens zur Tarnung von fremden Oberflächen im Kontakt mit dem Gerinnungssystem.

### 5.2. Immobilisierung auf Silikon:

Durch einen 1 m langen Silikonschlauch mit 3 mm Innendurchmesser wurde 30 Minuten lang bei 40°C 100 ml eines Gemisches aus Ethanol/Wasser 1/1 (V/V) im Kreis gepumpt. Dann wurden 2 ml 3-(Triethoxysilyl)-propylamin zugegeben und weitere 15 Stunden bei 40°C im Kreis gepumpt. Danach wurde noch jeweils 2 Stunden mit 100 ml Ethanol/Wasser und 100 ml Wasser gespült.

3 mg des deacetylierten und reacetylierten Heparins (Ac-Heparin) wurden bei 4°C in 30 ml 0,1M MES-Puffer pH 4,75 gelöst und mit 30 mg CME-CDI (N-Cyclohexyl-*N*'-(2-morpholinoethyl)carbodiimidmethyl-p-toluolsulfonat) versetzt. Diese Lösung wurde für 15 Stunden bei 4°C im Kreis durch den Schlauch gepumpt. Anschließend wurde mit Wasser, 4M NaCl-Lösung und Wasser für je 2 Stunden gespült.

### 5.3 Bestimmung der Thrombozytenzahl (EN30993-4)

Auf einen 1 m langen Silikonschlauch mit 3 mm Innendurchmesser wurden zwei 2 cm lange enganliegende Glasröhrchen geschoben. Dann wurde der Schlauch mit einem Schrumpfschlauch zu einem Kreis geschlossen und luftfrei über Spritzen mit 0,154M NaCl Lösung gefüllt. Dabei wurde mit einer Spritze die Lösung eingefüllt und mit der anderen Spritze die Luft herausgezogen. Mit den beiden Spritzen wurde die Lösung luftblasenfrei gegen citriertes Vollblut eines gesunden Probanden ausgetauscht. Danach wurden die Einstichlöcher der Injektionsnadeln durch Überschieben der Glasröhrchen verschlossen und der Schlauch in eine Dialysepumpe gespannt. Das Blut wurde 10 Minuten mit einer Flußrate von 150 ml/min umgepumpt. Der Thrombozytengehalt des Blutes wurde vor und nach der Perfusion mit einem Coulter Counter bestimmt. Für unbeschichtete Silikonschläuche lag der Thrombozytenverlust bei 10%. Dagegen lag er bei Silikonschläuchen, die nach Beispiel 5.2 beschichtet wurden, im Durchschnitt bei 0% (Anzahl der Versuche: n=3).

Auch in diesem dynamischen Testsystem zeigt sich, das die Aktivierung von Thrombozyten an einer Oberfläche beschichtet mit Ac-Heparin reduziert ist. Gleichzeitig läßt sich festhalten, dass die Immobilisierung von Heparin aus den einen negativen Effekt auf die Hemokompatibilität der eingesetzten Oberfläche ausübt. Dagegen zeigt Ac-Heparin, seiner passiven Natur entsprechend, keine Effekte im Kontakt mit den Thrombozyten.

### 5.4. Vollblutuntersuchungen an 316 LVM-Edelstahl-Koronarstents

Im Rahmen der Biokompatibilitätsuntersuchungen wurden 31 mm lange 316 LVM-Edelstahl-Stents mit Ac-Heparin kovalent beschichtet. Bei einer Gesamtoberfläche von 2cm² und einer Belegungsdichte von ca. 20 pm/cm² Stentoberfläche beträgt die Beladung eines solchen Stents ca. 0,35 µg Ac-Heparin. Als Vergleich: Die bei der Thromboseprophylaxe übliche Tagesdosis von Heparin beträgt dagegen 20-30 mg und entspräche somit dem mindestens 60.000-fachen Wert.

Diese Untersuchungen wurden am Institut für Physiologie an der RWTH Aachen mit dem etablierten hämodynamischen Chandler-Loop-System durchgeführt [A. Henseler, B. Oedekoven, C. Andersson, K. Mottaghy; *KARDIOTECHNIK* **3** (1999)]. Beschichtete und unbeschichtete Stents wurden in PVC-Schläuchen (medical grade PVC) mit 600 mm Länge und 4 mm Innendurchmesser expandiert und getestet. Die Ergebnisse dieser Versuche bestätigen die bezüglich der Siliconschläuche diskutierten Untersuchungen. Der anfänglich auf den Stent zurückzuführende Blutplättchenverlust im Perfusat von 50% wird durch die Veredlung der Stentoberfläche mit Ac-Heparin um über 80% reduziert.

Der Einfluß von in den Schlauch expandierten, oberflächenmodifizierten Edelstahl-Koronarstents auf den Thrombozytenverlust wird in weiteren Chandler-Tests während der 45minütigen Vollblutperfusion evaluiert. Hierzu wird zuerst der stentfreie PVC-Schlauch untersucht, woraus sich der Nullwert ergibt. Der Leerschlauch zeigt einen mittleren Plättchenverlust von 27,4 % bezüglich des Spenderblutes bei einer Standardabweichung von lediglich 3,6%. Diesen Basiswert zugrundelegend werden unterschiedlich oberflächenmodifizierte Stents in den PVC-Schläuchen expandiert und unter analogen Bedingungen auf den von ihnen verursachten Plättchenverlust hin untersucht. Auch hier stellt sich wieder heraus, dass die durch den Stent bedeckte Oberfläche, die lediglich ca. 0,84% der gesamten Testoberfläche ausmacht, einen signifikanten und reproduzierbaren Effekt auf den Thrombozytengehalt verursacht. Bezogen auf den Leerschlauch (Basiswert) liefert die Untersuchung des polierten, chemisch nicht oberflächenvergüteten Stents einen zusätzlichen mittleren Thrombozytenverlust von 22,7%. Damit verursacht diese im Vergleich zum PVC-Leerschlauch weniger als 1% messende Fremdoberfläche einen etwa vergleichbaren Thrombozytenverlust. Daraus leitet sich direkt ab, dass der als Stentmaterial verwendete medizinische Edelstahl 316 LVM, obwohl diese Testoberfläche nur 0,84% der gesamten Oberfläche ausmacht, verglichen mit einer medical grade PVC-Oberfläche, eine ca. 100-fach stärkere Plättchenschädigung induziert.

Die untersuchten Oberflächenvergütungen an den Edelstahl-Koronarstents zeigen sich in der Lage, das enorme Ausmaß der stentinduzierten Plättchenschädigung sehr deutlich zu reduzieren (siehe Fig. 4). Am effektivsten erwies sich mit 81,5% das Ac-Heparin (SH4).

Betrachtet man also die Auswirkungen der Ac-Heparin-beschichteten Stents auf den Thrombozytenverlust, ergeben sich gut übereinstimmende Werte. Die Korrelation der Thrombozytenverluste im Perfusat bzw. der Adhäsion der Thrombozyten an die dargebotenen Oberflächen zeigen die Verlässlichkeit der Ermittlungen.

### 5.4.1 Kovalente hemokompatible Beschichtung von Stents

Nicht expandierte Stents aus medizinischem Edelstahl LVM 316 wurden im Ultraschallbad 15 Minuten mit Aceton und Ethanol entfettet und bei 100°C im Trockenschrank getrocknet. Danach wurden sie für 5 Minuten in eine 2%ige Lösung von 3-Aminopropyltriethoxysilan in einem Gemisch Ethanol/Wasser (50/50 : (v/v)) getaucht und anschließend für 5 Minuten bei 100°C getrocknet. Anschließend wurden die Stents über Nacht mit demineralisiertem Wasser gewaschen.

3 mg desulfatiertes und reacetyliertes Heparin wurden bei 4°C in 30 ml 0,1 M MES-Puffer (2-(N-Morpholino)ethansulfonsäure) pH 4,75 gelöst und mit 30 mg N-Cyclohexyl-N'-(2-morpholinoethyl)carbodiimid-methyl-p-toluolsulfonat versetzt. In dieser Lösung wurden 10 Stents für 15 Stunden bei 4°C gerührt. Anschließend wurde mit Wasser, 4M NaCl-Lösung und Wasser für je 2 Stunden gespült.

### 5.4.2 Bestimmung des Glucosamingehaltes der beschichteten Stents mittels HPLC

Hydrolyse : Die beschichteten Stents werden in kleine Hydrolyserohre gegeben und mit 3ml 3M HCl genau eine Minute bei Raumtemperatur stehengelassen. Die Metallproben werden entfernt und die Röhrchen nach dem Verschließen 16h im Trockenschrank bei 100°C inkubiert. Danach lässt man abkühlen, es wird dreimal zur Trockne eingedampft und in 1 ml entgastem und filtriertem Wasser aufgenommen und gegen einen ebenfalls hydrolysierten Standard in der HPLC vermessen :

| Stent Nr. | Fläche Probe | Ac-Heparin [g/Probe] | Fläche [cm²] | Ac-Heparin [g/cm²] | Ac-Heparin [pmol/cm²] |
|---|---|---|---|---|---|
| 1 | 129,021 | 2,70647E-07 | 0,74 | 3,65739E-07 | 41,92 |
| 2 | 125,615 | 2,63502E-07 | 0,74 | 3,56084E-07 | 40,82 |
| 3 | 98,244 | 1,93072E-07 | 0,74 | 2,60908E-07 | 29,91 |
| 4 | 105,455 | 2,07243E-07 | 0,74 | 2,80058E-07 | 32,10 |
| 5 | 119,061 | 2,33982E-07 | 0,74 | 3,16192E-07 | 36,24 |
| 6 | 129,202 | 2,53911 E-07 | 0,74 | 3,43124E-07 | 39,33 |
| 7 | 125,766 | 2,53957E-07 | 0,74 | 3,43185E-07 | 39,34 |

### Beispiel 6

### In vivo Untersuchungen von beschichteten Koronarstents (Fig. 5)

### 6.1. In vivo Untersuchungen von Koronarstents beschichtet mit Ac-Heparin

Aufgrund der Daten zur Hemokompatibilität, die Ac-Heparin in den in-vitro-Versuchen geliefert hat, wurde die Eignung der Ac-Heparin-Oberfläche als athrombogene Beschichtung von Metallstents in vivo (im Tierversuch) untersucht. Das Ziel der Versuche bestand primär darin, den Einfluß der Ac-Heparin-Beschichtung auf die stentinduzierte Gefäßreaktion zu bewerten. Neben dem Registrieren möglicher thrombotischer Ereignisse wurden die für restenotische Prozesse relevanten Parameter wie Neointimafläche, Gefäßlumen und Stenosegrad erfasst.

Die Tierversuche wurden am Inselspital Bern (Schweiz) unter der Leitung von Prof. Hess einem anerkannten Kardiologen, durchgeführt. Für die Untersuchungen wurden 6 - 9 Monate alte Hausschweine, ein für die Bewertung von Stents seit langem etabliertes und anerkanntes Tiermodell, verwendet.

Erwartungsgemäß wurden in diesen Versuchen weder akute, subakute noch spätakute thrombotische Ereignisse registriert, was als Bestätigung der athrombogenen Eigenschaften des Ac-Heparins gewertet werden darf.

Nach vier Wochen wurden die Tiere euthanasiert, die gestenteten Koronararterien-Segmente entnommen und histomorphometrisch analysiert.

Hinweise auf eine mögliche akute oder subchronische Toxizität, Sensibilisierungsreaktionen oder anderweitige Irritationen als Folge der Implantation von Ac-Heparin-beschichteten Stents sind während der gesamten Versuchsphase, insbesondere bei der histologischen Auswertung, nicht festgestellt worden.
Während der Stentimplantation sowie im Follow-up wurden koronarangiographische Datensätze ermittelt, die eine Auswertung im Hinblick auf die Gefäßreaktion auf die Stentimplantation erlauben.
Der Unterschied zwischen dem unbeschichteten Kontrollstent und dem mit Ac-Heparin beschichteten Stent ist eindeutig. Die Ausbildung einer ausgeprägten Neointimaschicht ist bei dem unbeschichteten Kontrollstent sehr gut erkennbar. Schon nach vier Wochen zeigt sich die proliferationsfördernde Wirkung der unbeschichteten Stentoberfläche auf das umliegende Gewebe in einem solchen Ausmaß, dass letztendlich die Gefahr des Gefäßverschlusses im Stentbereich gegeben ist.
Im Fall des Ac-Heparin-beschichteten Stents lässt sich dagegen eine deutlich dünnere Neointimaschicht erkennen, die für ein reguliertes Einwachsen des Stents unter Erhalt eines weiten, freien Gefäßlumens spricht.

Die detaillierten histomorphometrischen und koronarangiographischen Daten untermauern diese Aussage, indem übereinstimmend festgestellt werden kann, dass durch die Ac-Heparin-Beschichtung (SH4) die Neointimahyperplasie ("Restenose") um ca. 17-20% im Vergleich zum unbeschichteten Kontrollstent zurückgedrängt wurde.

Dieses Ergebnis ist unerwartet und bemerkenswert zugleich. Sicher wird von einer athrombogenen Oberfläche nicht gefordert, zusätzlich zur Bereitstellung hemokompatibler Eigenschaften auch die zu einer Neointimahyperplasie führenden Prozesse zu beeinflussen, d.h. Restenosen zu verhindern.

Zum einen wird durch die dichte, dauerhafte Belegung der Stentoberfläche mit Ac-Heparin ein direkter Zellkontakt zur Metalloberfläche verhindert. Da in der Fachliteratur die Emission bestimmter Metallionen in das implantatnahe Gewebe als eine wahrscheinliche Ursache der Restenose diskutiert wird, könnte in einer von der Beschichtung bewirkten Unterbindung des direkten Metallkontaktes eine antirestenotische Wirksamkeit begründet sein.

Zum anderen ist ein derartiger positiver Nebeneffekt durchaus plausibel, weil auf einer passiven, athrombogenen Stentoberfläche mit dem Ausbleiben einer Thrombozytenaggregation auch die proliferativen Wirkungen der dabei freigesetzten Wachstumsfaktoren ausbleiben. Es fällt somit ein entscheidender, von der Lumenseite ausgehender Stimulus der neointimalen Proliferation aus.

### Beispiel 7

### In vivo Untersuchungen von Koronarstents beschichtet mit kovalent gebundenen Ac-Heparin und einer zweiten darüberliegenden Schicht aus PLGA 50/50

### 7.1 Hemokompatibilität der verwendeten Matrix in vitro

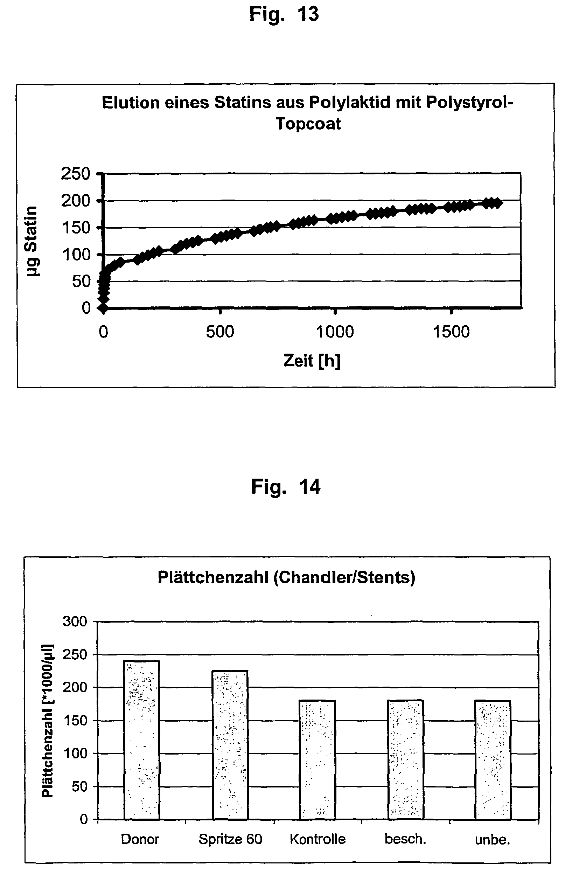

Zur Beurteilung der Hämokompatibilität von PLGA50/50 wurden Edelstahlstents mit dem Polymeren beschichtet und am Institut für Physiologie der RWTH Aachen mit Hilfe von in vitro-Vollbluttests geprüft. Das verwendete, etablierte und standardisierte Chandler-Loop-System ist ein dynamisches geschlossenes Schlauchsystem, das ohne die Verwendung einer Pumpe auskommt. Im Rahmen der Hämokompatibilitätsuntersuchungen wurden Blutbild, Plättchenfaktor 4 (PF4) und Komplementfaktor 5a (C5a) bestimmt (siehe Figuren 14-16). Zu Vergleichszwecken wurde der unbeschichtete Stent hinzugezogen.

### Versuchsdurchführung :

Spenderblut wird in 1,5 U/ml Heparin aufgenommen. Die Stents werden in PVC-Schläuche (I.D. 3,5 mm, L=95cm) eingeführt und mittels Ballonkatheter fixiert. Die 4 Schläuche (K3-K6) und zwei Leerschläuche (L1, L2) werden mit jeweils 7,5 ml isoton. Kochsalzlösung gefülllt und 15 Minuten mit 5 U/min bei 37°C im Chandler-Loop rotiert. Die vollständig entleerten Schläuche werden mit heparinisiertem Spenderblut (7,5 ml) vorsichtig befüllt und 60 Min. bei 5 U/min rotiert. Entsprechend den Antikoagulantien werden Proben in Monovetten bzw. Probengefäßen entnommen (PF4-CTAD, C5a-EDTA, BB-EDTA) und weiterverarbeitet.

Die Bestimmung der Thrombocytenzahl zeigt keinen signifikanten Unterschied zwischen den leeren Kontrollschläuchen, den beschichteten und unbeschichteten Stents. Der freigesetzte PF4 liegt bei beschichteten und unbeschichteten Stents auf gleichem Niveau, Die Bestimmung des aktivierten Komplement-faktors 5 (C5a) zeigt bei den beschichteten Stents eine geringere Aktivierung als bei den unbeschichteten Stents.

### 7.2 Hemokompatibilität der verwendeten Matrix in vivo

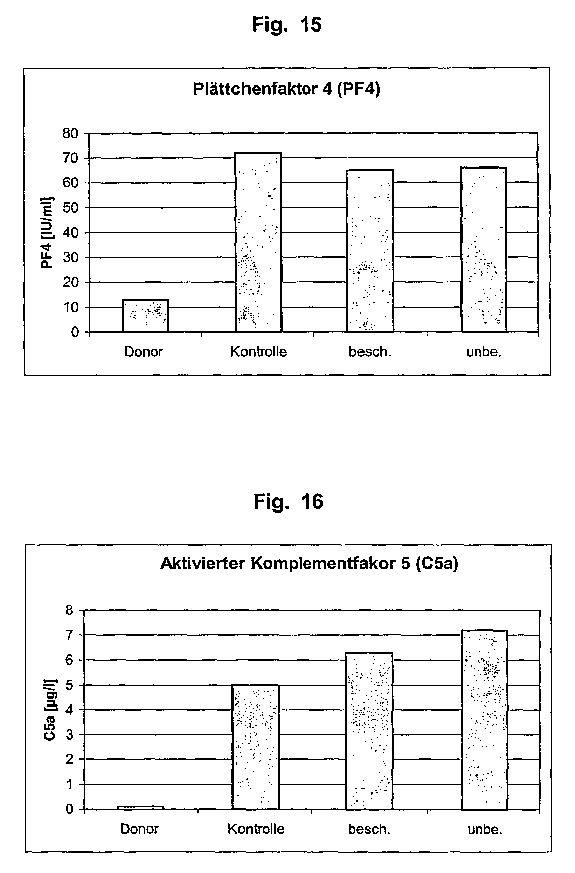

Das Ziel der Versuche bestand primär darin, den Einfluß der PLGA-Beschichtung auf die stentinduzierte Gefäßreaktion zu bewerten. Für die Untersuchungen wurden 28 sechs bis neun Monate alte Hausschweine, verwendet. Die gestenteten Bereiche wurden nach einer Woche (1WoFUP), einem Monat (4WoFUP), sechs Wochen (6WoFUP) und nach drei Monaten (12WoFUP) untersucht. Die erhaltenen Daten lassen einen unerwarteten erstaunlich positiven Effekt erkennen, der zweifelsohne auf die Anwesenheit von PLGA 50/50 zurückzuführen ist. Obwohl sich die Werte der Stenose nach 3 Monaten für den unbeschichteten Stent und für den beschichteten Stent kaum noch unterscheiden, ist die Reaktion der Gefäßwand auf den PLGA-beschichteten Stent wesentlich milder. Nach einer Woche liegt der Stenosewert mit 6% signifikant unter dem Wert der unbeschichteten Implantate mit 10,4%. Die Maskierung der Metalloberfläche ergibt nach vier Wochen sogar mit 10% (einem Anstieg von 33%) eine um mehr als Faktor zwei geringere Stenoserate als der unbeschichtete Stent, der nach dieser Zeit seinen maximalen Wert von 22,6 % (einem Anstieg von 54%) erreicht. Der beschichtete Stent zeigt ein Maximum nach sechs Wochen mit lediglich 12,33 %. Nach 12 Wochen gleichen sich die Werte beider Systeme mit ca. 11 % aneinander an (Fig.16).
Die Daten zu den restenotischen Prozesse wurden mittels quantitativer Koronarangiographie (QCA) und intravasculären Ultraschall-Untersuchungen (IVUS) ermittelt

### Beispiel 8

### Versuche zur Beschichtung von Oberflächen mit Tacrolimus:

### Vorversuche mit Toluidinblau:

Da Tacrolimus nur schlecht chemisch nachweisbar ist, werden erste Vorversuche mit Toluidinblau (Aldrich) durchgeführt.

| Chemikalien: | |
|---|---|
| Edelstahlröhrchen LVM 316 : | 2,5 cm lang, 2 mm Durchmesser |
| Polylaktid : | Fluka, Lot. 398555/123500, HNr. 0409 |
| Toluidinblau : | Aldrich, Lot. 19,816-1, HNr. 0430 |
| PBS-Puffer pH 7,4 : | 14,24 g Na₂HPO₄, 2,72 g NaH₂PO₄ und 9 g NaCl |

### Durchführung:

Der Stent wird auf der Analysenwaage abgewogen und das Gewicht notiert. In einem kleinen Hydrolyserohr werden 0,5 g Polylaktid in 2 ml CHCl₃ gelöst. Dazu wird im Wasserbad auf ca. 65°C erwärmt. Die Lösung wird dann im Tiefkühlfach abgekühlt. Dazu werden 200 µg Toluidinblau in 200 µl CHCl₃ gegeben. In diese Lösung wird der Stent getaucht. Nach einigen Minuten wird der Stent mit einer Pinzette aus der Lösung genommen und im Abzug solange an der Luft bewegt, bis das Lösungsmittel verdampft ist. Danach wird der Stent ein zweites mal eingetaucht. Der Stent wird nach dem Trocknen an der Luft noch für ca. 10 min an die Gefriertrocknung gehängt. Nach dem Trocknen wird der Stent erneut gewogen. Aus der Gewichtsdifferenz errechnet sich die Menge des immobilisierten Polylaktids mit Toluidinblau. (Probe 1).
Dieser Versuch wird mit derselben Lösung ein weiteres Mal wiederholt (Probe 2). Für Probe 3 wird die aus Versuch 1 (Probe 1) und Versuch 2 (Probe 2) resultierende Tauchlösung (1,93 ml) mit 0,825 mg Toluidinblau in 0,825 ml CHCl₃ und 250 mg Polylaktid versetzt. Das Polylaktid wird unter Erwärmung gelöst. Dann wird ein Stent zweimal darin wie oben beschrieben getaucht.

### Ergebnisse:

Die unbehandelten Stents hatten ein Gewicht von 176,0 mg und 180,9 mg. Nach dem Tauchen in die Polylaktidlösung wogen die Stents 200,9 und 205,2 mg.

Die Tauchlösung enthält 500 mg Polylaktid und 200 µg Toluidinblau aus diesem Verhältnis läßt sich die gebundene Toluidinblaumenge für die Proben 1 und 2 berechnen. Bei Probe 3 enthalten 2,755 ml Lösung 1 mg Toluidinblau und 638,6 mg Polylaktid (Einwaage-Verbrauch Probe 1 + 2; ca. 50 mg). Hier werden zwei Stents in einen Ansatz gegeben, um höhere Absorptionen zu erhalten. Da die Tauchlösung sehr zähflüssig war und sich dadurch eine sehr dicke Beschichtung ergab, wurde sie von 2,625 ml mit Chloroform auf 4 ml verdünnt.

### Konzentrationen in der Tauchlösung:

| Probe | Volumen (ml) | c (Polylactid mg/ml) | c (Toluidinblau µg/ml) |
|---|---|---|---|
| 1 | 2,2 | 227,3 | 90,9 |
| 2 | 2,2 | 227,3 | 90,9 |
| 3 | 2,755 | 231,8 | 363,0 |
| 4 | 4,0 | 134,5 | 212,5 |

Gewicht der Röhrchen und die daraus errechnete Beschichtung :

| Probe | Leergewicht | Gesamtgewicht | PL u. Toluidinblau | Toluidinblau |
|---|---|---|---|---|
| 1 | 176,0 mg | 200,9 mg | 24,9 mg | 9,96 µg |
| 2 | 180,9 mg | 205,2 mg | 24,3 mg | 9,72 µg |
| 3 | 317,2 mg | 410,8 mg | 93,6 mg | 135,73 µg |
| 4 | 180,8 mg | 194,8 mg | 14,8 mg | 23,38 µg |

### Beispiel 9

### Elutionsverhalten der Beschichtungen mit unterschiedlichen Konzentrationen:

Als Vorversuch wird ein UV-VIS Spektrum einer Toluidinblaulösung in Ethanol aufgenommen (C = 0,1 mg/ml) und das Absorptionsmaximum bestimmt. Die Toluidinblaukonzentration in der Lösung wird beim Absorptionsmaximum von 627 nm bestimmt. Dazu wird vorher eine Eichkurve erstellt.
Ein Stent wird in ein Becherglas mit 25 ml physiologischer Kochsalzlösung in einem Phosphatpuffer pH 7,4 (14,24 g NaH₂PO₄, 2,72 g K₂HPO₄ und 9 g NaCl) gehängt und vorsichtig bei Raumtemperatur gerührt. Nach 0,5, 1, 2, 3, 6, 24, 48 und 120 Stunden wird jeweils eine Probe von 3 ml entnommen, spektroskopisch vermessen und in den Ansatz zurückgegeben.

| Zeit/h | Abs. P1 | c (ng/ml) | Abs. P2 | c (ng/ml) | Abs. P3 | c (ng/ml) | Abs. P4 | c (ng/ml) |
|---|---|---|---|---|---|---|---|---|
| 0 | 0,0002 | 0 | -0,0002 | 0 | 0,0036 | 0 | 0,0063 | 0 |
| 0,5 | -0,0011 | 0 | 0,0011 | 6,4 | 0,0095 | 29,2 | 0,0125 | 30,7 |
| 1 | 0,0003 | 0,5 | 0,0014 | 7,9 | 0,0164 | 63,3 | 0,0121 | 28,7 |
| 2 | 0,0007 | 2,5 | 0,0008 | 5,0 | 0,0256 | 108,9 | 0,0131 | 33,7 |
| 3 | -0,0004 | 0 | 0,0006 | 4,0 | 0,0294 | 127,7 | 0,0136 | 36,1 |
| 6 | 0,0013 | 5,4 | 0,0015 | 8,4 | 0,0333 | 147,0 | 0,0142 | 39,1 |
| 24 | 0,0017 | 7,4 | 0,0020 | 10,8 | 0,0527 | 246,0 | 0,0239 | 176 |
| 48/96 | 0,0024 | 10,9 | 0,0033 | 17,3 | 0,1096 | 524,8 | 0,0147 | 41,6 |
| 120 | 0,0017 | 7,4 | 0,0038 | 19,8 | 0,1110 | 531,7 | 0,0161 | 48,5 |

Absorption der Proben nach unterschiedlichen Zeiten. Zur Berechnung der Konzentration wird die Küvettendifferenz (Abs. bei T =0) vom Meßwert abgezogen.
Nach 12 bzw. 13 Tagen wurde der Versuch abgebrochen. Auf allen Stents war nach Ablauf des Versuches noch eine Beschichtung vorhanden. Zur Bestimmung der in Lösung gegangenen Toluidinblau- bzw. Polylaktidmengen wurden die Stents mit Wasser und Ethanol gespült und danach für 1 h an die Gefriertrocknung gehängt, um sie dann zu wiegen.

| P. | Endgew. | Anfangsgew. | PL + Tb | gel. PL + Tb | gelöst Tolb. | Rest. Tolb. |
|---|---|---|---|---|---|---|
| 1 | 196,5 | 200,9 mg | 24,9 mg | 4,4 mg | 1,76 µg | 8,2 µg |
| 2 | 199,4 | 205,2 | 24,3 mg | 5,8 mg | 2,32 µg | 3,48 µg |
| 3 | 385,4 | 410,8 | 93,6 mg | 25,4 mg | 36,83 µg | 98,8 µg |
| 4 | 191,3 | 194,8 | 14,8 mg | 3,5 mg | 5,52 µg | 17,86 µg |

Bei Konzentrationen von 90 µg Toluidinblau pro ml Tauchlösung sind die freigesetzten Mengen Toluidinblau so gering, daß die Absorptionen an der Meßgrenze des Spektrometers liegen. Bei einer Konzentration von 200 µg/ml liegen die Werte nach wenigen Stunden im meßbaren Bereich. Es empfiehlt sich für die Messung zwei Proben in ein Becherglas (Elutionsgefäß) zu geben, um höhere Absorptionen zu erhalten. Bei der höchsten Polylaktid/Toluidinblaukonzentration scheint sich ein Sättigungseffekt einzustellen. Während die Elutionsrate bei den dünneren Proben nahezu linear verläuft. Auf allen Stents ist nach mehreren Tagen die Beschichtung noch immer zu erkennen.

Nach ca. 2 Wochen war im Mittel etwa 1/4 - 1/5 des gebunden Toluidinblau in Lösung gegangen. Daraus folgt, daß die Proben noch ca. weitere 8 bis 10 Wochen Toluidinblau eluiert hätten.

Die Tauchlösung darf nicht zu zähflüssig sein und sollte gekühlt werden, damit das Chloroform beim Herausziehen der Proben nicht zu schnell verdampft, da sonst die Dicke der Beschichtung zu groß und zu ungleichmäßig wird. Hier scheint die Polylaktidkonzentration in Probe 4 ausreichend (134 mg/ml). Zumal bei höheren Konzentrationen die Lösung extrem viskos wird und sich das Polylaktid nur sehr schwer lösen läßt.

### Beispiel 10

### Beschichtung der Stents nach dem Sprühverfahren

Die nach Beispiel 1 und Beispiel 2 vorbereiteten nicht expandierten Stents werden gewogen und horizontal auf eine dünne Metallstange (d= 0,2mm) gehängt, die auf die Rotationsachse der Rotations- und Vorschubanlage gesteckt ist und mit 28 U/min rotiert. Die Stents werden so aufgebracht, das die Innenseite des Stents die Stange nicht berührt. Bei einer Vorschubamplitude von 2,2,cm und einer Vorschubgeschwindigkeit von 4cm/s und einem Abstand von 6 cm zwischen Stent und Düse wird der Stent mit der jeweiligen Sprühlösung besprüht. Nach dem Trocknen (ca 15 min) bei Raumtemperatur und folgend im Abzug über Nacht wird erneut gewogen.

### Beispiel 11

### Beschichtung der Stents mit reiner Matrix

### Herstellung der Sprühlösung :

176 mg Polylaktid wird eingewogen und mit Chloroform auf 20 g aufgefüllt.
Die Stents werden mit je 3 ml der Sprühlösung besprüht vor und nach dem Sprühen gewogen und die sich ergebende Schichtdicke durch Vermessung unter dem Mikroskop bei 100facher Vergrößerung ermittelt.

| Stent Nr. | Vor Beschichtung | Nach Beschichtung | Masse Beschichtung | Schichtdicke |
|---|---|---|---|---|
| 1 | 0,0193 g | 0,0205 g | 1,2 mg | 10,4 µm |
| 2 | 0,0193 g | 0,0205 g | 1,2 mg | 10,4 µm |
| 3 | 0,0204 g | 0,0216 g | 1,2 mg | 10,4 µm |
| 4 | 0,0206 g | 0,0217 g | 1,1 mg | 10,4 µm |

### Beispiel 12

### Beschichtung der Stents mit reinem Wirkstoff

Herstellung der Sprühlösung : 44 mg Taxol werden in 6g Chloroform gelöst.
Die Stents werden vor und nach dem Sprühen gewogen.

| Stent Nr. | Vor Beschichtung | Nach Beschichtung | Masse Beschichtung |
|---|---|---|---|
| 1 | 0,0194 g | 0,0197 g | 0,30 mg |

### Beispiel 13

### Ermittlung des Elutionsverhaltens in PBS-Puffer

Je ein Stent wird in einem genügend kleinen Gefäß mit 2 ml PBS-Puffer versetzt, mit Parafilm verschlossen und im Trockenschrank bei 37°C inkubiert. Nach Ablauf der gewählten Zeitintervalle wird jeweils der Überstand abpipettiert und dessen UV-Absorption bei 306 nm gemessen.

### Beispiel 14

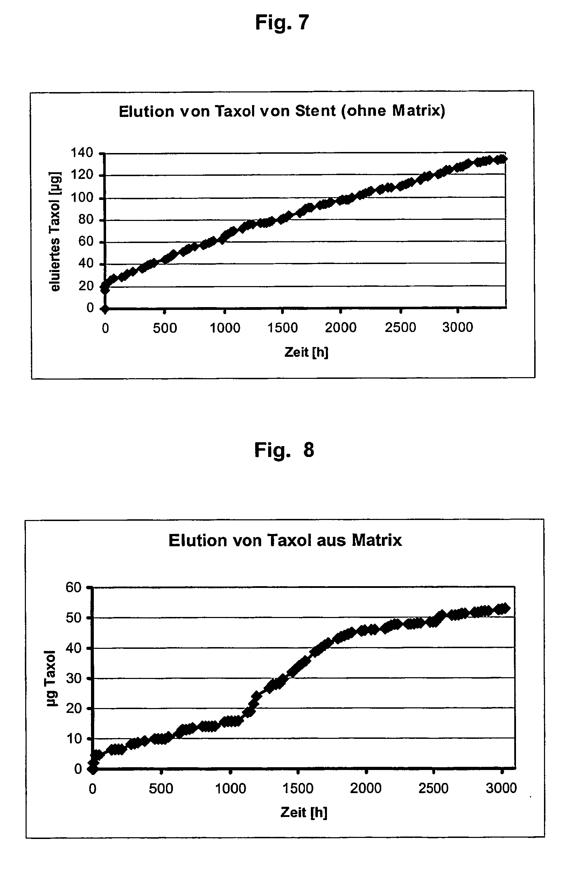

### Beschichtung der hemokompatibel ausgerüsteten Stents mit wirkstoffbeladener Matrix (Figur 7)

Sprühlösung : Polylaktid RG502/Taxol - Lösung wird aus 145,2 mg Polylaktid und 48,4 mg Taxol auf 22 g mit Chloroform aufgefüllt.

| Stent | Sprühlösung | Gewicht vorh (g) | Gewicht nachher(g) | Masse Beschich | Masse Wirkstoff | Wirkstoff µg/mm² | Schicht dicke |
|---|---|---|---|---|---|---|---|
| 1 | 0,8 ml | 0,02180 | 0,02215 | 0,35 mg | 146 µg | 1,97 | 7,9 µm |
| 2 | 0,8 ml | 0,02105 | 0,02142 | 0,37 mg | 154 µg | 2,08 | 6,7 µm |
| 3 | 0,8 ml | 0,02247 | 0,02285 | 0,38 mg | 158 µg | 2,14 | 9,8 µm |
| 4 | 0,8 ml | 0,02395 | 0,02432 | 0,37 mg | 154 µg | 2,08 | 11,0 µm |
| 5 | 0,8 ml | 0,02247 | 0,02286 | 0,39 mg | 163 µg | 2,20 | 9,1 µm |
| 6 | 0,8 ml | 0,02442 | 0,02482 | 0,40 mg | 167 µg | 2,26 | 12,2 µm |

### Beispiel 15

### Beschichtung der Stents mit wirkstoffbeladener Matrix und Wirkstoff als Topcoat (Figur 8)

Basiscoat : 19,8 mg Polylaktid und 6,6 mg Taxol werden mit Chloroform auf 3 g aufgefüllt
Topcoat : 8,8 mg Taxol werden mit Chloroform auf 2 g aufgefüllt.

| Stent | Sprüh-Lösung | Gewicht vorh (g) | Gewicht nachher(g) | Masse Beschich | Masse Wirkstoff | Wirkstoff µg/mm² | Schicht dicke |
|---|---|---|---|---|---|---|---|
| 1 | 0,85 ml | 0,0235 | 0,0238 | 0,30 mg | 131 µg | 1,56 | 9,7µm |
| 2 | 0,85 ml | 0,0260 | 0,0264 | 0,40 mg | 175 µg | 2,09 | 10,1 µm |

### Beispiel 16

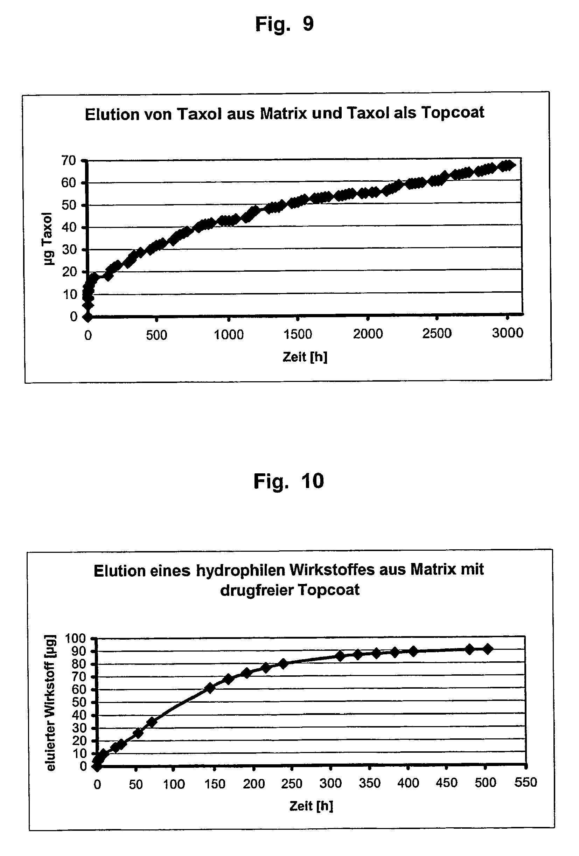

### Beschichtung der Stents mit einen hydrophilen Wirkstoff enthaltenen Polylactid und einer wirkstofffreier Matrix als Topcoat (Figur 9)

### Sprühlösungen

Basiscoat : 22 mg Polylaktid und 22 mg hydrophiler Wirkstoff werden eingewogen und mit Chloroform auf 5 g aufgefüllt.
Topcoat : 22 mg Polylaktid und 22 mg Polystyrol werden eingewogen und mit Chloroform auf 5 g aufgefüllt.

| Sprühlösung | Vor Beschichtung | Nach Beschichtung | Masse Beschichtung | Masse Wirkstoff |
|---|---|---|---|---|
| 0,85 ml | 0,0135 g | 0,0143 g | 0,8 mg | 200 µg |

## Patentansprüche

1. Verbindungen der allgemeinen Formel Ia und Ib 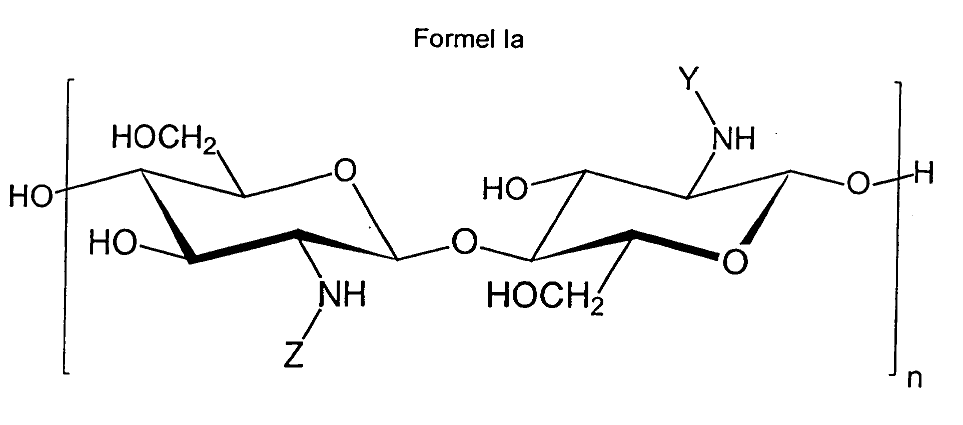 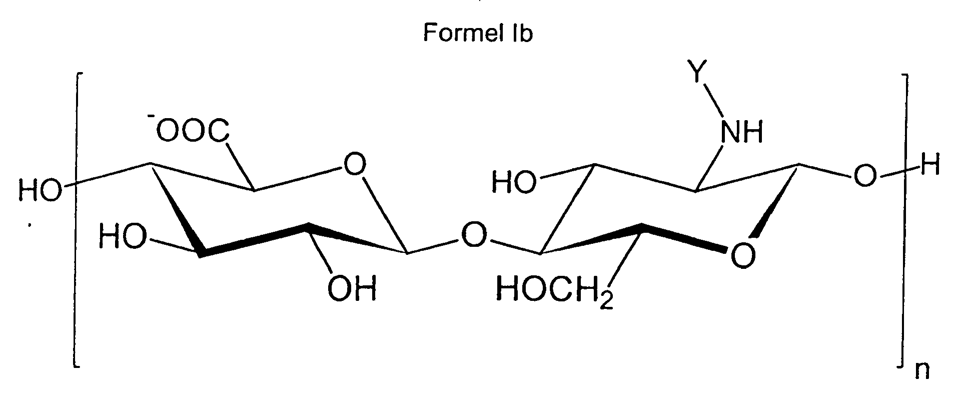 worin
n eine ganze Zahl zwischen 4 und 1050 bedeutet,
Y und Z unabhängig voneinander die Reste -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO darstellen, sowie Salze dieser Verbindungen.

2. Verbindungen der allgemeinen Formel Ia gemäß Anspruch 1, worin Y und Z unabhängig voneinander die Reste -COCH₃, -COC₂H₅, -COC₃H₇, -CH₂COO⁻, -C₂H₄COO⁻, -C₃H₆COO⁻ darstellen, sowie Salze dieser Verbindungen.

3. Verbindungen der allgemeinen Formel Ib gemäß Anspruch 1, worin Y die Reste ―COCH₃, ―COC₂H₅, ―COC₃H₇ darstellt, sowie Salze dieser Verbindungen.

4. Verfahren zur Herstellung der Verbindungen der allgemeinen Formel Ia, **dadurch gekennzeichnet, daß** Heparansulfat und/oder Heparansulfat Heparin mittels Säure im wesentlichen vollständig desulfatiert und anschließend N-acyliert wird.

5. Verfahren zur Herstellung der Verbindungen der allgemeinen Formel Ib, **dadurch gekennzeichnet, daß**
a) Chitosan partiell N-carboxyalkyliert und danach N-acyliert wird, oder
b) Chitosan partiell N-acyliert und danach N-carboxyalkyliert wird, oder
c) Chitin partiell deacetyliert und danach N-carboxyalkyliert wird.

6. Verfahren nach Anspruch 5, **dadurch gekennzeichnet, daß** die Aminogruppen von Chitosan oder Chitin im wesentlichen zur Hälfte acyliert und zur anderen Hälfte carboxyalkyliert werden.

7. Verbindungen der allgemeinen Formel Ia und/oder Ib erhältlich nach einem Verfahren gemäß eines der Ansprüche 4 - 6.

8. Verbindungen der allgemeinen Formel Ia und/oder Ib gemäß eines der Ansprüche 1, 2, 3 oder 7, **dadurch gekennzeichnet, daß** der Gehalt an Sulfatgruppen pro Disaccharideinheit weniger als 0,05 beträgt.

9. Verbindungen der allgemeinen Formel Ia und/oder Ib gemäß eines der Ansprüche 1, 2, 3, 7 oder 8,-**dadurch gekennzeichnet, daß** der Gehalt an freien Aminogruppen weniger als 1 % bezogen auf sämtliche -NH-Y-Gruppen beträgt.

10. Verbindungen der allgemeinen Formel Ia und/oder Ib gemäß eines der Ansprüche 1 - 3, 7 - 9, **dadurch gekennzeichnet, daß** die Abfolge der Zuckerbausteine im wesentlichen alternierend ist.

11. Verbindungen der allgemeinen Formel Ia gemäß eines der Ansprüche 1 - 3, 7 - 10, **dadurch gekennzeichnet, daß** 45% - 55% aller Aminogruppen den Rest Y und die restlichen Aminogruppen den Rest Z tragen.

12. Verwendung der Verbindungen der allgemeinen Formel Ia und/oder Ib zur Herstellung von hemokompatiblen Oberflächen von Medizinprodukten.

13. Verwendung gemäß Anspruch 12, **dadurch gekennzeichnet, daß** die Verbindungen der allgemeinen Formel Ia und/oder Ib kovalent an die Oberfläche gebunden werden.

14. Verwendung von Oligo- und/oder Polysacchariden zur hemokompatiblen Beschichtung von Oberflächen, **dadurch gekennzeichnet, daß** die Oligo- und/oder Polysaccharide zwischen 40% und 60% den Zuckerbaustein N-Acylglucosamin oder N-Acylgalactosamin enthalten und im wesentlichen die restlichen Zuckerbausteine einen Carboxylrest pro Zuckerbaustein aufweisen.

15. Verwendung nach Anspruch 14, **dadurch gekennzeichnet, daß** im wesentlichen jeder zweite Zuckerbaustein der Oligo- und/oder Polysaccharide N-Acylglucosamin oder N-Acylgalactosamin ist.

16. Verwendung gemäß Anspruch 14 oder 15, **dadurch gekennzeichnet, daß** es sich bei N-Acylglucosamin um N-Acetylglucosamin und bei N-Acylgalactosamin um N-Acetylgalactosamin handelt.

17. Verwendung gemäß Anspruch 14, 15 oder 16, **dadurch gekennzeichnet, daß** es sich bei den restlichen Zuckerbausteinen um Uronsäuren handelt.

18. Verwendung gemäß Anspruch 17, **dadurch gekennzeichnet, daß** es sich bei den Uronsäuren im wesentlichen um D-Glucuronsäure und L-Iduronsäure handelt.

19. Verwendung nach einem der Ansprüche 12 - 18, **dadurch gekennzeichnet, daß** die Abfolge der Zuckerbausteine im wesentlichen alternierend ist.

20. Verwendung nach einem der Ansprüche 14 - 19, **dadurch gekennzeichnet, daß** die Oligo- und/oder Polysaccharide im wesentlichen desulfatiertes und im wesentlichen N-acyliertes Heparansulfat sowie partiell N-carboxyalkyliertes und N-acyliertes Chitosan umfassen.

21. Verfahren zur hemokompatiblen Beschichtung von biologischen und/oder künstlichen Oberflächen von Medizinprodukten umfassend die folgenden Schritte:
a) Bereitstellen einer Oberfläche eines Medizinprodukts und
b) Aufbringen mindestens eines Oligo- und/oder Polysaccharids gemäß einem der Ansprüche 1 - 3, 7 - 11, 14 - 20 als hemokompatible Schicht auf diese Oberfläche
und/oder
b') Aufbringen einer biostabilen Schicht auf die Oberfläche des Medizinprodukts oder der hemokompatiblen Schicht.

22. Verfahren nach Anspruch 21, wobei die hemokompatible Schicht oder die biostabile Schicht im Tauch- oder Sprühverfahren mit mindestens einer bioabbaubaren und/oder biostabilen Schicht, welche mindestens einen Wirkstoff kovalent und/oder adhäsiv gebunden enthält, überzogen wird.

23. Verfahren nach Anspruch 21 umfassend den weiteren Schritt c):
c. Aufbringen von mindestens einem Wirkstoff in und/oder auf die hemokompatible Schicht oder die biostabile Schicht.

24. Verfahren nach Anspruch 23, wobei der mindestens eine Wirkstoff im Tauch- oder Sprühverfahren auf und/oder in die hemokompatible Schicht oder die biostabile Schicht auf- und/oder eingebracht wird und/oder der mindestens eine Wirkstoff durch kovalente und/oder adhäsive Kopplung an die hemokompatible Schicht oder die biostabile Schicht gebunden wird.

25. Verfahren nach einem der Ansprüche 21 - 24, umfassend den weiteren Schritt d):
d. Aufbringen mindestens einer bioabbaubaren Schicht und/oder mindestens einer biostabilen Schicht auf die hemokompatible Schicht bzw. die Wirkstoffschicht,
oder
d') Aufbringen mindestens eines Oligo- und/oder Polysaccharids gemäß einem der Ansprüche 1 - 3, 7 - 11, 14 - 20 als hemokompatible Schicht auf die biostabile Schicht bzw. die Wirkstoffschicht.

26. Verfahren nach einem der Ansprüche 21 - 25, umfassend den weiteren Schritt e.):
e. Aufbringen von mindestens einem Wirksoff in und/oder auf die mindestens eine bioabbaubare und/oder biostabile Schicht oder die hemokompatible Schicht.

27. Verfahren nach Anspruch 26, wobei der mindestens eine Wirkstoff itm Tauch- oder Sprühverfahren auf und/oder in die mindestens eine bioabbaubare und/oder biostabile Schicht oder die hemokompatible Schicht auf- und/oder eingebracht wird und/oder der mindestens eine Wirkstoff durch kovalente und/oder adhäsive Kopplung an die mindestens eine bioabbaubare und/oder biostabile Schicht oder hemokompatible Schicht gebunden wird.

28. Verfahren nach einem der Ansprüche 25 - 27, wobei die biostabile und/der bioabbaubare Schicht kovalent und/oder adhäsiv an die Oberfläche des Medizinprodukts gebunden wird und die hemokompatible Schicht kovalent an die biostabile Schicht gebunden wird und diese vollständig oder unvollständig überzieht.

29. Verfahren nach einem der Ansprüche 21 - 28, **dadurch gekennzeichnet, daß** die hemokompatible Schicht aus Heparin nativen Ursprungs regioselektiv hergestellter Derivate unterschiedlicher Sulfatierungsgrade und Acylierungsgrade im Molekulargewichtsbereich des für die antithrombotische Wirkung verantwortlichen Pentasaccharides bis zum Standardmolekulargewicht des käuflichen Heparins von ca. 13kD, aus Heparansulfate und seine Derivate, Oligo- und Polysaccharide der Erythrocytenglycocalix, desulfatiertes und N-reac(et)yliertes Heparin, N-carboxymethyliertes und/oder partiell N-ac(et)yliertes Chitosan sowie aus Mischungen dieser Substanzen besteht.

30. Verfahren nach einem der Ansprüche 21 - 29, **dadurch gekennzeichnet, dass** als bioabbaubare Substanzen für die bioabbaubare Schicht Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherestermultiblockpolymere wie z.B. PEG und Poly(butylenterephtalat. Polypivotolactone, Polyglycolsäuretrimethyl-carbonate Polycaprolactonglycolide, Poly(g-ethylglutamat), Poly(DTH-Iminocarbonat), Poly(DTE-co-DT-carbonat), Poly(Bisphenol A-iminocarbonat), Polyorthoester, Polyglycolsäuretrimethyl-carbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Chitosan und seine Derivate, Heparansulfate uns eine Derivate, Heparine, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen eingesetzt werden.

31. Verfahren nach einem der Ansprüche 21 - 30, **dadurch gekennzeichnet, dass** als biostabile Substanzen für die biostabile Schicht Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate. Chitosane und Copolymere und/oder Mischungen derselben eingesetzt werden.

32. Verfahren nach einem der Ansprüche 21 - 31, **dadurch gekennzeichnet, dass** die Wirkstoffe aus der Gruppe ausgewählt werden, welche Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Thymosin α-1, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, (2-Methylthiazolidin-2,4-dicarbonsäure), Tialin-Na (Natriumsalz von Tialin), Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, Dermicidin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte stimulating hormon (α-MSH), aktiviertes Protein C, IL1-β-Inhibitor, Fumarsäure und deren Ester, Calcipotriol, Tacalcitol, Lapachol, β-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Exemestan, Letrozol, Goserelin, Chephalomannin, Basiliximab, Trastuzumab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere u.a., synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Thialin-Na, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol, Vitamin B1,B2, B6 und B12, Folsäure, Tranirast, Molsidomin, Teepolyphenole, Epicatechingallat, Epigallocatechingallat, Boswellinsäuren und ihre Derivate, Leflunomid, Anakinra, Etanercept, Sulfasalazin Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron., natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A,B und C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, I_asiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Melanocyte Stimulating Hormon (alpha-MSH), Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B umfaßt.

33. Verfahren nach einem der Ansprüche 21 - 32, **dadurch gekennzeichnet, daß** die Aufbringung oder Immobilisierung der Oligo- und/oder Polysaccharide gemäß einem der Ansprüche 1 - 3, 7 - 11, 14 - 20 durch hydrophobe Wechselwirkungen, van der Waals Kräfte, elektrostatische Wechselwirkungen, Wasserstoffbrücken, ionische Wechselwirkungen, Quervernetzung und/oder kovalente Bindung erfolgt.

34. Medizinprodukt erhältlich nach einem Verfahren gemäß eines des Ansprüche 21 - 33.

35. Medizinprodukt, wobei die Oberfläche des Medizinprodukts direkt und/oder über mindestens eine dazwischenliegende biostabile und/oder bioabbaubare Schicht und/oder Wirkstoffschicht mit einer hemokompatiblen Schicht bestehend aus mindestens einem Oligo- und/oder Polysaccharid gemäß den Ansprüchen 1 - 3, 7 - 11, 14 - 20 überzogen ist.

36. Medizinprodukt gemäß Anspruch 35, wobei sich unter der hemokompatiblen Schicht oder zwischen zwei hemokompatiblen Schichten mindestens eine biostabile Schicht und/oder bioabbaubare Schicht befindet.

37. Medizinprodukt, nach Anspruch 35 oder 36, wobei die hemokompatible Schicht mit mindestens einer weiteren, darüberliegenden biostabilen und/oder bioabbaubaren Schicht vollständig oder/und unvollständig überzogen ist.

38. Medizinprodukt gemäß Anspruch 36 oder 37, wobei mindestens eine Wirkstoffschicht zwischen der biostabilen und/oder bioabbaubaren Schicht und der hemokompatiblen Schicht liegt, welche mindestens einen antiproliferativen, antiinflammatorischen und/oder antithrombotischen Wirkstoff kovalent und/oder adhäsiv gebunden enthält.

39. Medizinprodukt gemäß einem der Ansprüche 35 - 38, wobei mindestens ein antiproliferativer, antiinflammatorischer und/oder antithrombotischer Wirkstoff kovalent und/oder adhäsiv in und/oder auf der hemokompatiblen Schicht und/oder der biostabilen und/oder bioabbaubaren Schicht gebunden ist.

40. Medizinprodukt gemäß einem der Ansprüche 35 - 39, **dadurch gekennzeichnet, daß** die verwendeten Wirkstoffe aus der Gruppe ausgewählt werden, welche Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Thymosin α-1, Tremozolomid, Thiotepa, Tialin (2-Methylthiazolidin-2,4-dicarbonsäure), Tialin-Na (Natriumsalz von Tialin) Aunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C; IL1-β-Inhibitor, Fumarsäure und deren Ester, Dermicidin, Calcipotriol, Tacalcitol, Lapachol, β-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Letrozol, Goserelin, Chephalomannin, Trastuzumab, Exemestan, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere u.a., synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plasminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide. Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron., natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrosti-stachin, Ovatodiolide, 4,7-Oxycycloanisomelsäure, Baccharinoide B1, B2, B3 und B7, Tubeimosid, Bruceanole A,B und C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B umfaßt.

41. Medizinprodukt nach Anspruch 40 **dadurch gekennzeichnet, dass** es sich bei den verwendeten Wirkstoffen um Tacrolimus, Pimecrolimus, PI 88, Thymosin α-1, PETN, Baccatine und seine Derivate, Docetaxel, Colchicin, Paclitaxel und seine Derivate, Trapidil, α- und β-Estradiol, Dermicidin, Tialin-Natrium, Simvastatin Macrocyclisches Kohlensuboxid (MCS) und dessen Derivate, Sirolimus, Tyrphostine, D24851, Colchicin, Fumarsäure und ihre Ester, aktiviertes Protein C, Interleukin 1β-Inhibitoren und Melanocyte-stimulating hormon (α-MSH) sowie Mischungen dieser Wirkstoffe handelt.

42. Medizinprodukt nach einem der Ansprüche 34 - 41, **dadurch gekennzeichnet, dass** es sich bei dem Medizinprodukt um Prothesen, Organe, Gefäße, Aorten, Herzklappen, Schläuchen, Organersatzteilen, Implantaten, Fasern, Hohlfasern, Stents, Kanülen, Spritzen, Membranen, Konserven, Blutbehältern, Titerplatten, Herzschrittmachern, Adsorbermedien, Chromatographiemedien, Chromatographiesäulen, Dialysatoren, Verbindungsstücke, Sensoren, Ventile, Zentrifugenkammern, Wäremaustauschern, Endoskope, Filter, Pumpenkammern handelt.

43. Medizinprodukte nach Anspruch 42, **dadurch gekennzeichnet, dass** es sich bei dem Medizinprodukt um einen Stent handelt.

44. Stent gemäß Anspruch 43, wobei die aufgetragenen Polymermengen zwischen 0,01 mg bis 3 mg / Schicht, bevorzugt 0,20 mg bis 1 mg und insbesondere bevorzugt zwischen 0,2 mg bis 0,5 mg / Schicht liegen.

45. Stent gemäß Anspruch 43 oder 44, **dadurch gekennzeichnet, dass** der antiproliferative, antiinflammatorische und/oder antithrombotische Wirkstoff in einer pharmazeutisch aktiven Konzentration von 0,001 - 10 mg pro cm² Stentoberfläche und pro Schicht enthalten ist.

46. Stent gemäß eines der Ansprüche 43 - 45 geeignet zur Verhinderung oder Reduzierung der Restenose.

47. Stent gemäß eines der Ansprüche 43 - 45 geeignet zur kontinuierlichen Freisetzung mindestens eines antiproliferativen, antiinflammatorischen und/oder antithrombotischen Wirkstoffs.

48. Verwendung der Medizinprodukte nach einem der Ansprüche 34 - 41 für den direkten Kontakt mit Blut.

49. Verwendung der Medizinprodukte nach einem der Ansprüche 34 - 41 zur Verhinderung oder Verringerung der Anhaftung von Proteinen auf den beschichteten Oberflächen der Medizinprodukte.

50. Verwendung nach Anspruch 48 oder 49, **dadurch gekennzeichnet, daß** die hemokompatibel beschichtete Oberfläche von Mikrotiterplatten oder anderen Trägermedien für diagnostische Nachweisverfahren die unspezifische Ablagerung von Proteinen verhindert oder verringert.

51. Verwendung nach Anspruch 48 oder 49, **dadurch gekennzeichnet, daß** die hemokompatibel beschichtete Oberfläche von Adsorbermedien oder Chromatographiemedien die unspezifische Ablagerung von Proteinen verhindert oder verringert.

## Claims

1. Compounds of the general formulas Ia and Ib 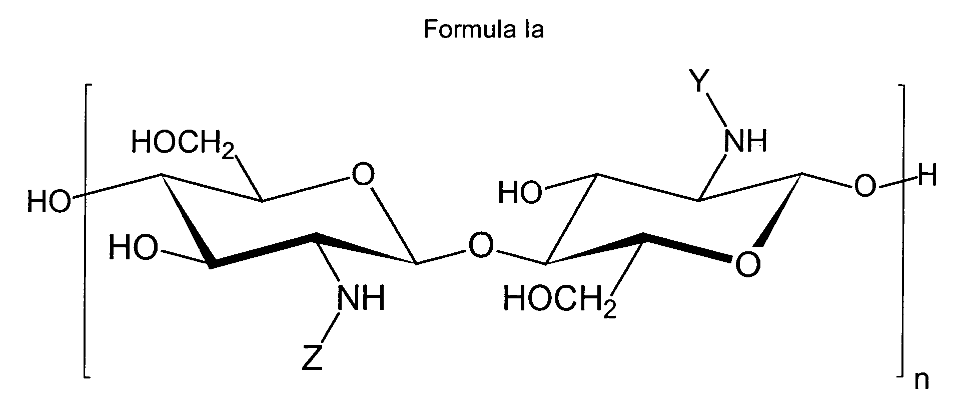 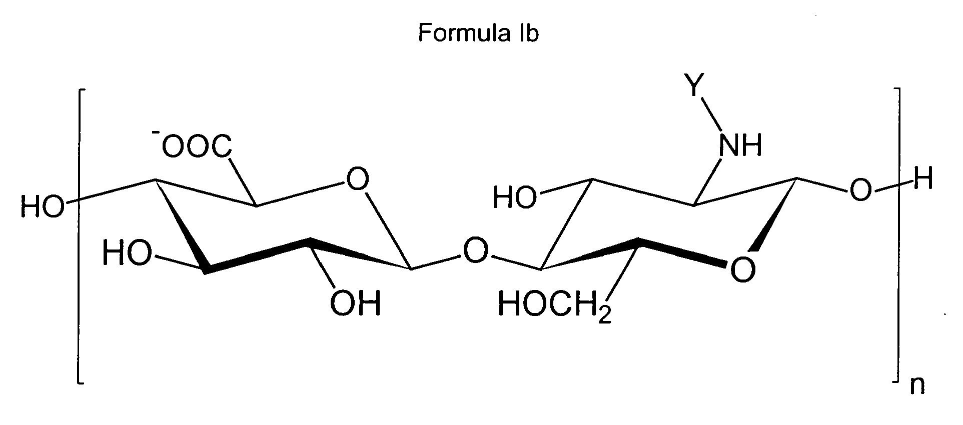 wherein
n is an integer between 4 and 1050,
Y and Z, independently of each other, represent the groups -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻, and salts of said compounds.

2. Compounds of the general formula Ia according to claim 1, wherein Y and Z, independently of each other, represent the groups ―COCH₃, -COC₂H₅, -COC₃H₇, -CH₂COO⁻, -C₂H₄COO⁻, -C₃H₆COO⁻, as well as salts of said compounds.

3. Compounds of the general formula Ib according to claim 1, wherein Y represents the groups -COCH₃, -COC₂H₅, -COC₃H₇, as well as salts of said compounds.

4. Process for the production of the compounds of the general formula Ia, **characterized in that** heparan sulphate and/ or heparin is substantially entirely desulphated and subsequently N-acylated by means of an acid.

5. Process for the production of the compounds of the general formula Ib, **characterized in that**
a) chitosan is partially N-carboxyalkylated and then N-acylated, or
b) chitosan is partially N-acylated and then N-carboxyalkylated, or
c) chitin is partially deacetylated and then N-carboxyalkylated.

6. Process according to claim 5, **characterized in that** substantially half of the amino groups of chitosan or chitin are acylated and the other half thereof are carboxyalkylated.

7. Compounds of the general formula Ia and/or Ib obtainable according to a process according to one of the claims 4 - 6.

8. Compounds of the general formula Ia and/ or Ib according to one of the claims 1, 2, 3 or 7, **characterized in that** the content of sulphate groups per disaccharide unit is less than 0.05.

9. Compounds of the general formula Ia and/ or Ib according to one of the claims 1, 2, 3, 7 or 8, **characterized in that** the content of free amino groups is less than 1 % with respect to all of the -NH-Y groups.

10. Compounds of the general formula Ia and/ or Ib according to one of the claims 1 - 3, 7 - 9, **characterized in that** the sequence of the sugar units is substantially alternating.

11. Compounds of the general formula Ia according to one of the claims 1 - 3, 7 - 10, **characterized in that** 45% - 55% of all of the amino groups carry the group Y and the remaining amino groups carry the group Z.

12. Use of the compounds of the general formula Ia and/ or Ib for the production of hemocompatible surfaces of medical devices.

13. Use according to claim 12, **characterized in that** the compounds of the general formula Ia and/ or Ib are bound covalently to the surface.

14. Use of oligosaccharides and/ or polysaccharides for the hemocompatible coating of surfaces, **characterized in that** the oligosaccharides and/ or polysaccharides contain the sugar unit N-acylglucosamine or N-acylgalactosamine between 40% and 60%, and substantially the remaining sugar units have one carboxyl group per sugar unit.

15. Use according to claim 14, **characterized in that** substantially each second sugar unit of the oligosaccharides and/ or polysaccharides is N-acylglucosamine or N-acylgalactosamine.

16. Use according to claim 14 or 15, **characterized in that** in the case of N-acylglucosamine N-acetylglucosamine and in the case of N-acylgalactosamine N-acetylgalactosamine is concerned.

17. Use according to claim 14, 15 or 16, **characterized in that** the remaining sugar units are uronic acids.

18. Use according to claim 17, **characterized in that** the uronic acids are substantially D-glucuronic acid and L-iduronic acid.

19. Use according to one of the claims 12 - 18, **characterized in that** the sequence of said sugar units is substantially alternating.

20. Use according to one of the claims 14 - 19, **characterized in that** the oligosaccharides and/ or polysaccharides comprise substantially desulphated and substantially N-acylated heparan sulphate as well as partially N-carboxyalkylated and N-acylated chitosan.

21. Method for the hemocompatible coating of biological and/ or artificial surfaces of medical devices comprising the following steps:
a) providing a surface of a medical device and
b) deposition of at least one oligosaccharide and/ or polysaccharide according to one of the claims 1 - 3, 7 - 11, 14 - 20 as hemocompatible layer on this surface
and/ or
b') deposition of a biostable layer on the surface of the medical device or the hemocompatible layer.

22. Method according to claim 21, wherein the hemocompatible layer or the biostable layer is coated by means of a dipping or a spraying method with at least one biodegradable and/ or biostable layer which comprises at least one active agent covalently and/ or adhesively bound.

23. Method according to claim 21, comprising the further step c):
c) deposition of at least one active agent in and/ or on the hemocompatible layer or the biostable layer.

24. Method according to claim 23, wherein the at least one active agent is deposited and/ or implemented by means of dipping or spraying methods on and/ or in the hemocompatible layer or on and/ or in the biostable layer and/ or the at least one active agent is bound by means of covalent and/ or adhesive coupling to the hemocompatible layer or the biostable layer.

25. Method according to one of the claims 21 - 24, comprising the further step d):
d) deposition of at least one biodegradable layer and/ or at least one biostable layer on the hemocompatible layer or the active agent layer, respectively,
or
d') deposition of at least one oligosaccharide and/ or polysaccharide according to one of the claims 1 - 3, 7 - 11, 14 - 20 as hemocompatible layer on the biostable layer or the active agent layer, respectively.

26. Method according to one of the claims 21 - 25, comprising the further step e):
e) deposition of at least one active agent in and/ or on the at least one biodegradable and/ or biostable layer or the hemocompatible layer.

27. Method according to claim 26, wherein the at least one active agent is deposited and/ or implemented by means of dipping or spraying methods on and/ or in the at least one biodegradable and/ or biostable layer or the hemocompatible layer and/ or the at least one active agent is bound by means of covalent and/ or adhesive coupling to the at least one biodegradable and/ or biostable layer or the hemocompatible layer.

28. Method according to one of the claims 25 - 27, wherein the biostable and/ or biodegradable layer is covalently and/ or adhesively bound on the surface of the medical device and the hemocompatible layer is covalently bound to the biostable layer and covers it completely or incompletely.

29. Method according to one of the claims 21 - 28, **characterized in that** the hemocompatible layer comprises heparin of native origin of regioselectively synthesized derivatives of different sulphation coefficients and acylation coefficients in the molecular weight range of the pentasaccharide, which is responsible for the antithrombotic activity, up to the standard molecular weight of the purchasable heparin of approximately 13 kD, of heparan sulphate and its derivatives, oligosaccharides and polysaccharides of the erythrocytic glycocalix, desulphated and N-reac(et)ylated heparin, N-carboxymethylated and/ or partially N-ac(et)ylated chitosan as well as mixtures of these substances.

30. Method according to one of the claims 21 - 29, **characterized in that** polyvalerolactones, poly-ε-decalactones, polylactonic acid, polyglycolic acid, polylactides, polyglycolides, copolymers of the polylactides and polyglycolides, poly□ε-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-paradioxanones, polyanhydrides as polymaleic anhydrides, polyhydroxymethacrylates, fibrin, polycyanoacrylates, polycaprolactonedimethylacrylates, poly-b-maleic acid, polycaprolactonebutyl-acrylates, multiblock polymers such as from oligocaprolactonedioles and oligodioxanonedioles, polyetherester multiblock polymers such as PEG and poly(butyleneterephtalates), polypivotolactones, polyglycolic acid trimethyl-carbonates, polycaprolactone-glycolides, poly(g-ethylglutamate), poly(DTH-iminocarbonate), poly(DTE-co-DT-carbonate), poly(bisphenol-A-iminocarbonate), polyorthoesters, polyglycolic acid trimethyl-carbonates, polytrimethylcarbonates, polyiminocarbonates, poly(N-vinyl)-pyrrolidone, polyvinylalcoholes, polyesteramides, glycolated polyesters, polyphosphoesters, polyphosphazenes, poly[p-carboxyphenoxy)propane], polyhydroxypentane acid, polyanhydrides, polyethyleneoxidepropyleneoxide, soft polyurethanes, polyurethanes with amino acid rests in the backbone, polyetheresters such as polyethyleneoxide, polyalkeneoxalates, polyorthoesters as well as their copolymers, lipids, carrageenanes, fibrinogen, starch, collagen, protein based polymers, polyamino acids, synthetic polyamino acids, zein, modified zein, polyhydroxyalkanoates, pectic acid, actinic acid, modified and non modified fibrin and casein, carboxymethylsulphate, albumin, moreover hyaluronic acid, chitosan and its derivatives, heparansulphates and its derivatives, heparin, chondroitinesulphate, dextran, b-cyclodextrines, copolymers with PEG and polypropyleneglycol, gummi arabicum, guar, gelatine, collagen, collagen-N-Hydroxysuccinimide, lipids, phospholipids, modifications and copolymers and/ or mixtures of these substances are used as biodegradable substances for the biodegradable layer.

31. Method according to one the claims 21 - 30, **characterized in that** polyacrylic acid and polyacrylates such as polymethylmethacrylate, polybutylmethacrylate, polyacrylamide, polyacrylonitriles, polyamides, polyetheramides, polyethylenamine, polyimides, polycarbonates, polycarbourethanes, polyvinyl ketones, polyvinyl halogenides, polyvinylidene halogenides, polyvinyl ethers, polyvinyl aromates, polyvinyl esters, polyvinyl pyrrollidones, polyoxymethylenes, polytetramethylene oxide, polyethylene, polypropylene, polytetrafluoro-ethylene, polyurethanes, polyether urethanes, silicone-polyether-urethanes, silicone-polyurethanes, silicone-polycarbonate-urethanes, polyolefine elastomers, polyisobutylenes, fluorosilicones, carboxymethylchitosans, polyaryletherether ketones, polyetherether ketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulphones, epoxy resins, ABS resins, EPDM gums, silicones such as polysiloxanes, polydimethylsiloxanes, polyvinyl halogens and copolymers, cellulose ethers, cellulose triacetates, chitosans and copolymers and/ or mixtures of these substances are used as biostable substances for the biostable layer.

32. Method according to one of the claims 21 - 31, **characterized in that** the active agents are chosen from the group which contains sirolimus (rapamycin), everolimus, pimecrolimus, somatostatin, tacrolimus, roxithromycin, (daunamycin), ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, (etoposide), teniposide, nimustine, carmustine, lomustine, cyclophosphamide, 4-hydroxyoxycyclophosphamide, estramustine, melphalan, ifosfamide, trofosfamide, thymosin α-1, chlorambucil, bendamustine, dacarbazine, busulfan, procarbazine, treosulfan, (temozolomide), thiotepa, daunorubicin, doxorubicin, aclarubicin, epirubicin, idarubicin, bleomycin, mitomycin, dactinomycin, methotrexate, fludarabine, 2-methylthiazolidine-2,4-dicarboxylic acid, tialin-Na (sodium salt of tialin), fludarabine-5'-dihydrogenphosphate, cladribine, mercaptopurine, thioguanine, cytarabine, fluorouracil, gemcitabine, capecitabine, docetaxel, carboplatin, cisplatin, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethemide, adriamycin, azithromycin, spiramycin, cepharanthin, dermicidin, SMC proliferation inhibitor-2w, epothilone A and B, mitoxantrone, azathioprine, mycophenolate mofetil, c-myc antisense, b-myc antisense, betulinic acid, camptothecin, PI-88 (sulphated oligosaccharide), melanocyte stimulating hormone (α-MSH), activated protein C, IL1-β inhibitor, fumaric acid and its esters, calcipotriol, tacalcitol, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, goserelin, chephalomannine, basiliximab, trastuzumab, daclizumab, selectin (cytokine antagonist), CETP inhibitor, cadherins, cytokinin inhibitors, COX-2 inhibitor, NFkB, angiopeptin, ciprofloxacin, camptothecin, fluroblastin, monoclonal antibodies, which inhibit the muscle cell proliferation, bFGF antagonists, probucol, prostaglandins, 1,11-dimethoxycanthin-6-one, 1-hydroxy-11-methoxycanthin-6-one, scopoletin, colchicine, NO donors such as pentaerythritol tetranitrate and sydnonimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostins), cyclosporine A, paclitaxel and derivatives thereof such as 6-α-hydroxy-paclitaxel, baccatin, taxotere and other macrocyclic oligomers of carbon suboxide (MCS) obtained synthetically and from native sources and derivatives thereof, mofebutazone, acemetacin, diclofenac, lonazolac, dapsone, o-carbamoyl-phenoxy-acetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocadazole, S 100 protein, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plaminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefoxitin, tobramycin, gentamycin, penicillins such as dicloxacillin, oxacillin, sulfonamides, metronidazole, antithrombotics such as argatroban, aspirin, abciximab, synthetic antithrombin, bivalirudin, coumadin, enoxaparin, desulphated and N-reacetylated heparin, tissue plasminogen activator, GpIIb/IIIa platelet membrane receptor, factor Xₐ inhibitor antibody, heparin, hirudin, r-hirudin, PPACK, protamine, thialin-Na, prourokinase, streptokinase, warfarin, urokinase, vasodilators such as dipyridamol, trapidil, nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65, NF-kB or Bcl-xL antisense oligonucleotides, halofuginone, nifedipine, tocopherol, vitamin B1,B2, B6 and B12, folic acid, tranilast, molsidomine, tea polyphenols, epicatechin gallate, epigallocatechin gallate, Boswellic acids and derivatives thereof, leflunomide, anakinra, etanercept, sulfasalazine, dicloxacillin, tetracycline, triamcinolone, mutamycin, procainimide, retinoic acid, quinidine, disopyrimide, flecainide, propafenone, sotolol, amidorone, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprotozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-α-senecioyloxychaparrin, taxamairin A and B, regenilol, triptolide, moreover cymarin, apocymarin, aristolochic acid, anopterin, hydroxyanopterin, anemonin, protoanemonin, berberine, cheliburin chloride, cicutoxin, sinococuline, combrestatin A and B, cudraisoflavone A, curcumin, dihydronitidine, nitidine chloride, 12-β-hydroxypregnadien-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, oxoushinsunine, aristolactam-All, bisparthenolidine, periplocoside A, ghalakinoside, deoxypsorospermin, psycorubin, ricin A, sanguinarine, manwu wheat acid, methylsorbifolin, melanocyte stimulating hormone (alpha-MSH), sphatheliachromen, stizophyllin, akagerine, dihydrousambaraensine, hydroxyusambarine, strychnopentamine, strychnophylline, usambarine, usambarensine, berberine, liriodenine, daphnoretin, lariciresinol, methoxylariciresinol, syringaresinol, umbelliferone, afromoson, acetylvismione B, desacetylvismione A, vismione A and B.

33. Method according to one of the claims 21 - 32, **characterized in that** the deposition or the immobilization of the oligosaccharides and/ or polysaccharides according to one of the claims 1 - 3, 7 - 11, 14 - 20 is achieved by means of hydrophobic interactions, van der Waals forces, electrostatic interactions, hydrogen bonds, ionic interactions, cross-linking and/ or covalent bonding.

34. Medical device obtainable by one method according to one of the claims 21 - 33.

35. Medical device, wherein the surface of the medical device is coated directly and/ or by means of at least one interjacent biostable and/ or biodegradable layer and/ or active agent layer with a hemocompatible layer comprising at least one oligosaccharide and/ or polysaccharide according to the claims 1 - 3, 7 - 11, 14 - 20.

36. Medical device according to claim 35, wherein at least one biostable and/ or biodegradable layer is present under the hemocompatible layer or between two hemocompatible layers.

37. Medical device according to claims 35 or 36, wherein the hemocompatible layer is coated completely and/ or incompletely with at least another, suprajacent biostable and/ or biodegradable layer.

38. Medical device according to claims 36 or 37, wherein at least one active agent layer is present between the biostable and/ or biodegradable layer and the hemocompatible layer, which comprises at least one antiproliferative, anti-inflammatory and/ or antithrombotic active agent covalently and/ or adhesively bound.

39. Medical device according to one of the claims 35 - 38, wherein at least one antiproliferative, anti-inflammatory and/ or antithrombotic active agent is bound covalently and/ or adhesively in and/ or on the hemocompatible layer and/ or the biostable and/ or biodegradable layer.

40. Medical device according to one of the claims 35 - 39, **characterized in that** the used active agents are chosen from the group, which contains sirolimus (rapamycin), everolimus, pimecrolimus, somatostatin, tacrolimus, roxithromycin, daunamycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, 4-hydroxyoxycyclophosphamide, estramustine, melphalan, ifosfamide, trofosfamid, chlorambucil, bendamustine, dacarbazine, busulfan, procarbazine, treosulfan, thymosin α-1, temozolomide, thiotepa, thialin (2-methylthiazolidine-2,4-dicarboxylic acid), thialin-Na (sodium salt of thialin), aunorubicin, doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, gemcitabine, capecitabine, docetaxel, carboplatin, cisplatin, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethemide, adriamycin, azithromycin, spiramycin, cepharanthin, smc proliferation inhibitor-2w, epothilone A and B, azathioprine, mycophenolate mofetil, c-myc antisense, b-myc antisense, betulinic acid, camptothecin, PI-88 (sulphated oligosaccharide), melanocyte stimulating hormone (α-MSH), activated protein C, IL1-β inhibitor, fumaric acid and its esters, dermicidin, calcipotriol, tacalcitol, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, goserelin, chephalomannine, trastuzumab, basiliximab, daclizumab, selectin (cytokine antagonist), CETP inhibitor, cadherins, cytokinin inhibitors, COX-2 inhibitor, NFkB, angiopeptin, ciprofloxacin, camptothecin, fluroblastin, monoclonal antibodies, which inhibit the muscle cell proliferation, bFGF antagonists, probucol, prostaglandins, 1,11-dimethoxycanthin-6-one, 1-hydroxy-11-methoxycanthin-6-one, scopoletin, colchicine, NO donors such as pentaerythritol tetranitrate and sydnonimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostins), cyclosporine A, paclitaxel and derivatives thereof such as 6-α-hydroxy-paclitaxel, baccatin, taxotere and others, synthetically and from native sources obtained macrocyclic oligomers of carbon suboxide (MCS) and derivatives thereof, mofebutazone, acemetacin, diclofenac, lonazolac, dapsone, o-carbamoyl-phenoxy-acetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocadazole, S 100 protein, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plaminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefotixin, tobramycin, gentamycin, penicillins such as dicloxacillin, oxacillin, sulfonamides, metronidazole, antithrombotics such as argatroban, aspirin, abciximab, synthetic antithrombin, bivalirudin, coumadin, enoxaparin, desulphated and N-reacetylated heparin, tissue plasminogen activator, GpIIb/IIIa platelet membrane receptor, factor Xₐ inhibitor antibody, heparin, hirudin, r-hirudin, PPACK, protamine, prourokinase, streptokinase, warfarin, urokinase, vasodilators such as dipyramidole, trapidil, nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65, NF-kB or Bcl-xL antisense oligonucleotides, halofuginone, nifedipine, tocopherol, molsidomine, tea polyphenols, epicatechin gallate, epigallocatechin gallate, Boswellic acids and derivatives thereof, leflunomide, anakinra, etanercept, sulfasalazine, etoposide, dicloxacillin, tetracycline, triamcinolone, mutamycin, procainimid, retinoic acid, quinidine, disopyrimide, flecainide, propafenone, sotolol, amidorone, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprotozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-α-senecioyloxychaparrin, taxamairin A and B, regenilol, triptolide, moreover cymarin, apocymarin, aristolochic acid, anopterine, hydroxyanopterine, anemonin, protoanemonin, berberine, cheliburin chloride, cicutoxin, sinococuline, combrestratin A and B, cudraisoflavone A, curcumin, dihydronitidine, nitidine chloride, 12-β-hydroxypregnadien-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, bisparthenolidine, oxoushinsunine, aristolactam-All, bisparthenolidine, periplocoside A, ghalakinoside, ursolic acid, deoxypsorospermin, psycorubin, ricin A, sanguinarine, manwu wheat acid, methylsorbifolin, sphatheliachromen, stizophyllin, mansonine, strebloside, akagerine, dihydrousambaraensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferone, afromoson, acetylvismione B, desacetylvismione A, vismione A and B.

41. Medical device according to claim 40, **characterized in that** in the case of the used active agents tacrolimus, pimecrolimus, PI 88, thymosin α-1, PETN, baccatins and its derivatives, docetaxel, colchicine, paclitaxel and its derivatives, trapidil, α- and β-estradiol, dermicidin, thialin-sodium, simvastatin, macrocyclic suboxide (MCS) and its derivatives, sirolimus, tyrphostins, D24851, colchicine, fumaric acid and its esters, activated protein C, interleukin 1β inhibitors and melanocyte stimulating hormone (α-MSH) as well as mixtures of these active agents are concerned.

42. Medical device according to one of the claims 34 - 41, **characterized in that** the medical device comprises prostheses, organs, vessels, aortas, heart valves, tubes, organ spare parts, implants, fibers, hollow fibers, stents, hollow needles, syringes, membranes, tinned goods, blood containers, titrimetric plates, pacemakers, adsorbing media, chromatography media, chromatography columns, dialyzers, connection parts, sensors, valves, centrifugal chambers, recuperators, endoscopes, filters, pump chambers.

43. Medical device according to claim 42, **characterized in that** the medical device is a stent.

44. Stents according to claim 43, wherein the polymer is deposited in amounts between 0.01 mg to 3 mg / layer, preferred between 0.20 mg to 1 mg and especially preferred between 0.2 mg to 0.5 mg / layer.

45. Stent according to claims 43 or 44, **characterized in that** the antiproliferative, anti-inflammatory and/ or antithrombotic active agent is comprised in a pharmaceutically active concentration of 0.001 - 10 mg per cm² stent surface and per layer.

46. Stent according to one of the claims 43 - 45 suitable for the prevention or reduction of restenosis.

47. Stent according to one of the claims 43 - 45 suitable for continuous release of at least one antiproliferative, anti-inflammatory and/ or antithrombotic active agent.

48. Use of the medical devices according to one of the claims 34 - 41 for the direct contact with blood.

49. Use of the medical devices according to one of the claims 34 - 41 for prevention or reduction of the adhesion of proteins on the coated surfaces of the medical devices.

50. Use according to claims 48 or 49, **characterized in that** the hemocompatibly coated surface of microtiter plates or other carrier means used for diagnostic detection methods prevents or reduces the unspecific deposition of proteins.

51. Use according to claims 48 or 49, **characterized in that** the hemocompatibly coated surface of adsorber media or chromatography media prevents or reduces the unspecific deposition of proteins.

## Revendications

1. Composés selon les formules générales Ia et Ib 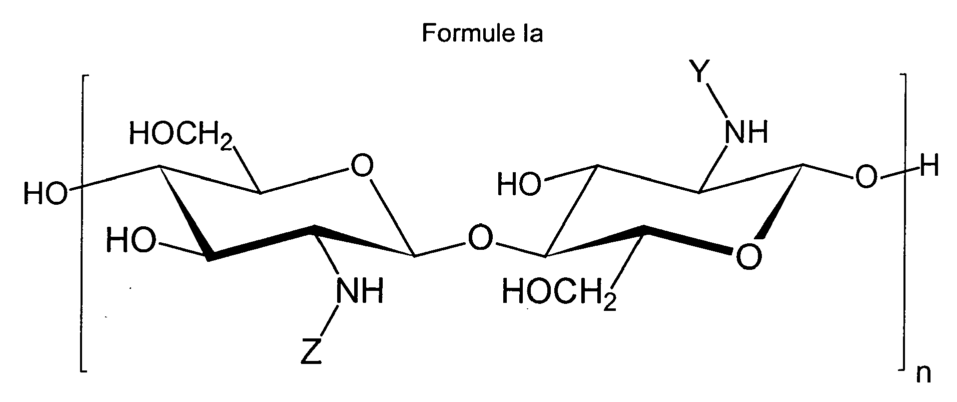 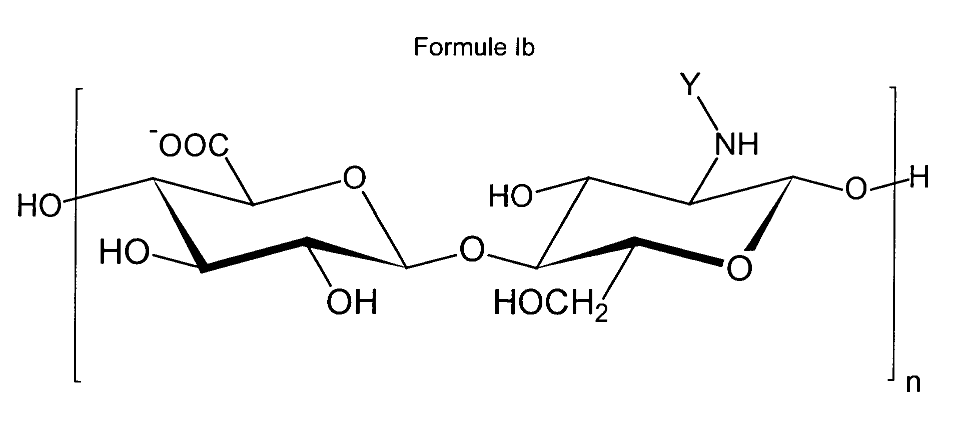 où
n représente un entier entre 4 et 1050,
Y et Z représentent, indépendamment l'un de l'autre, les résidus -CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻ ainsi que des sels de ces composés.

2. Composés selon la formule générale Ia suivant la revendication 1, où Y et Z représentent, indépendamment l'un de l'autre, les résidus ―COCH₃, -COC₂H₅, -COC₃H₇, -CH₂COO⁻, -C₃H₆COO⁻, ainsi que des sels de ces composés.

3. Composés selon la formule générale Ib suivant la revendication 1, où Y représente les résidus ―COCH₃, ―COC₂H₅, ―COC₃H₇, ainsi que des sels de ces composés.

4. Méthode de production des composés de la formule générale Ia, **caractérisée en ce que** l'héparane sulfate et/ ou l'héparine sont/ est, pour l'essentiel, complètement desulfatée(s) et sont/ est ensuite N-acylée(s).

5. Méthode de production des composés de la formule générale Ib, **caractérisée en ce que**
a) le chitosane est partiellement n-carboxylé et ensuite N-acylé , ou
b) le chitosane est partiellement N-acylé et ensuite N-carboxylé , ou
b) la chitine est partiellement N-acylée et ensuite N-carboxylée

6. Méthode suivant la revendication 5, **caractérisée en ce que** les groupes amino du chitosane ou de la chitine sont, pour l'essentiel, à moitié acylés et à l'autre moitié carboxyalkylés .

7. Composés conformes aux formules générales la et/ ou Ib, qui peuvent être obtenus suivant une méthode selon l'une des revendications 4 - 6.

8. Composés conformes aux formules générales Ia et/ ou Ib selon l'une des revendications 1, 2, 3 ou 7, **caractérisés en ce que** la quantité de groupes sulfate par unité de disaccharide est moins de 0,05.

9. Composés conformes aux formules générales Ia et/ ou Ib selon l'une des revendications 1, 2, 3, 7 ou 8, **caractérisés en ce que** la quantité de groupes amino libres est de moins de 1 % relatif à la totalité de groupes -NH-Y.

10. Composés conformes aux formules générales Ia et/ ou Ib selon l'une des revendications 1 - 3, 7 - 9, **caractérisés en ce que** la séquence des sucres est, pour l'essentielle, une séquence alternée.

11. Composés conformes aux formules générales Ia et/ ou Ib selon l'une des revendications 1 - 3, 7 - 10 **caractérisés en ce que** de 45 % à 55 % de tous les groupes amino portent le résidu Y et les autres groupes amino portent le résidu Z.

12. Utilisation des composés conformes aux formules générales Ia et/ ou Ib pour la fabrication de surfaces hémocompatibles de produits médicaux.

13. Utilisation selon la revendication 12, **caractérisée en ce que** les composés conformes aux formules générales Ia et/ ou Ib sont liés covalemment à la surface.

14. Utilisation de oligosaccharides et/ ou polysaccharides pour le revêtement hémocompatible de surfaces, **caractérisée en ce que** les oligosaccharides et/ou polysaccharides comportent entre 40 % et 60 % des sucres N-acylglucosamine ou N-acylgalactosamine et les autres sucres, pour l'essentielle, comportent un résidu de carboxyle par sucre.

15. Utilisation selon la revendication 14, **caractérisée en ce que**, pour l'essentielle, un sucre sur deux est l'oligosaccharide et/ ou le polysaccharide N-acylglucosamine ou N-acylgalactosamine.

16. Utilisation selon la revendication 14 ou 15, **caractérisée en ce que** la N-acylglucosamine est la N-acetylglucosamine et la N-acylgalactosamine est la N-acetylgalactosamine.

17. Utilisation selon les revendications 14, 15 ou 16, **caractérisée en ce que** les autres sucres sont des acides uroniques.

18. Utilisation selon la revendication 17, **caractérisée en ce que** les acides uroniques sont, pour l'essentielle, l'acide D-glucuronique et l'acide L-iduronique.

19. Utilisation selon l'une des revendications 12 - 18, **caractérisée en ce que** la séquence des sucres est, pour l'essentielle, une séquence alternée.

20. Utilisation selon l'une des revendications 14 - 19, **caractérisée en ce que** les oligosaccharides et/ ou les polysaccharides comprennent de l'héparane sulfate, qui est essentiellement désulfatée et essentiellement n-acylée, ainsi que du chitosane partiellement n-carboxalkylé et N-acylé.

21. Méthode de revêtement hémocompatible des surfaces biologiques et/ ou artificielles de produits médicaux comprenant les étapes suivantes :
a) la mise à disposition d'une surface d'un produit médical et
b) l'application d'au moins un oligosaccharide et/ ou polysaccharide selon l'une des revendications 1 - 3, 7 - 11, 14 - 20 en qualité de couche hémocompatible sur cette surface
et/ ou
b') l'application d'une couche biostable sur la surface du produit médical ou de la couche hémocompatible.

22. Méthode selon la revendication 21, où la couche hémocompatible ou la couche biostable sont revêtues, par immersion ou par pulvérisation, d'au moins une couche biodégradable et/ ou biostable qui contient au moins une substance active covalemment et/ ou adhésivement liée.

23. Méthode selon la revendication 21 comprenant l'étape ultérieure c)
c.) l'introduction d'au moins une substance active dans et/ ou son application sur la couche hémocompatible ou la couche biostable.

24. Méthode selon la revendication 23, où ladite au moins une substance active est appliquée sur et/ ou introduite dans la couche hémocompatible ou appliquée sur et/ ou introduite dans la couche biostable par immersion ou par pulvérisation et/ ou ladite au moins une substance active est liée à la couche hémocompatible ou à la couche biostable par couplage covalent et/ ou adhésif.

25. Méthode selon l'une des revendications 21 - 24, comprenant l'étape ultérieure d)
d.) l'application d'au moins une couche biodégradable et/ ou d'au moins une couche biostable sur la couche hémocompatible ou respectivement sur la couche contenant la substance active.
ou
d') l'application d'au moins un oligosaccharide et/ ou polysaccharide selon l'une des revendications 1 - 3, 7 - 11, 14 - 20 en qualité de couche hémocompatible sur la couche biostable ou respectivement sur la couche contenant la substance active.

26. Méthode selon l'une des revendications 21 - 25, comprenant l'étape ultérieure e)
e.) l'introduction d'au moins une substance active dans et/ ou son application sur la dite au moins une couche biodégradable et/ ou biostable ou la couche hémocompatible.

27. Méthode selon la revendication 26, où ladite au moins une substance active est appliquée sur et/ ou introduite dans ladite au moins une couche biodégradable et/ ou biostable ou sur et/ ou dans la couche hémocompatible par immersion ou par pulvérisation et/ ou ladite au moins une substance active est liée à ladite au moins une couche biodégradable et/ ou biostable ou hémocompatible par couplage covalent et/ ou adhésif.

28. Méthode selon l'une des revendications 25 - 27, où la couche biostable et/ou la couche biodégradable sont liées covalemment et/ ou adhésivement à la surface du produit médical et où la couche hémocompatible est covalemment liée à la couche biostable et recouvre celle-là complètement ou incomplètement.

29. Méthode selon l'une des revendications 21 - 28, **caractérisée en ce que** la couche hémocompatible consiste en des dérivés produits de façon regioséléctive à partir de héparine d'origine native ayant des dégrées de sulfatation et de acylation différentes quant au poids moléculaire du pentasaccharide responsable de l'effet antithrombotique jusqu'au standard de poids moléculaire de l'héparine commerciale de ca. 13 kD ; en héparane sulfate et ses dérivés, en oligosaccharides et polysaccharides de la glycocalix des érythrocytes, en héparine désulfatée et N-réac(ét)ylée et en chitosane , N-carboxymethylé et/ ou partiellement n-ac(ét)ylé ainsi que en des mélanges de ces substances.

30. Méthodes selon l'une des revendications 21 - 29, **caractérisée en ce que** polyvalérolactone, poly-ε-décalatone, l'acide poly-lactonique, l'acide polyglycolique, polylactides, polyglycolides, copolymères des polylactides et polyglycolides, poly-ε-caprolactone, l'acide butyrique polyhydroxy, polyhydroxy butyrates, polyhydroxyvalérates, polyhydroxybutyrates-co-valérates, poly(1,4-dioxane-2,3-dione), poly(1,3-dioxane-2-one), poly-para-dioxanone, polyanhydride comme les anhydrides de l'acide polymaléique, polyhydroxyméthacrylates, fibrine, polycyanoacrylates, polycaprolactone diméthylacrylates, poly-b-acide maléique, acrylates de polycaprolactonbutyl, polymères séquencés multiples come p.e. de oligocaprolactondioles et oligodioxanedioles, polymères séquencés multiples de polyesters comme p.e. PEG et poly(butylène téréphtalate), polypivotolactones, triméthyl carbonates de l'acide polyglycolique, polycaprolactone glycolides, poly(g-ethylglutamate), poly(DTH-imino-carbonate), poly(DTE-co-DT-carbonate), poly(bisphenol A-imino-carbonate), polyorthoesters, acide polyglycolique triméthyl-carbonates, poly-triméthyl-carbonates, polyimino-carbonates, poly(N-vinyle)-pyrrolidone, les alcools de polyvinyle, polyesteramides, polyesters glycolés, polyphosphoesters, polyphosphazenes, poly[p-carboxyphenoxy)propane], acide valérianique polyhydroxy, polyanhydrides, oxyde de polyéthylène-oxyde de propylène, polyuréthanes souples, polyuréthanes ayant un résidu d'acide aminé dans le tronc, polyétheresters comme l'oxyde de polyéthylène, l'oxalate polyakène, polyorthoesters ainsi que leurs copolymères, lipides, carraghénanes, fibrinogène, amidon, collagène, polymères à base de protéines, les acides polyaminés, les acides polyaminés synthétiques, zéine, zéine modifiée, polyhydroxyalkanoates, l'acide pectique, l'acide actique, fibrine et caséine modifiées et non modifiées, carboxyméthyl sulfate, albumine, de plus l'acide hyaluronique, chitosane et ses dérivés, sulfates d'héparane et ses dérivés, héparine, chondroïtine sulfates, dextrane, b-cyclodextrines, et copolymères avec PEG et polypropylène glycol, gomme arabique, guar, gelatine, collagène, collagène-N-hydroxy succinimide, lipides, phospholipides, modifications et copolymères et/ou des mélanges des substance mentionnées ci-dessus peuvent être utilisés.

31. Méthode selon l'une des revendications 21 - 30, **caractérisée en ce que** les substances qui peuvent être utilisées en qualité de substances biostables pour la couche biostable sont les suivantes : l'acide polyacrylique et polyacrylates comme polyméthylméthacrylate, polybutylméthacrylate, polyacrylamide, polyacrylonitriles, polyamides, polyétheramides, polyéthylène amines, polyimides, polycarbonates, polycarbo-uréthane, cétones de polyvinyle, halogénides de polyvinyle, esters de polyvinyle, pyrrollidones de polyvinyle, polyoxymethylènes, oxyde de polytétraméthylène, polyéthylène, polypropylène, polytétrafluoréthylène, polyuréthanes, polyétheruréthanes, polyétheruréthanes de silicone, polyuréthanes de silicone, polycarbonate-uréthanes de silicone, polyoléfine élastomères, polyisobutylènes, fluorosilicones, carboxyméthyl chitosanes, cétones de polyarylétheréther, cétones de polyétheréther, polyéthylène téréphtalate, polyvalérates, carboxyméthyl cellulose, cellulose, viscose, triacétates de viscose, nitrates de cellulose, acétates de cellulose, hydroxyethyl cellulose, butyrates de cellulose, acetatebutyrates de cellulose, copolymères d'éthylène-vinyle acétate, polysulfones, résines époxy, résines ABS, gommes EPDM, silicones comme les polysiloxanes, le polydiméthylsiloxanes, les halogènes de polyvinyle et copolymères, éthers de cellulose, triacétates de cellulose, chitosanes et copolymères et/ ou des mélanges de ceux-ci.

32. Méthode selon l'une des revendication 21 - 31, **caractérisée en ce que** les substances actives sont choisi parmi le groupe comprenant les substances suivantes : sirolimus (rapamycine), everolimus, pimecrolimus, somatostatine, tacrolimus, roxithromycine, daunamycine, ascomycine, bafilomycine, erythromycine, midecamycine, josamycine, concanamycine, clarithromycine, troléandomycine, folimycine, cerivastatine, simvastatine, lovastatine, fluvastatine, rosuvastatine, atorvastatine, pravastatine, pitavastatine, vinblastine, vincristine, vindesine, vinorelbine, etoposide, teniposide, nimustine, carmustine, lomustine, cyclophosphamide, 4-hydroxyoxycyclophosphamide, estramustine, melphalan, ifosfamide, trofosfamide, thymosine α-1, chlorambucil, bendamustine, dacarbazine, busulfan, procarbazine, treosulfan, temozolomide, thiotépa, daunorubicine, doxorubicine, aclarubicine, épirubicine, mitoxantrone, idarubicine, bléomycine, mitomycine, dactinomycine, méthotrexate, fludarabine, (acide 2-méthylthiazolidine-2,4-dicarbonique), thialin-Na (sel de sodium de thialin), fludarabine-5'-dihydrogénophosphate, cladribine, mercaptopurine, thioguanine, cytarabine, fluorouracil, gemcitabine, capecitabine, docétaxel, carboplatine, cisplatine, oxaliplatine, amsacrine, irinotécan, topotécan, hydroxycarbamide, miltéfosine, pentostatine, aldesleukine, trétinoïne, asparaginase, pegaspargase, anastrozole, exémestane, létrozole, formestane, aminoglutéthimide, adriamycine, azithromycine, spiramycine, cepharanthine, dermicidine, inhibiteur 2w de la prolifération des cellules musculaires lisses, épothilone A et B, mitoxantrone, azathioprine, mycophénolate mofétil, c-myc antisens, b-myc antisens, acide betulinique, camptothécine, PI-88 (oligosaccharide sulfaté), melanocyte-stimulating hormone (α-MSH), protéine C activée, inhibiteur de IL1-β, acide fumarique et ses esters, calcipotriol, tacalcitol, lapachol, β-lapachone, podophyllotoxine, bétuline, 2-ethylhydrazide de l'acide podophyllique, molgramostime (rhuGM-CSF), peginterféron α-2b, lénograstime (r-HuG-CSF), filgrastime, macrogol, goséréline, cephalomannine, basiliximab, trastuzumab, daclizumab, sélectine (cytokine antagoniste), inhibiteur de la CETP, cadhérine, inhibiteurs de la cytokine, inhibiteur de la COX-2, NFkB, angiopeptine, ciprofloxacine, camptothécine, fluroblastine, anticorps monoclonales inhibitant la prolifération des cellules musculaires, bFGF-antagonistes, probucol, prostaglandine, 1,11-diméthoxycanthin-6-on, 1-hydroxy-11-méthoxycanthin-6-on, scopoletine, colchicine, NO donneurs comme le pentaerythrityl tetranitrate et les sydnoimines, dérivés de S-Nitroso, tamoxifène, staurosporine, β-estradiol, α-estradiol, estriol, estrone, ethinylestradiol, fosfestrol, medroxy-progestérone, cipionates d'estradiol, benzoates d'estradiol, tranilast, kamebakaurin et autres terpénoïdes utilisés pour le traitement anticancéreux, vérapamil, inhibiteurs de tyrosine kinase (tyrphostine), cyclosporine A, paclitaxel est ses dérivés comme le 6-α-hydroxy-paclitaxel, baccatine, taxotères et autres, oligomères macrocycliques du suboxide de carbone (MCS) et ses dérivés, produits synthétiquement ainsi que provenant de sources natives, mofebutazone, acémetacine, diclofenac, lonazolac, dapson, acide acétique o-carbamoylphénoxy, lidocaine, ketoprofène, acide méfénamique, piroxicam, meloxicam, chloroquine phosphate, penicillamine, hydroxychloroquine, auranofine, aurothiomalate de sodium, oxaceprol, célécoxib, β-sitosterine, ademetionine, myrtecaine, polidocanol, nonivamide, levomenthol, benzocaïne, aescine, ellipticine, D-24851 (Calbiochem), colcemide, cytochalasine A-E, indanocine, nocodazole, protéine S 100, bacitracine, antagonistes du récepteur de ka vitronectine, azélastine, stimulateur de la guanidyl cyclase, inhibiteur tissulaire des protéinases métalliques 1 et 2, acides nucléiques libres, acides nucléiques incorporés dans des transmetteurs de virus, fragments de DNA et RNA, inhibiteur activateur plasminogène 1, inhibiteur activateur plasminogène 2, oligonucléotides antisens, inhibiteurs VEGF, IGF-1, substances actives provenant du groupe des antibiotiques comme céfadroxil, céfazoline, céfaclor, céfoxitine, tobramycine, gentamycine, pénicillines comme dicloxacilline, oxacilline, sulfonamides, metronidazol, antithrombotiques comme argatroban, aspirine, abciximab, antithrombine synthétique, bivalirudine, coumadine, enoxaparine, héparine desulfatée et N-acetylée, activateur tissulaire du plasminogène, récepteur membrane plaquettaire GpIIb/IIIa, anticorps contre l'inhibiteur facteur Xa, héparine, hirudine, r-hirudine, PPACK, protamine, prourokinase, streptokinase, wafarine, urokinase ; vasodilateurs comme le dipyridamol, trapidil, nitroprusside, antagonistes de PDGF comme la triazolopyrimidine et seramine, inhibiteurs de l'ACE comme captopril, cilazapril, lisinopril, enalapril, losartan, inhibiteurs de la thioprotease, prostacycline, vapiprost, interféron α, β et γ, antagonistes de l'histamine, bloqueurs de la sérotonine, inhibiteurs d'apoptose, régulateurs de l'apoptose comme p65, oligonucléotides antisens NFkB ou Bcl-xL, halofuginone, nifédipine, tocophérol, vitamines B1, B2, B6 et B12, acide folique, tranilast, molsidomine, polyphénoles du thé, épicatéchine gallate, épigallocatéchine gallate, acides boswelliques et leurs dérivés, leflunomide, anakinra, etanercept, sulfasalazine, dicloxacilline, tetracycline, triamcinolone, mutamycin, procainamde, acide rétinoïque, quinidine, disopyramide, flecainide, propafénone, sotalol, amidorone ; stéroïdes produites en manière naturelle et synthétique comme bryophylline A, inotodiol, maquiroside A, ghalakinoside, mansonine, strebloside, hydrocortisone, bétaméthasone, déxaméthason, substances non-stéroïdiens (NSAIDS) comme fénoprofène, ibuprofène, indométhacine, naproxène, phenylbutazone et d'autres agents antiviraux comme acyclovir, ganciclovir et zidovudine ; antimycotiques comme clotrimazol, flucytosine, griséofulvine, kétoconazole, miconazole, nystatine, terbinafine ; agents antiprotozoaires comme chloroquine, mefloquine, quinine, en outre terpénoïdes naturelles comme hippocaesculin, barringtogenol-C21-angelate, 14-déhydro-agrostistachin, agroskerine, agrostistachine, 17-hydroxy-agrostistachine, ovatodiolides, acide 4,7-oxycycloanisomelique, baccharinoides B1, B2, B3 et B7, tubeimoside, bruceanoles A,B et C, bruceantinosides C, yadanziosides N et P, isodéoxyelephantopine, tomenphantopine A et B, coronarine A, B, C et D, acide ursolique, acide hyptatique A, zéorine, iso-iridogermanal, maytenfoliol, effusantine A, excisanin A et B, longikaurin B, sculponéatin C, kamebaunin, leukamenin A et B, 13,18-déhydro-6-alpha-senecioyloxychaparrin, taxamairin A et B, regenilol, triptolide ; en outre cymarine, apocymarine, acide aristolochique, anopterine, hydroxy anopterine, anémonine, proto-anémonine, berbérine, chloride de cheliburine, ciguatoxine, sinococuline, combreastatine A et B, curdraisoflvaone A, curcumine, dihydronitidine, chloride de nitidine, 12-beta-hydroxy-pregnadiène-3,20-dione, bilobol, ginkgol, acide ginkgolique, helenalin, indicin, indicin-N-oxide, lasiocarpine, inotodiol, glycoside 1a, podophyllotoxine, justicidine A et B, larréatin, malloterin, mallotochromanol, isobutyryl-mallotochromanol, maquiroside A, marchantine A, maytansine, lycoridicine, margetine, pancratistatine, liriodenine, oxoushinsunine, aristolactame-AII, bisparthenolidine, periplocoside A, ghalakinoside, acide usolique, deoxy-psorospermine, psycorubine, ricine A, sanguinarine, acide Manwuweiz (Manwuweizsäure), méthylsorbifolin, sphatheliachromes, stizophylline, akagerine, dihydrousambaraensin, hydroxyusambarine, strychnopentamine, strychnophylline, usambarine, usambarensine, liriodenine, daphnoretine, lariciresinol, methoxy-lariciresinol, syringaresinol, umbelliferone, afromoson, acétyl-vismione B, déacetyl-vismione A, vismione A et B.

33. Méthode selon l'une des revendications 21 - 32, **caractérisée en ce que** l'application ou l'immobilisation des oligosaccharides et/ ou polysaccharides selon l'une des revendications 1 - 3, 7 - 11, 14 - 20 s'effectue par des interactions hydrophobes, des forces van der Waal, des interactions électrostatiques, des liaisons hydrogènes, des interactions ioniques, des réticulations transversales et/ ou des liaisons covalentes.

34. Produit médical qui peut être obtenu suivant une méthode selon l'une des revendications 21 - 33.

35. Produit médical, où la surface du produit médical est recouvert d'une couche hémocompatible, ou directement et/ ou par au moins une couche biostable et/ ou biodégradable et/ ou une couche active interjacentes, consistant d'au moins un oligosaccharide et/ ou polysaccharide selon les revendications 1 - 3, 7 - 11, 14 - 20.

36. Produit médical selon la revendication 35, où au moins une couche biostable et/ ou biodégradable est située au-dessous de ou entre deux couches hémocompatibles.

37. Produit médical selon la revendication 35 ou 36, où la couche hémocompatible est recouvert, complètement ou incomplètement, avec au moins une autre couche biostable et/ ou biodégradable située au-dessus de la couche hémocompatible.

38. Produit médical selon la revendication 36 ou 37, où au moins une couche active est située entre la couche biostable et/ ou biodégradable qui contient au moins une substance active qui est antiproliférative, antiinflammatoire et/ou antithrombotique et qui est covalemment et/ ou adhésivement liée.

39. Produit médical selon l'une des revendications 35 - 38, où au moins une substance active qui est antiproliférative, antiinflammatoire et/ ou antithrombotique est liée covalemment et/ ou adhésivement dans et/ ou à la couche hémocompatible et/ ou la couche biostable et/ ou biodégradable.

40. Produit médical selon l'une des revendication 35 - 39, **caractérisé en ce que** les substances actives utilisées sont sélectionnées du groupe comprenant sirolimus (rapamycine), everolimus, pimecrolimus, somatostatine, tacrolimus, roxithromycine, daunamycine, ascomycine, bafilomycine, erythromycine, midecamycine, josamycine, concanamycine, clarithromycine, troleandomycine, folimycine, cerivastatine, simvastatine, lovastatine, fluvastatine, rosuvastatine, atorvastatine, pravastatine, pitavastatine, vinblastine, vincristine, vindesine, vinorelbine, etoposide, teniposide, nimustine, carmustine, lomustine, cyclophosphamide, 4-hydroxyoxycyclophosphamide, estramustine, melphalane, ifosfamide, trofosfamide, chlorambucile, bendamustine, dacarbazine, busulphane, procarbazine, treosulfan, thymosine α-1, temozolomide, thiotepa, thialin (acide 2-methylthiazolidine-2,4-dicarbonique), thialin-Na (sel de sodium de thialin), aunorubicine, doxorubicine, aclarubicine, épirubicine, mitoxantrone, idarubicine, bléomycine, mitomycine, dactinomycine, methotrexate, fludarabine, fludarabine-5'-dihydrogenophosphate, cladribine, mercaptopurine, thioguanine, cytarabine, fluorouracil, gemcitabine, capécitabine, docetaxel, carboplatine, cisplatine, oxaliplatine, amsacrine, irinotécan, topotécan, hydroxycarbamide, miltefosine, pentostatine, aldesleukine, trétinoïne, asparaginase, pegaspargase, anastrozole, exémestane, létrozole, formestane, aminoglutéthimide, adriamycine, azithromycine, spiramycine, cepharanthine, inhibiteur 2w de la prolifération des cellules musculaires lisses, épothilone A et B, azathioprine, mycophénolate mofétil, c-myc antisens, b-myc antisens, acide betulinique, camptothécine, PI-88 (oligosaccharide sulfaté), melanocyte-stimulating hormone (α-MSH), protéine C activée, inhibiteur de IL1-β, acide fumarique et ses esters, dermicidine, calcipotriol, tacalcitol, lapachol, β-lapachone, podophyllotoxine, bétuline, 2-ethylhydrzide de l'acide podophyllique, molgramostime (rhuGm-CSF), peginterféron alfa-2b, lénograstime (r-HuG-CSF), filgrastime, macrogol, goséréline, cephalomannine, trastuzumab, exemestan, basiliximab, daclizumab, sélectine (cytokine antagoniste), inhibiteur CETP, cadhérine, inhibiteurs de la cytokinine, inhibiteur de la COX-2, NFkB, angiopeptine, ciprofloxacine, camptothécine, fluroblastine, anticorps monoclonaux inhibitants la prolifération des cellules musculaires, bFGF antagonistes , probucol, prostaglandine, 1,11-dimethoxycanthin-6-one, 1-hydroxy-11-méthoxycanthin-6-one, scopoletine, colchicine, donneurs NO comme le pentaerythrityl tetranitrate et les sydnoimines , dérivés de S-nitroso , tamoxifène, staurosporine, β-estradiol, α-estradiol, estriol, estrone, ethinylestradiol, fosfestrol, medroxy-progestérone, cipionates d'estradiol, , benzoates d'estradiol , tranilast, Kamebakaurin et d'autres terpénoïdes utilisés pour le traitement anticancéreux, vérapamil, inhibiteurs de la tyrosine kinase (tyrphostine), cyclosporine A, paclitaxel et ses dérivés comme 6-α-hydroxy-paclitaxel, baccatine, taxotères et d'autres oligomères macrocycliques du suboxide de carbone (MCS) et ses dérivés, produits synthétiquement ainsi que provenant de sources natives, mofébutazone, acémetacine, diclofenac, lonazolac, dapson, acide acétique o-carbamoylphénoxy, lidocaine, ketoprofène, acide méfénamique, piroxicam, meloxicam, chloroquine phosphate, pénicillamine, hydroxy chloroquine, auranofine, aurothiomalate de sodium, oxaceprol, celecoxib, β-sitosterine, ademetionine, myrtecaine, polidocanol, nonivamide, levomenthol, benzocaine, aescine, ellipticine, D-24851 (Calbiochem), colcemide, cytochalasine A-E, indanocine, nocodazole, protéine S 100, bacitracine, récepteur de la vitronectine antagonistes, azélastine, stimulateur de la guanidyl cyclase, inhibiteur tissulaire des protéinases métalliques 1 et 2, acides nucléiques libres, acides nucléiques incorporés dans des transmetteurs de virus, fragments de DNA et RNA, inhibiteur de l'activateur plasminogène 1, inhibiteur d'activateur plasminogène 2, oligonucléotides antisens, inhibiteurs VEGF, IGF-1 ; substances actives provenant du groupe des antibiotiques comme céfadroxil, céfazoline, céfaclor, céfoxitine, tobramycine, gentamycine ; pénicillines comme dicloxacilline, oxacilline, sulfonamides, metronidazol, antithrombotiques comme argatroban, aspirine, abciximab, antithrombine synthétique, bivalirudine, coumadine, enoxaparine, héparine desulfatée et N-acetylée, prourokinase, activateur tissulaire du plasminogène, récepteur membrane plaquettaire GpIIb/IIIa, anticorps contre l'inhibiteur facteur Xa, héparine, hirudine, r-hirudine, PPACK, protamine, prourokinase, streptokinase, wafarine, urokinase ; vasodilateurs comme dipyridamol, trapidil, nitroprusside ; PDGF antagonistes comme triazolopyrimidine et seramine ; inhibiteurs de l'ACE comme captopril, cilazapril, lisinopril, enalapril, losartan, inhibiteurs de thio protease, prostacycline, vapiprost, interféron α, β et γ, antagonistes de l'histamine, bloqueurs de la sérotonine ; inhibiteurs d'apoptose : régulateurs d'apoptose comme p65, oligonucléotides antisens Bcl-xL ou NF-kB, halofuginone, nifedipine, tocopherol, tranilast, molsidomine, polyphénoles du thé, épicatéchine gallate, épigallocatéchine gallate, acides boswelliques et leurs dérivés, leflunomide, anakinra, étanercept, sulfasalazine, étoposide, dicloxacilline, tetracycline, triamcinolone, mutamycin, procainimide, acide rétinoïque, quinidine, disopyrimide, flécaïnide, propafénone, sotalol, amidorone ; stéroïdes produites en manière naturelle et synthétique comme bryophylline A, inotodiol, maquiroside A, ghalakinoside, mansonine, strebloside, hydrocortisone, bétaméthasone, déxaméthason ; substances non-stéroïdiennes (NSAIDS) comme fénoprofène, ibuprofène, indométhacine, naproxène, phenylbutazone et d'autres agents antiviraux comme acyclovir, ganciclovir et zidovudine; antimycotiques comme clotrimazol, flucytosine, griséofulvine, kétoconazole, miconazole, nystatine, terbinafine ; agents antiprotozoaires comme chloroquine, mefloquine, quinine, en outre terpénoïdes naturelles comme hippocaesculin, barringtogenol-C21-angelate, 14-déhydroagrostistachin, agroskerine, agrostistachin, 17-hydroxy-agrostistachin, ovatodiolides, acide 4,7-oxycycloanisomelique, baccharinoides B1, B2, B3 et B7, tubeimoside, bruceanoles A, B et C, bruceantinosides C, yadanziosides N et P isodeoxyelephantopine, tomenphantopine A et B, coronarine A, B, C et D, acide ursolique, acide hyptatique A, zéorine, iso-iridogermanal, maytenfoliol, effusantine A, excisanine A et B, longikaurine B, sculponeatine C, kamebaunine, leukamenine A et B, 13,18-déhydro-6-alpha-Senecioyloxychaparrin, taxamairine A et B, regenilol, triptolide, en outre cymarine, apocymarine, acide aristolochique, anopterine, hydroxy anopterine, anémonine, proto-anémonine, berbérine, chloride de cheliburine, ciguatoxine, sinococuline, bombreastatine A et B, curdraisoflvaone A, curcumine, dihydronitidine, chloride de nitidine, 12-beta-hydroxy-pregnadiène-3,20-dione, bilobol, ginkgol, acide ginkgolique, helenaline, indicine, indicine-N-oxide, lasiocarpine, inotodiol, glycoside 1a, podophyllotoxine, justicidine A et B, larréatine, malloterine, mallotochromanol, isobutyryl-mallotochromanol, maquiroside A, marchantine A, maytansine, lycoridicin, margetin, pancratistatine, liriodenine, bisparthenolidine, oxoushinsunin, aristolactame-All, bisparthenolidine, periplocoside A, ghalakinoside, deoxy-psorospermine, psycorubine, ricine A, sanguinarine, acide Manwuweiz (Manwuweizsäure), méthylsorbifolin, sphatheliachromes, stizophyllin, akagerine, dihydrousambaraensine, hydroxy-usambarine, strychnopentamine, strychnophylline, usambarine, usambarensine, liriodenine, daphnoretine, lariciresinole, methoxylariciresinol, syringaresinol, umbelliferone, afromoson, actylvismione B, déacetyl-vismione A, vismione A et B.

41. Produit médical selon la revendication 40, **caractérisé en ce que** les substances actives utilisées sont les suivantes: tacrolimus, pimecrolimus, PI 88, thymosine α-1, baccatine et ses dérivés, docétaxel, colchicine, paclitaxel et ses dérivés, trapidil, estradiol α et β, dermidicidine, thialine sodium, simvastatine, oligomères macrocycliques du suboxide de carbone (MCS) et ses dérivés, sirolimus, tyrphostins, D24851, colchicine, acide fumarique et ses esters, protéine activée C, inhibiteurs 1β interleukine et melanocyte-stimulating hormone (α-MSH) ainsi que des mélanges de ces substances actives.

42. Produit médical selon l'une des revendications 34 - 41, **caractérisé en ce que** le produit médical concerne des prothèses, des organes, des vases, des aortes, des valvules, des tubes, des pièces de rechange organiques, des implants, des fibres, des fibres creuses, des stents, des canules, des seringues, des membranes, des conserves, des flacons de sang conservés, des plaques de titration, des stimulateurs cardiaques, des phases d'adsorption, des phases chromatographiques, des colonnes de chromatographie, des dialysateurs, des pièces de connexion, des senseurs, des valvules, des chambres de centrifugeuse, des échangeurs de chaleur, des endoscopes, des filtres, des chambres de pompage.

43. Produit médical selon la revendication 42, **caractérisé en ce que** le produit médical est un stent.

44. Stents selon la revendication 43 où les quantités de polymères appliquées sont entre 0,01 mg à 3 mg/ couche, en préférence 0,20 mg à 1 mg et particulièrement préféré entre 0,2 mg à 0,5 mg/ couche.

45. Stent selon la revendication 43 ou 44, **caractérisé en ce que** la substance active antiproliférative, antiinflammatoire et/ ou antithrombotique est présente dans une concentration pharmaceutiquement active de 0,001 - 10 mg par cm² de la surface du stent et par couche.

46. Stent selon l'une des revendication 43 - 45 susceptible à la prévention ou à la réduction de la resténose.

47. Stent selon l'une des revendications 43 - 45 susceptible à la libération continuée d'au moins une substance active qui est antiproliferative, antiinflammatoire et/ ou antithrombotique.

48. Utilisation des produits médicaux selon l'une des revendications 34 - 41 pour le contact direct avec le sang.

49. Utilisation des produits médicaux selon l'une des revendications 34 - 41 pour la prévention ou la réduction de l'adhésion de protéines sur les surfaces revêtues des produits médicaux.

50. Utilisation selon la revendication 48 ou 49, **caractérisée en ce que** la surface revêtue en manière hémocompatible des plaques de microtitration ou d'autres vecteurs utilisés pour les procédés de détection thérapeutique empêche ou réduit le dépôt de protéines .

51. Utilisation selon la revendication 48 ou 49, **caractérisée en ce que** la surface hémocompatible de phases d'adsorption ou chromatographiques fait en sorte que le dépôt aspécifique de protéines soit évité ou réduit.
